(19) Europäisches Patentamt
European Patent Office
Office européen des brevets

(11) Publication number: **0 115 394 B1**

(12) # EUROPEAN PATENT SPECIFICATION

(45) Date of publication of patent specification: 28.06.89

(21) Application number: 84300239.5

(22) Date of filing: 16.01.84

(51) Int. Cl.[4]: A 61 K 31/54, A 61 K 31/535, A 61 K 31/495, C 07 D 279/18, C 07 D 265/38, C 07 D 241/46, C 07 D 293/10, C 07 D 279/34, C 07 D 279/36, C 07 D 265/34

(54) **Phenothiazone derivatives and analogs.**

(30) Priority: 21.01.83 US 459924
28.09.83 US 536487

(43) Date of publication of application:
08.08.84 Bulletin 84/32

(45) Publication of the grant of the patent:
28.06.89 Bulletin 89/26

(84) Designated Contracting States:
CH DE FR GB IT LI NL

(56) References cited:
CHEMICAL ABSTRACTS, vol. 72, no. 13, 30th March 1970, page 406, no. 66965m, Columbus, Ohio, USA; & JP - A - 69 27588 (FUJISAWA PHARMACEUTICAL CO., LTD.) 15-11-1969

CHEMICAL ABSTRACTS, vol. 90, no. 9, 26th February 1979, page 133, no. 67656v, Columbus, Ohio, USA; I. GHIZDAVU et al.: "Study on the mode of action of phenothiazine and its derivatives on insects" & BUL. INST. AGRON. CLUJ-NAPOCA 1977, 31, 65-71

(73) Proprietor: MERCK FROSST CANADA INC.
16711 Trans-Canada Highway
Kirkland Quebec (CA)

(72) Inventor: Guindon, Yvan
409 Place Closse
Ile Bizard Quebec, H9C 1Y7 (CA)
Inventor: Fortin, Rejean
4820 Boulevard Leger
Montreal Quebec, H1H 1N3 (CA)
Inventor: Lau, Cheuk K.
15569 Fernand St.
Pierrefonds Quebec, H9H 1M6 (CA)
Inventor: Rokach, Joshua
416 Place Canterbury Chomedey
Laval Quebec, H7W 4G9 (CA)
Inventor: Yoakim, Christiane
5770 Cote St. Luc Road, Apt. 12
Montreal Quebec, H3X 2G1 (CA)

(74) Representative: Crampton, Keith John Allen et al
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 115 394 B1

Courier Press, Leamington Spa, England.

(56) References cited:

CHEMICAL ABSTRACTS, vol. 78, no. 7, 19th February 1973, page 485, no. 43395k, Columbus, Ohio, USA; V.I. SHVEDOV et al.: "Synthesis and reactions of 1,2,3,4-tetrahydrophenothiazines" & KHIM. GETEROTSIKL. SOEDIN. 1972, (11), 1509-1513

CHEMICAL ABSTRACTS, vol. 92, no. 15, 14th April 1980, page 12, no. 121490t, Columbus, Ohio, USA; S.C. MITCHELL et al.: "Metabolism of phenothiazine in the guinea pig" & DRUG METAB. DISPOS. 1979, 7(6), 399-403

JOURNAL OF THE CHEMICAL SOCIETY, PERKIN TRANSACTIONS I, no. 3, March 1982, pages 831-834; T.L. GILCHRIST et al.: "1H-1,4-Benzothiazines. New cyclic sulphonium ylides"

CHEMICAL ABSTRACTS, vol. 99, no. 9, 29th August 1983, page 622, no. 70656m, Columbus, Ohio, USA; M. RAILEANU: "New synthesis of phenothiazine 5-oxide and 3-phenothiazone" & REV. CHIM. (BUCHAREST) 1983, 34(2), 113-115

**Description**

BACKGROUND OF THE INVENTION

This invention involves certain phenothiazone derivatives and analogs. These compounds are useful as inhibitors of mammalian leukotriene biosynthesis. As such, these compounds are useful therapeutic agents for treating allergic conditions, asthma, cardiovascular disorders and inflammation.

The leukotrienes are a novel group of biologically active mediators derived from arachidonic acid through the action of lipoxygenase enzyme systems. There are two groups of leukotrienes derived from the common unstable precursor Leukotriene $A_4$. The first of these are the peptido-lipid leukotrienes, the most important being Leukotrienes $C_4$ and $D_4$. These compounds collectively account for the biologically active material known as the slow reacting substance of anaphylaxis.

The leukotrienes are potent smooth muscle contracting agents, particulalry on respiratory smooth muscle but also on other tissues (e.g. gall bladder). In addition, they promote mucous production, modulate vascular permeability changes and are potent inflammatory agents in human skin. The most important compound in the second group of leukotrienes is Leukotriene $B_4$, a dihydroxy fatty acid. This compound is a potent chemotactic agent for neutrophils and eosinophils and in addition, may modulate a number of other functions of these cells. It also effects other cell types such as lymphocytes and for example may modulate the action of T-suppressor cells and natural killer cells. When injected *in vivo*, in addition to promoting the accumulation of leukocytes, Leukotriene $B_4$ is also a potent hyperalgesic agent and can modulate vascular permeability changes through a neutrophil dependent mechanism. Both groups of leukotrienes are formed following oxygenation of arachidonic acid through the action of a 5-lipoxygenase enzyme. See for example, D. M. Bailey *et al., Ann. Rpts. Med. Chem. 17* 203 (1982).

Respiratory Conditions

a) *Asthma.* The leukotrienes are potent spasmogens of human trachea, bronchus and lung parenchymal strips, and when administered to normal volunteers as aerosols are 3,800 times more potent than histamine at inducing a 50% decrease in air flow at 30% of vital capacity. They mediate increases in vascular permeability in animals and promote mucous production in human bronchial explants. In addition, Leukotriene $B_4$ may also mediate mucous production and could be an important mediator of neutrophil and eosinophil accumulation in asthmatic lungs. 5-Lipoxygenase products are also thought to be regulators of mast cell degranulation and recent studies with human lung mast cells have suggested that 5-Lipoxygenase inhibitors, but not corticosteroids, may suppress antigen-induced mast cell degranulation. *In vitro* studies have shown that antigen challenge of human lung results in the release of leukotrienes and in addition purified human mast cells can produce substantial amounts of leukotrienes. There is therefore good evidence that the leukotrienes are important mediators of human asthma. 5-Lipoxygenase inhibitors would therefore be a new class of drugs for the treatment of asthma. See for example, B. Samuelsson, *Science 220* 568—575 (1983).

Skin Diseases

a) *Psoriasis.* Psoriasis is a human skin disease which effects between two and six percent of the population. Thee is no adequate therapy for psoriasis and related skin conditions. The evidence for leukotriene involvement in these diseases is as follows. One of the earliest events in the development of prepapillary lesions is the recruitment of leukocytes to the skin site. Injection of Leukotriene $B_4$ into human skin results in a pronounced neutrophil accumulation. There are gross abnormalities in arachidonic acid metabolism in human psoriatic skin. In particular, highly elevated levels of free arachidonic acid can be measured as well as large amounts of lipoxygenase products. Leukotriene $B_4$ has been detected in psoriatic lesions, but not in uninvolved skin, in biologically significant amounts.

Allergic Conditions

a) Leukotrienes can be measured in nasal washings from patients with allergic rhinitis and are greatly elevated following antigen challenge. Leukotrienes may mediate this disease through their ability to regulate mast cell degranulation, by modulating mucous production and mucocillary clearance and by mediating the accumulation of inflammatory leukocytes.

Leukotrienes can also mediate other diseases. These include atopic dermatitis, gouty arthritis and gall bladder spasms. In addition, they may have a role in cardiovascular disease because leukotrienes $C_4$ and $D_4$ act as coronary and cerebral arterial vasoconstrictors and these compounds may also have negative inotropic effects on the myocardium. In addition, the leukotrienes are important mediators of inflammatory diseases through their ability to modulate leukocyte and lymphocyte function.

A number of Phenothiazone derivatives of the general Formula:

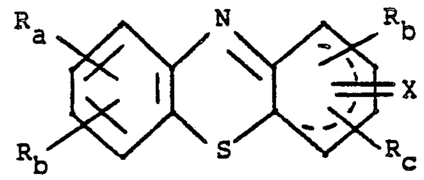

A

especially when X is O are taught in the literature; see for example Terdic *et al., Rev. Roum. Chim. 13,* 833—8 (1968; Beckett *et al., Xenobiotica 8,* 721—36 (1978); Panea *et al., Rev. Roum. Chim. 25,* 691—5 (1980); Bhargava *et al.,; Gazz. Chim. Ital. 110,* 201—3 (1980); Bodea *et al., Ann. Chem. 698,* 186—90, (1966); Sugita *et al., Nippon Kagaku Zasshi 89,* 309—15 (1968); Broser *et al., Rev. Roum. Chem. 17,* 1747—53 (1972); Bodea *et al., Ann. Chem. 715,* 122—7 (1968); Bodea *et al., Rev. Roum. Chim. 13,* 971—6 1241—4 (1968); Sugita *et al.,* Japanese Patent No. 73, 22,714 (1973); Tsyino, *Tet. Lett.* (10), 763—6 (1969); Roseboum *et al., J. Pharm. Sci. 66,* 1395—8 (1977); Shakii *et al.,* Yakugaku Zasshi *86,* 541—3 (1966); Bodea *et al., Ann. Chem. 614,* 171—6 (1958); Collier *et al., Can. J. Med. Sci. 31,* 195—201 (1953); and Collier *et al., Can. J. Med. Sci. 30,* 443—6 (1952). However, none of the compounds of Formula A is taught to have mammalian leukotriene biosynthesis inhibitor activity.

It has been discovered that compounds of Formula A and especially those where X is O are effective inhibitors of mammalian leukotriene biosynthesis and are thus useful therapeutic agents for treating conditions such as asthma, allergies, inflammation and certain skin diseases in humans.

This invention provides the use, for the manufacture of a medicament for inhibiting mammalian leukotriene biosynthesis or action, of a compound of the Formula I, a compound that is a salt of a compound of the Formula I, or a pharmaceutical composition containing such a compound:

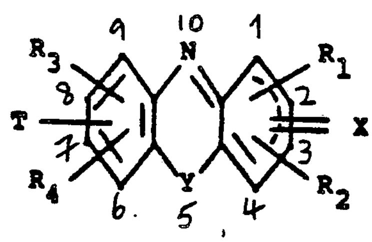

I

in which

X is in the 1 or 3 position and is O, S or NR;

R is H, $C_{1-6}$ branched or linear alkyl, CN or phenyl;

Y is O, Se, S, SO, $SO_2$ or NR; and the broken line represents a double bond between the 1 and 2 or 2 and 3 positions;

each of $R_1$, $R_2$, $R_3$ and $R_4$ independently of the others, is

(1) hydrogen,

(2) $C_{1-6}$ alkyl,

(3) $C_{2-6}$ alkenyl,

(4) $—(CH_2)_n$ M

where n is 0 or an integer from 1 to 6 and M is

(a) $OR_5$,

(b) halogen,

(c) $CF_3$,

(d) $SR_5$ where $R_5$ is H; $C_1—C_6$ alkyl; benzyl; phenyl or substituted phenyl where the substituents are $C_{1-3}$ alkyl, halogen, CN, $CF_3$, $COOR_6$, $CH_2COOR_6$, $(CH_2)_nNR_8R_9$ where n is 0, 1 or 2, $C_{1-3}$ alkoxy, OH or $C_{1-6}$ haloalkyl; $—(CH_2)_mCOOR_6$, where m is 0 or an integer from 1 to 6 and $R_6$ is H, phenyl or $C_{1-6}$ alkyl; CN, formyl, $CF_3$ or $CH_2—R_{12}$, where $R_{12}$ is $C_{1-5}$ alkyl, phenyl or dimethylamino;

(e) phenyl or substituted phenyl as defined above for $R_5$;

(f) $COOR_6$;

(g) $$-\overset{O}{\overset{\|}{C}}-R_{14}$$

where $R_{14}$ is H, $(CH_2)_n$ $COOR_6$ where n is 0 or an integer from 1 to 4, $C_{1-6}$ alkyl, $CF_3$, phenyl, or substituted phenyl as defined above for $R_5$;

(h) tetrazole;

(i) $$—NH—\overset{O}{\overset{\|}{C}}—R_7$$

where $R_7$ is $C_{1-6}$ alkyl, benzyl or phenyl;

(j) $-NR_8R_9$ where $R_8$ and $R_9$ are independently selected from H, phenyl or substituted phenyl as defined above for $R_5$ or $C_{1-4}$ alkyl, $C_{1-4}$ alkylaminoalkyl, or may be joined through the N to form a 4-methyl piperazinyl radical;

(k) $-NHSO_2R_{10}$ where $R_{10}$ is OH, $C_{1-6}$ alkyl, $C_{1-6}$ alkoxy, phenyl or $CF_3$;

(l) 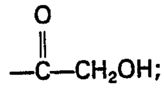

(m) $-SOR_{11}$ where $R_{11}$ is $C_{1-6}$ alkyl, phenyl or substituted phenyl as defined above for $R_5$, $(CH_2)_mCOOR_6$ where m is 1 to 6, CN, formyl or $CF_3$;

(n) $-CONR_8R_9$;

(o) $-SO_2NR_8R_9$;

(p) $-SO_2R_{13}$ where $R_{13}$ is OH, H, $C_{1-6}$ alkyl, phenyl or substituted phenyl as defined above for $R_5$, $(CH_2)_mCOOR_6$ where m is 1 to 6, CN or $CF_3$;

(q) $NO_2$;

(r) 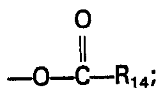

(s) 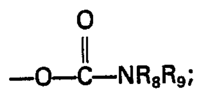

(t) 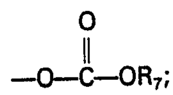

(u) $-CN$; or

(v) $NR_{15}R_{16}$ where $R_{15}$ and $R_{16}$ are such that $HNR_{15}R_{26}$ is an essential amino acid;

or any two of $R_1$, $R_2$, $R_3$ and $R_4$ are joined to form a fourth saturated or unsaturated $C_{5-6}$ ring; and

T is H, halogen or $CF_3$.

Certain compounds of Formula (I) are novel and constitute another embodiment of the invention.

The numbers surrounding Formula I designate the substituent positions. T, $R_1$, $R_2$, $R_3$ and $R_4$ may be positioned anywhere in the structure. $R_1$, $R_2$, $R_3$ and $R_4$ may also be joined, e.g. as $-(CH_2)_{3-4}-$, to add a fourth ring to the basic three ring structure. This fourth ring may have five or six carbon atoms and may be saturated or unsaturated. For example, compounds of Formula II may be prepared by linking two of the substituents groups; $R_1$, $R_2$, $R_3$, $R_4$:

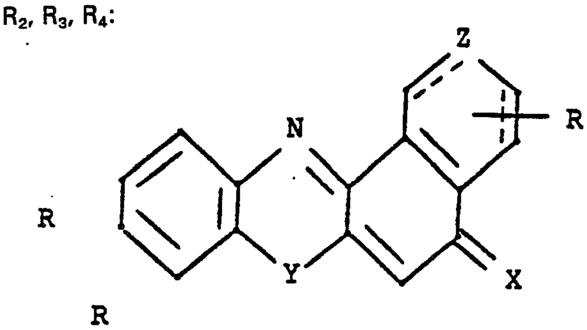

II

wherein Z may be CH, $CH_2$ or a bond, the broken lines represent optional double bonds and R represents the substituent groups of Formula I ($R_1$, $R_2$, $R_3$, $R_4$ and/or T) not used to create the fourth ring.

The preferred compounds of Formula II have the structure II(a):

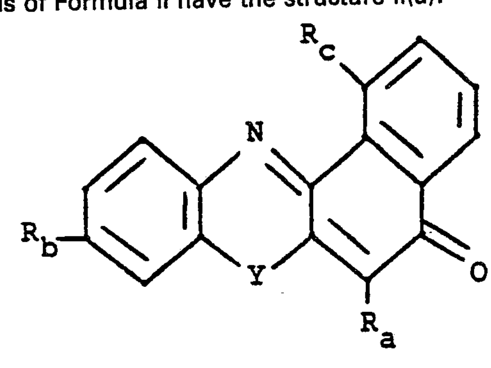

II(a)

wherein $R_a$ is selected from hydrogen, halogen (F, Br, Cl, I), $CH_3$, $CF_3$, $COR_d$, $NHR_d$, $SR_d$ and $OR_d$; $R_b$ is selected from hydrogen, halogen (F, Br, Cl, I), $CH_3$, $CF_3$, $CH_2OH$, $OR_d$, $SR_d$, $COR_d$, $COOR_d$, $CH_2COOR_d$ and $CH(CH_3)COOR_d$; $R_d$ is hydrogen, phenyl, $C_{1-4}$ straight or branched alkyl; $R_c$ is selected from hydrogen or $OR_d$; and Y is selected from O, S, SO or $SO_2$.

Where possible, appropriate pharmaceutically acceptable salts of Formula I are included e.g., carboxylic or mineral acid addition salts where Formula I is basic and metal salts e.g. Na, K, $NH_4$ where Formula I is acidic.

The term alkyl, unless indicated otherwise, indicates straight chain, branched chain and cycloalkyl groups. The term halogen or halo, unless otherwise indicated, includes Cl, Br, I and F.

A group of preferred compositions contain a compound of the Formula:

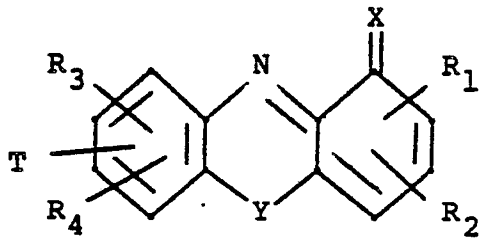

I(a)

More preferred Formula I(a) compounds are those wherein X is O or NH and Y is S, O, SO or $SO_2$. Still more preferred I(a) compounds are those having the Formula:

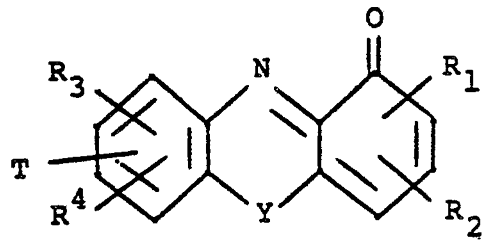

I(a)

wherein:

a) T, $R_3$ and $R_2$ are hydrogens,
b) T, $R_1$, $R_2$ are hydrogens,
c) T, $R_3$ and $R_4$ are hydrogens,
d) T, $R_1$, $R_2$, $R_3$ are hydrogens or
e) T, $R_3$, $R_4$, $R_2$ are hydrogens.

wherein $R_1$, $R_2$, $R_3$, $R_4$ and T are as defined for Formula I.

Another group of preferred compositions contain compounds of the Formula I(b):

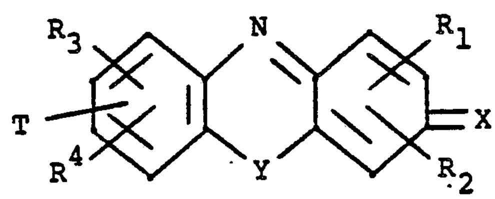

I(b)

More preferred compounds of Formula Ib are those having the Formula wherein X is O or NH and Y is O, S, SO, $SO_2$. Still more preferred compounds of Formula I(b) are those of the formula I(c):

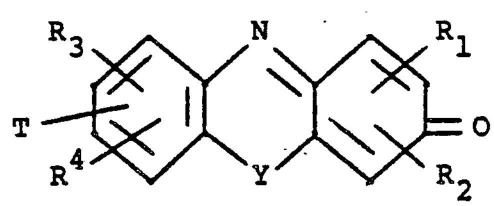

I(c)

I(c)

wherein:

a) T, $R_3$ and $R_2$ are hydrogens,
b) T, $R_1$, $R_2$ are hydrogens,
c) T, $R_3$ and $R_4$ are hydrogens,
d) T, $R_1$, $R_2$, $R_3$ are hydrogens,

e) T, $R_3$, $R_4$, $R_2$ are hydrogens,
f) T, $R_3$ and $R_4$ are hydrogens and $R_1$ is in position 4,
g) T, $R_3$, $R_2$ are hydrogens and $R_1$ is in position 4,
h) T, $R_4$, $R_3$, $R_2$ are hydrogens and $R_1$ is in position 4,
i) $R_2$ and $R_3$ are hydrogens,
j) $R_1$ and $R_2$ are hydrogens,
k) $R_3$ and $R_4$ are hydrogens,
l) $R_2$, $R_3$ are hydrogens and T is in position 4,
m) T and $R_3$ are hydrogens and $R_2$ is in position 4,
n) T and $R_3$ are hydrogens and $R_1$ is in position 4 and $R_2$ is in position 2,
o) T and $R_3$ are hydrogens and $R_4$ is in position 7, $R_2$ is in position 4 and $R_1$ is in position 2.
(p) T is hydrogen, $R_1$, $R_2$, $R_3$, and $R_4$ are in positions 1, 2, 4 and 7, respectively.

A particularly preferred series of Formula I(c) compounds are those in which n = 0 or 1 in the unit $(CH_2)_nM$.

Examples of Formula I compounds useful in the present compositions are tabulated below. In each of the tables the numbers preceding the T and the $R_1$—$R_4$ definitions indicate the substituent position in the structure.

TABLE 1

Compounds of the Formula

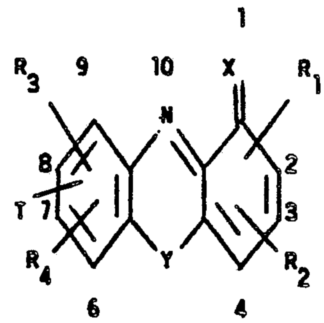

| Number | Y | X | $R_1$ | $R_2$ | $R_3$ | $R_4$ | T |
|---|---|---|---|---|---|---|---|
| 1 | O | O | 2-t-Bu | 8-t-Bu | 4-t-Bu | 6-t-Bu | H |
| 2 | O | O | 2-t-Bu | H | 4-Me | H | H |
| 3 | N—$CH_3$, S, O, Se, SO or $SO_2$ | O | 2-Cl | H | H | H | H |
| 4 | " | O | 2-$SCF_3$ | H | H | H | |
| 5 | " | O | 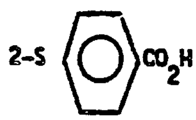 | H | H | H | H |
| 6 | " | O | 2-CN | H | H | H | H |
| 7 | " | O | H | 3-$CO_2$Et | H | H | H |
| 8 | " | O | H | 3-Cl | H | H | H |
| 9 | " | O | H | H | 4-Cl | H | H |
| 10 | " | O | H | H | 4-$SO_2CH_3$ | H | H |
| 11 | " | O | 2-Cl | H | 4-Cl | H | H |
| 12 | " | NH | 2-Cl | H | 4-Cl | H | H |
| 13 | " | NH | H | H | H | H | H |
| 14 | N—CN | O | 2-Cl | H | 4-Cl | H | H |
| 15 | S | O | H | H | H | H | H |
| 16 | S | O | 2-Cl | 3-Cl | 4-Cl | 7-Cl | 9-Cl |
| 17 | S | O | 2-Br | 3-Br | 4-Br | 7-Br | 9-Br |
| 18 | S | O | H | H | H | 7-$SO_2CH_3$ | H |
| 19 | S | O | 2-Cl | H | 4-$SO_2CH_3$ | H | H |
| 20 | S | O | 2-F | H | 4-Cl | H | H |
| 21 | S | O | 2-Br | H | H | H | H |

TABLE 1 continued

| Number | Y | X | $R_1$ | $R_2$ | $R_3$ | $R_4$ | T |
|---|---|---|---|---|---|---|---|
| 22 | S | O | 2-$CF_3$ | H | H | H | H |
| 23 | S | O | 2-$SCF_3$ | H | H | H | H |
| 24 | S | O | 2-$SO_2CF_3$ | H | H | H | H |
| 25 | S | O | H | 3-Cl | H | H | H |
| 26 | S | O | H | 3-$CO_2Et$ | H | H | H |
| 27 | S | O | H | 3-$CO_2H$ | H | H | H |
| 28 | S | O | H | 3-CN | H | H | H |
| 29 | S | O | H | 3-$SCF_3$ | H | H | H |
| 30 | S | O | H | H | 4-Cl | H | H |
| 31 | S | O | H | H | 4-$SCF_3$ | H | H |
| 32 | S | O | H | H | 4-Cl | H | H |
| 33 | S | O | 2-Br | H | 4-Br | H | H |
| 34 | S | O | 2-Cl | H | H | 8-CN | H |
| 35 | S | O | 2-Cl | H | H | 8-$CO_2Et$ | H |
| 36 | S | O | 2-Cl | H | H | 8-$CO_2H$ | H |
| 37 | S | O | 2-Cl | H | H | 8-$CF_3$ | H |
| 38 | S | O | 2-Cl | H | H | 7-$SO_2CH_3$ | H |
| 39 | S | O | H | 3-$CONMe_2$ | H | H | H |
| 40 | S | O | 2-Cl | H | H | 7-$OCH_3$ | H |
| 41 | S | O | 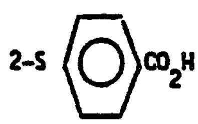 | H | H | H | H |
| 42 | S | O | 2-$SO_2CH_3$ | H | H | H | H |
| 43 | S | O | 2-$CH_2CH{=}CH_2$ | H | 4-$CH_2CH{=}CH_2$ | H | H |
| 44 | S | O | H | 3-$N(CH_3)_2$ | H | H | H |
| 45 | S | O | H | H | 4-Cl | 7-S—$C_6H_6$ | H |
| 46 | S | O | 2-$CH_2CO_2H$ | H | H | H | H |
| 47 | S | O | 2-Cl | H | 4-$SCH_2CO_2H$ | H | H |
| 48 | S | O | 2-$COCH_3$ | H | H | 7-$OCH_3$ | H |
| 49 | S | O | H | H | 4-CO—$C_6H_6$ | 7-$OCH_3$ | H |
| 50 | S | NH | 2-Cl | H | 4-Cl | H | H |

TABLE 1 continued

| Number | Y | X | $R_1$ | $R_2$ | $R_3$ | $R_4$ | T |
|---|---|---|---|---|---|---|---|
| 51 | S | NH | H | 3-$N(CH_3)_2$ | H | H | H |
| 52 | S | NH | 2-$SCH_3$ | H | 4-$SCH_3$ | H | H |
| 53 | S | O | H | H | H | H | H |
| 54 | S | NH | H | H | H | H | H |
| 55 | S | NH.HCl | H | H | H | H | H |
| 56 | S | O | H | H | H | H | H |
| 57 | O | O | H | H | H | H | H |
| 58 | O | NH | H | H | H | H | H |
| 59 | O | S | H | H | H | H | H |
| 60 | O | NH.HCl | H | H | H | H | H |
| 61 | Se | O | H | H | H | H | H |
| 62 | Se | NH | H | H | H | H | H |
| 63 | Se | S | H | H | H | H | H |
| 64 | NH | NH.HCl | H | H | H | H | H |
| 65 | NH | S | H | H | H | H | H |
| 66 | O | O | 4-Cl | H | H | H | H |
| 67 | O | O | 4-Cl | H | 7-OMe | H | H |
| 68 | O | O | 4-Me | H | H | H | H |
| 69 | O | O | H | 2-Cl | H | H | H |
| 70 | O | O | 4-Cl | 2-S-pPAA* | H | H | H |
| 71 | Se | O | 4-Cl | H | H | H | H |
| 72 | Se | O | 4-Cl | H | 7-OMe | H | H |
| 73 | Se | O | 4-Me | H | H | H | H |
| 74 | Se | O | 4-Cl | 2-S-pPAA* | H | H | H |
| 75 | N—$CH_3$ | O | 4-Cl | H | H | H | H |
| 76 | N—$C_6H_6$ | O | 4-Cl | H | 7-OMe | H | H |
| 77 | N—H | O | 4-Cl | 2-S-pPAA* | H | H | H |
| 78 | S | O | 4-Cl | H | H | H | H |
| 79 | SO | O | H | H | H | H | H |
| 80 | $SO_2$ | O | H | H | H | H | H |
| 81 | $SO_2$ | O | 4-Cl | H | H | H | H |

TABLE 1 continued

| Number | Y | X | $R_1$ | $R_2$ | $R_3$ | $R_4$ | T |
|---|---|---|---|---|---|---|---|
| 82 | N—Me | O | H | H | H | H | H |
| 83 | N—Me | O | 4-Cl | H | H | H | H |
| 84 | N—Me | O | 4-Cl | H | 7-OMe | H | H |
| 85 | N—Me | O | 4-Br | H | 7-OMe | 2-OMe | H |
| 86 | NCN | O | 4-Br | H | 7-OMe | 2-OMe | H |
| 87 | NCN | O | 4-Cl | H | H | H | H |
| 88 | NH | O | 2-Cl | H | H | H | H |
| 89 | NH | O | 4-Cl | H | H | H | H |
| 90 | S | O | 2-t-Bu | 4-t-Bu | H | H | H |
| 91 | S | O | 2-t-Bu | 4-t-Bu | 9-OMe | H | H |
| 92 | S | O | 2-t-Bu | 4-t-Bu | 7-F | H | H |
| 93 | S | O | 2-t-Bu | 4-t-Bu | 7-Me | H | H |
| 94 | S | O | 2-t-Bu | 4-t-Bu | 7-SMe | H | H |

*p-PAA = para-phenylacetic acid

TABLE 2

Compounds of the Formula

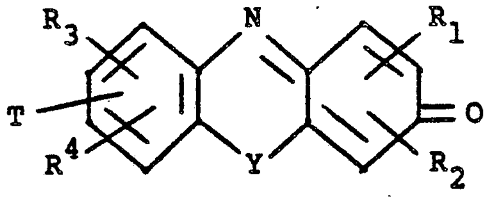

| Number | Y | $R_1$ | $R_2$ | $R_3$ | $R_4$ | T |
|---|---|---|---|---|---|---|
| 95 | S | H | H | H | H | H |
| 96 | S | 2-Cl | H | H | H | H |
| 97 | S | H | H | 6-Cl | H | H |
| 98 | S | H | H | 7-Cl | H | H |
| 99 | S | H | H | 8-Cl | H | H |
| 100 | S | H | H | 9-Cl | H | H |
| 101 | S | 1-Cl | H | H | H | H |
| 102 | S | 1-Cl | 4-Cl | H | H | H |
| 103 | S | 2-Cl | 4-Cl | H | H | 1-Cl |
| 104 | S | 2-N(Me)$_2$ | H | H | H | H |
| 105 | S | 2-SMe | H | H | H | H |
| 106 | S | 2-S-pPAA | H | H | H | H |
| 107 | S | 2-C(O)$CH_3$ | H | H | H | H |
| 108 | S | 2-OMe | H | H | H | H |
| 109 | S | H | H | H | 7-$CH_2CO_2H$ | H |
| 110 | S | H | H | H | 8-$CH_2COOH$ | H |
| 111 | S | H | 2-$SO_3$ | H | H | H |
| 112 | S | 2-N(Me)$_2$ | H | H | H | H |
| 113 | S | 2-SMe | H | H | H | H |
| 114 | S | 2-C(O)$CH_3$ | H | H | H | H |
| 115 | S | 2-OMe | H | H | H | H |
| 116 | S | 2-$CH_2CO_2H$ | H | H | H | H |
| 117 | S | 2-$CH(CH_3)CO_2H$ | H | H | H | H |
| 118 | S | 4-$CH_2COOH$ | H | H | H | H |
| 119 | S | 4-$CH(CH_3)CO_2H$ | H | H | H | H |
| 120 | S | H | H | 7-OH | 6-propyl | H |

TABLE 2 continued

| Number | Y | $R_1$ | $R_2$ | $R_3$ | $R_4$ | T |
|---|---|---|---|---|---|---|
| 121 | S | 4-Cl | H | H | H | H |
| 122 | S | 4-F | H | H | H | H |
| 123 | S | 4-F | H | 7-Cl | H | H |
| 124 | S | 4-Et | H | H | H | H |
| 125 | S | 4-Et | H | 7-OMe | H | H |
| 126 | S | 4-Et | H | 7-Cl | H | H |
| 127 | S | 4-Cl | H | 7-OMe | H | H |
| 128 | S | 4-OMe | H | 7-Cl | H | H |
| 129 | S | 4-Cl | H | 6-Cl | H | H |
| 130 | S | 4-Cl | H | 8-Cl | H | H |
| 131 | S | 4-Cl | H | 9-Cl | H | H |
| 132 | S | 4-Cl | H | 6-OMe | H | H |
| 133 | S | 4-Cl | H | 8-OMe | H | H |
| 134 | S | 4-Cl | H | 9-Et | H | h |
| 135 | S | 4-Cl | H | 6-Et | H | H |
| 136 | S | 4-Cl | H | 7-Et | H | H |
| 137 | S | 4-Cl | H | 8-Et | H | H |
| 138 | S | 4-Cl | 1-Et | H | H | H |
| 139 | S | 4-Cl | 2-Et | H | H | H |
| 140 | S | 4-Cl | 1-$CH_2COOH$ | H | H | H |
| 141 | S | 4-Cl | 2-$CH_2COOH$ | H | H | H |
| 142 | S | 4-Cl | H | 6-$CH_2COOH$ | H | H |
| 143 | S | 4-Cl | H | 7-$CH_2COOH$ | H | H |
| 144 | S | 4-Cl | H | 8-$CH_2COOH$ | H | H |
| 145 | S | 4-Cl | 2-$N(Me)_2$ | H | H | H |
| 146 | S | 4-Cl | 1-$N(Me)_2$ | H | H | H |
| 147 | S | 4-Cl | 2-$N(Me)_2$ | 7-OMe | H | H |
| 148 | S | 4-Cl | 2-$N(Me)_2$ | 7-Cl | H | H |
| 149 | S | 4-Cl | 2-SMe | H | H | H |
| 150 | S | 4-Cl | 2-$SCH_2COOH$ | H | H | H |

TABLE 2 continued

| Number | Y | $R_1$ | $R_2$ | $R_3$ | $R_4$ | T |
|---|---|---|---|---|---|---|
| 151 | S | 4-Cl | 2-S-pPAA | H | H | H |
| 152 | S | 4-Cl | 1-S-pPAA | H | H | H |
| 153 | S | 4-Cl | 2-S-pPAA | 7-OMe | H | H |
| 154 | S | 4-Cl | 2-$SO_3H$ | H | H | H |
| 155 | S | 4-Cl | 2-OMe | H | H | H |
| 156 | S | 4-Cl | 2-OMe | 7-Cl | H | H |
| 157 | S | 4-Cl | H | 7F | H | H |
| 158 | S | 4-OMe | H | 7-OMe | H | H |
| 159 | S | 4-OMe | H | 7-Me | H | H |
| 160 | S | 4-OMe | 2-SMe | H | H | H |
| 161 | S | 4-SMe | H | H | H | H |
| 162 | S | 4-Br | H | H | H | H |
| 163 | S | 4-I | H | H | H | H |
| 164 | S | 4-Br | H | 7-OMe | H | H |
| 165 | S | 4-I | H | 7-OMe | H | H |
| 166 | S | 4-Br | 2-Me | H | H | H |
| 167 | S | 4-I | 2-Me | H | H | H |
| 168 | S | 4-Cl | H | 7/8-$(CH_2)_4$— | | H |
| 169 | S | 4-Cl | H | 7/8-$(CH_2)_3$— | | H |
| 170 | S | 4-Br | 2-OMe | 7-OMe | H | H |
| 171 | S | 2-OMe | 7-OMe | H | H | H |
| 172 | S | 1-OMe | 7-OMe | H | H | H |
| 173 | S | 2-OMe | 7-OMe | H | H | 1-Br |
| 174 | S | 1-OMe | 7-OMe | H | h | 2-Br |
| 175 | S | 1-OMe | 7-OMe | H | H | 4-Br |
| 176 | S | 1-OMe | 7-OMe | H | H | 2-Cl |
| 177 | S | 1-OMe | 7-OMe | H | H | 4-Cl |
| 178 | S | 2-OMe | 7-OMe | H | H | 1-Cl |
| 179 | S | 2-OMe | 7-OMe | H | H | 4-Cl |
| 180 | S | 2-OEt | 7-OEt | H | H | 1-Br |

TABLE 2 continued

| Number | Y | $R_1$ | $R_2$ | $R_3$ | $R_4$ | T |
|---|---|---|---|---|---|---|
| 181 | S | 2-OEt | 7-OEt | H | H | 4-Br |
| 182 | S | 2-OEt | 7-OEt | H | H | 1-Cl |
| 183 | S | 2-OEt | 7-OEt | H | H | 4-Cl |
| 184 | S | 2-OMe | 7-OMe | 8-OMe | H | 1-Br |
| 185 | S | 2-OMe | 7-OMe | 8-OMe | H | 4-Br |
| 186 | S | 2-OMe | 7-OMe | H | H | 4-F |
| 187 | S | 2-OMe | 7-OMe | H | H | 4-$CF_3$ |
| 188 | S | 2-OMe | 7-OEt | H | H | 4-Br |
| 189 | S | 2-OMe | 7-OEt | H | H | 4-Cl |
| 190 | S | 2-OMe | 7-OEt | H | H | 4-F |
| 191 | S | 2-OMe | 7-OEt | H | H | 4-$CF_3$ |
| 192 | S | 2-OEt | 7-OMe | H | H | 4-Br |
| 193 | S | 2-OEt | 7-OMe | H | H | 4-Cl |
| 194 | S | 2-OEt | 7-OMe | H | H | 4-F |
| 195 | S | 2-OEt | 7-OMe | H | H | 4-$CF_3$ |
| 196 | S | 1-OMe | 2-OMe | 7-OMe | H | 4-Br |
| 197 | S | 1-OMe | 2-OMe | H | H | H |
| 198 | S | 1-OMe | 2-OMe | H | H | 4-Br |
| 199 | O | | Same as Numbers 90—198 | | | |
| 200 | $SO_2$ | 4-OH | H | H | H | H |
| 201 | $SO_2$ | 1-OMe | 2-OMe | 4-$CH_3$ | H | H |
| 202 | $SO_2$ | 2-OMe | 7-OMe | 4-OH | H | H |
| 203 | $SO_2$ | 2-OMe | 4-OH | H | H | H |
| 204 | $SO_2$ | 1-OMe | 4-OH | H | H | H |
| 205 | $SO_2$ | 2-Me | 4-OH | H | H | H |
| 206 | $SO_2$ | 2-Cl | 4-OH | H | H | H |
| 207 | $SO_2$ | 2-OEt | 7-OEt | 4-OH | H | H |
| 208 | $SO_2$ | 2-$SO_2$Me | 4-OH | H | H | H |
| 209 | $SO_2$ | 4-OMe | H | H | H | H |
| 210 | $SO_2$ | 2-OMe | 4-OMe | 7-OMe | H | H |

TABLE 2 continued

| Number | Y | $R_1$ | $R_2$ | $R_3$ | $R_4$ | T |
|---|---|---|---|---|---|---|
| 211 | O | 1-$CO_2H$ | 4-OH | 7-$NMe_2$ | H | H |
| 212 | O | 1-Cl | 2-Cl | 4-Cl | H | 7-Cl |
| 213 | S | 9-OMe | H | H | H | H |
| 214 | S | 2-OMe | H | H | H | H |
| 215 | S | 2-OMe | 4-OMe | H | H | H |
| 216 | S | 1-OMe | 2-OMe | 4-Me | H | H |
| 217 | S | 4-OMe | H | H | H | H |
| 218 | S | 1-OMe | 7-OMe | H | H | 4-Br |
| 219 | S | 1-OMe | 7-OMe | 2-Cl | H | 4-Cl |
| 220 | S | 1-OMe | 7-OMe | H | H | 4-Cl |
| 221 | S | 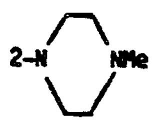 | 7-OMe | H | H | H |
| 222 | S | 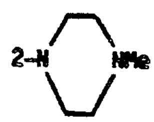 | 7-OMe | H | H | 4-Br |
| 223 | $SO_2$ | 2-OMe | 4-OH | 7-OMe | H | H |
| 224 | $SO_2$ | 1-NHPr | 4-NHPr | H | H | H |
| | $SO_2$ | | | | | |
| 225 | $SO_2$ | 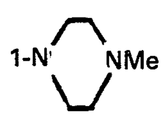 | 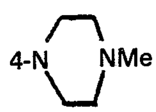 | H | H | H |
| 226 | $SO_2$ | 2-OMe | 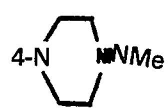 | 7-OMe | H | H |
| 227 | $SO_2$ | 2-OMe | 7-OMe | H | H | H |
| 228 | $SO_2$ | 2-OMe | 4-NHPr | 7-OMe | H | H |
| 229 | S | 1-NHPr | 4-NHPr | H | H | H |
| 230 | S | 1-NHPr | 4-NHPr | 7-OMe | H | H |
| 231 | S | 1-NHPr | 4-NHPr | H | H | H |
| 232 | S | 2-NHPr | 4-NHPr | 7-OMe | H | H |
| 233 | S | 2-OMe | 4-$NH_2$ | 7-OMe | H | H |
| 234 | S | 2-OMe | 4-NHPr | 7-OMe | H | H |

TABLE 2 continued

| Number | Y | $R_1$ | $R_2$ | $R_3$ | $R_4$ | T |
|---|---|---|---|---|---|---|
| 235 | O | 1-OMe | 4-Cl | 7-OMe | H | H |
| 236 | O | 1-OMe | 4-Br | 7-OMe | H | H |
| 237 | O | 1-NHPr | 4-NHPr | H | H | H |
| 238 | $SO_2$ | 1-OMe | 4-CN | 7-OMe | H | H |
| 239 | $SO_2$ | 2-OMe | 4-$NHCH_2CO_2R$* | 7-OMe | H | H |
| 240 | $SO_2$ | 2-OMe | 4-S-7-Bu | 7-OMe | H | H |
| 241 | $SO_2$ | 2-OMe | 4-$CH_2CO_2R$* | 7-OMe | H | H |
| 242 | $SO_2$ | 2-OMe | 4-$SO_2Me$ | 7-OMe | H | H |
| 243 | S | 2-S-n-Bu | H | H | H | H |
| 244 | S | 4-S-n-Bu | H | H | H | H |
| 245 | S | 2-Me | 4-S-n-Bu | H | H | H |
| 246 | S | 2-OMe | 7-Me | H | H | 4-Br |
| 247 | S | 2-OMe | 7-$CF_3$ | H | H | 4-Br |
| 248 | S | 2-OMe | 7-F | H | H | 4-Br |
| 249 | S | 2-OMe | 7-Cl | H | H | 4-Br |
| 250 | S | 2-OMe | 7-Br | H | H | 4-Br |
| 251 | S | 2-OMe | 7-$NMe_2$ | H | H | 4-Br |
| 252 | S | 2-OMe | 7-SMe | H | H | 4-Br |
| 253 | S | 2-OMe | 7-$SO_2Me$ | H | H | 4-Br |
| 254 | S | 2-OMe | 7-Ph | H | H | 4-Br |
| 255 | S | 1-Me | H | H | H | H |
| 256 | S | 2-Me | H | H | H | H |
| 257 | S | 2-OEt | H | H | H | H |
| 258 | S | 7-Cl | H | H | H | H |
| 259 | S | 9-Cl | H | H | H | H |
| 260 | S | 7-F | H | H | H | H |
| 261 | S | 7-Me | H | H | H | H |
| 262 | S | 7-OMe | H | H | H | H |
| 263 | S | 2-Cl | H | H | H | H |

*R is H or $C_1C_4$ alkyl.

TABLE 2 continued

| Number | Y | $R_1$ | $R_2$ | $R_3$ | $R_4$ | T |
|---|---|---|---|---|---|---|
| 264 | S | 1-Me | 7-Me | H | H | H |
| 265 | S | 1-Me | 7-Me | H | H | H |
| 266 | S | 2-OMe | 7-OEt | H | H | H |
| 267 | O | 2-$NH_2$ | H | H | H | H |
| 268 | O | 7-OH | H | H | H | H |
| 269 | O | COMe | H | H | H | H |
| 270 | $SO_2$ | 2-OMe | 7-OMe | H | H | 4-Br |
| 271 | S | 2-NHPr | 4-NHPr | H | H | H |
| 272 | S | 2-Cl | H | H | H | 4-Cl |
| 273 | S | 1-Me | 7-Me | H | H | 4-Cl |

TABLE 3

Compounds of the Formula

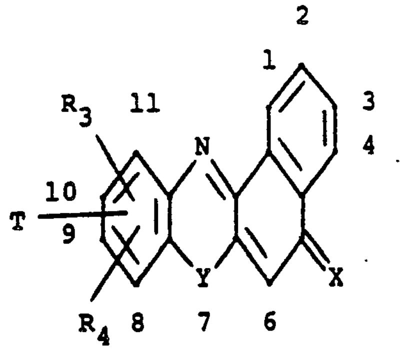

| Example | Y | X | $R_3$ | $R_4$ | T |
|---|---|---|---|---|---|
| 1 | O | O | H | H | H |
| 2 | S | O | H | H | H |
| 3 | SO | O | H | H | H |
| 4 | $SO_2$ | O | H | H | H |
| 5 | SO | O | H | H | 6-Cl |
| 6 | S | O | 6-$COCH_3$ | H | H |
| 7 | S | O | 6-$CH_3$ | H | H |
| 8 | $SO_2$ | O | 6-OH | H | H |
| 9 | $SO_2$ | O | 6-OMe | H | H |
| 10 | S | O | 9-OMe | H | H |
| 11 | S | O | 6-OH | H | H |
| 12 | S | O | 6-OMe | H | H |
| 13 | S | O | 6-NHCOMe | H | H |
| 14 | S | O | 6-NHPh | H | H |
| 15 | S | O | H | H | 6-Br |
| 16 | S | O | 6-NHMe | H | H |
| 17 | S | O | 6-NH-t-Bu | H | H |
| 18 | S | O | 6-NH-COMe | H | 9-Cl |
| 19 | S | O | 6-NH-COMe | 9-Ome | H |
| 20 | S | O | 6-NHPh-p-Br | H | 9-Cl |
| 21 | O | O | H | H | 6-Cl |
| 22 | O | O | H | H | 6-Br |
| 23 | O | O | 9-OMe | H | 6-Br |

TABLE 3 continued

| Example | Y | X | $R_3$ | $R_4$ | T |
|---|---|---|---|---|---|
| 24 | O | O | 9-OMe | 6-NHPr | H |
| 25 | S | O | 6-$CF_3$ | H | H |
| 26 | S | O | 6-S-n-Bu | H | H |
| 27 | S | O | 6-OMe | H | 9-Cl |
| 28 | S | O | 9-OMe | H | 6-Cl |
| 29 | S | O | 6-OMe | 9-OMe | H |
| 30 | S | O | 6-Cl | 9-Me | 11-Br |
| 31 | S | O | 6-NHPh | 9-Me | 11-Br |
| 32 | S | O | 6-Me | H | H |
| 33 | O | NH | 9-$NMe_2$ | 10-Me | H |
| 34 | O | NH | 9-$N(Et)_2$ | H | H |

A quite specific embodiment of the present invention is represented by the tripeptide γ-Glutamylcysteylglycine derivatives: 2-5-glutathionyl-3H-phenothiazin-3-one and 4-chloro-2-5-glutathionyl-phenothiazine-3-one.

The compounds of the Formula I have unexpected activity as inhibitors of the mammalian biosynthesis of leukotriene $B_4$, as well as leukotrienes $C_4$, $D_4$, $E_4$ and $F_4$, the active elements of the slow reacting substance of anaphylaxis (SRS—A). The compounds of Formula I act as inhibitors of the mammalian 5-Lipoxygenase enzyme system of the arachidonic acid cascade. This inhibition of the mammalian biosynthesis of leukotrienes indicated that the compositions would be useful to treat, prevent or ameliorate, in mammals and especially in humans 1) pulmonary conditions including diseases such as ashma, 2) allergies and allergic reactions such as allergic rhinitis, contact dermatitis, allergic conjuntivitis and the like, 3) inflammation such as arthritides, 4) pain, 5) skin conditions such as psoriasis and the like and 5) cardiovascular conditions such as angina.

Representative compounds of Formula I have been tested using one or more of the following assays to determine their mammalian leukotriene biosynthesis inhibiting activity and other relevant activities.

RBL—1 5-Lipoxygenase

Rat basophilic leukemia (RBL—1) cells were sonicated and centrifuged at 125000 xg. The resulting supernatant fraction was incubated with arachidonic acid (labelled with $^{14}C$) to convert a portion of it to $^{14}C$—5(S)-hydroxyicosatetraenoic acid (5—HETE). Compounds being evaluated as inhibitors of 5-Lipoxygenase were added prior to the addition of arachidonic acid. 5—HETE was isolated by extraction and paper chromatography, and quantitated by determining the amount of radioactivity (cpm) associated with 5-HETE.

Reference: Egan, R. W., Tischler, A. M. Baptista, E. H., Ham, E. A., Soderman, D. D., and Gale, P. H., *Advances in Prostaglandin, Thromboxane and Leukotriene Research 11* 151 (1983), (Samuelson, B., Ramwell, P. W., and Paoletti, R. (eds.), Raven Press, N.Y.

Mouse Macrophage Assay

Mouse peritoneal macrophages were treated sequentially with arachidonic acid (labelled with tritium); the compound being evaluated as an inhibitor, and a stimulator (zymosan). Metabolites derived from arachidonic acid ($PGE_2$, 6-keto PG-$F_{1\alpha}$ and leukotriene $C_4$) were separated from the incubation medium by extraction and chromatography, and then quantitated by determining the amount of radioactivity (Cpm) associated with each of them. Inhibitors caused a reduction in the amount of radioactivity (cpm) associated with a given metabolite. (This protocol is identical to that described in the reference exept that the radioactivity herein associated with the $LTC_4$ was determined by counting an aliquot of the final aqueous solution directly rather than chromatographing it first).

Reference: Humes, J. L. *et al., J. Biol. Chem. 257,* 1591—4 (1982).

Antigen Challenge 'in vitro' Assay

Male guinea pigs weighing 300—350 g were sensitized by injecting (I.P.) ml of a suspension containing 0.4 mg of egg albumin (Ovalbumin, Grade V, Sigma Chemical Co.) and 4.0 g aluminum hydroxide in 19.6 ml of saline. Two weeks were permitted for sensitization to occur.

Three sensitized guinea pigs were stunned and exsanguinated. The tracheas were removed, freed of adhering tissue and divided longitudinally by cutting through the cartilaginous tissue directly opposite the muscle insertion. Each opened trachea was then transected between every second cartilage. Four of the cut sections were tied together, end to end, in a series with a No. 7 silk thread ensuring that the tracheal muscles were all in the same vertical plane. Thus, each chain consisted of tissue from three different animals.

The chain so formed was then suspended under 1 g of tension (by silk ties at each end) in a 20 ml organ bath containing 10 ml of modified* Krebs-Henseleit buffer solution gassed with 95% $O_2$ and 5% $CO_2$ at 37°C. Mepyramine (0.55 μg/ml) and indomethacin (2.67 μg/ml) were added to the buffer to avoid the contribution of histamine receptors and cyclooxygenase products to the contraction. To record responses one end of the tracheal chain was attached to a Gould-Statham UC—2 force displacement transducer which was connected to a Beckman Type R-dynograph. The preparations were allowed to equilibrate for one hour during which time the tissues were automatically washed (10 ml volume displacement) every 6 minutes.

After the equilibration period the tissues were primed with methacholine (3 μg/ml; $1.5 \times 10^{-5}$M), washed and allowed to recover to baseline. The tissues were treated again with a second dose of methacholine, washed, allowed to return to baseline and washed for an additional hour.

Two chains were used as a control. These were incubated in a concentration of egg albumin sufficient to induce an average contraction of 50—80% of the methacholine response.

Each compound to be tested was added to two other baths (at a final concentration in each bath of 10 μg/ml) 15 minutes prior to challenging the fresh chains with egg albumin.

The response of the challenged tissue was expressed as a percentage of the methacholine maximum. The % inhibition for each compound was then calculated. Compounds which at 10 μg/ml (final conc.) inhibited the egg albumin response by 50% or more were retested at a lower concentration.

Asthmatic Rat Assay

Rats were obtained from an inbred line of asthmatic rats. Both female and male rats from 200 to 300 g were used.

Egg albumin (EA), grade V, crystallized and lyophilized, was obtained from Sigma Chemical Co., St Louis. *Bordetella pertussis* vaccine, containing $30 \times 10^9$ killed bacteria per ml was obtained from the Institut Armand-Frappier, Laval des Rapides, Quebec. Aluminum hydroxide was obtained from the Regis Chemical Company, Chicago.

The challenge and subsequent respiratory recordings were carried out in a clear plastic box with internal dimensions 10 × 6 × 4 inches. The top of the box was removable; in use, it was held firmly in place by four clamps and an airtight seal was maintained by a soft rubber gasket. Through the center of each end of the chamber a Devilbiss nebulizer (No. 40) was inserted via an airtight seal and each end of the box also had an outlet. A Fleisch No. 0000 pneumotachograph was inserted into one end of the box and coupled to a Grass volumetric pressure transducer (PT5—A) which was then connected to a Beckman Type R Dynograph through appropriate couplers. While aerosolizing the antigen, the outlets were open and the pneumotachograph was isolated from the chamber. The outlets were closed and the pneumotachograph and the chamber were connected during the recording or the respiratory patterns. For challenge, 2 ml of a 3% solution of antigen in saline was placed into each neubulizer and the aerosol was generated with air from a small Potter diaphragm pump operating at 10 psi and a flow of 8 liters/minute.

Rats were sensitized by injecting (s.c.) 1 ml of a suspension containing 1 mg EA and 200 mg aluminum hydroxide in saline. Simultaneously, they received an injection (i.p.) of 0.5 ml of *B. pertussis* vaccine. They were used between days 14 and 18 postsensitization. In order to eliminate the serotonin component of the response, rats were pretreated intravenously 5 minutes prior to aerosol challenge with 30 mg/kg methylserzide. Rats were then exposed to an aerosol of 3% EA in saline for exactly 1 minute, then their respiratory profiles were recorded for a further 25—30 minutes. The duration of continuous dyspnoea was measured from the respiratory recordings.

Compounds were generally administered either intraperitoneally 1 hour prior to challenge or orally 1½ hours prior to challenge. They were either dissolved in dimethylsulfoxide or suspended in 0.1% methocel and 0.5% Tween 80. The volume injected has 2 ml/kg (intraperitoneally) or 10 ml/kg (orally). Prior to oral treatment rats were starved overnight. Their activity was determined in terms of their ability to decrease the duration of symptoms of dyspnoea in comparison with a group of vehicle-treated controls. Usually, a compound was evaluated at a series of doses and an $ED_{50}$ was determined. This was defined as the dose (mg/kg) which would inhibit the duration of symptoms by 50%.

---

*modified Krebs solution in grams/liter and (mM):
NaCl — 6.87 (120); glucose — 2.1 (11); $NaHCO_3$ — 2.1 (25); KCl — 0.32 (4.72); $CaCl_2$ — 0.28 (2.5); $MgSO_4.7H_2O$ — 0.11 (0.5); $KH_2PO_4$ — 0.16 (1.2); pH at bathing solution = 7.35 ± 0.05.

PAF-Induced Hyperalgesia Assay

Female Sprague-Dawley rats, 35—40 g were fasted overnight. Platelet activating factor, PAF, (L-lecithin B-acetyl O-alkyl) 1 µg/0.1 ml was given by subplantar injection in the rat paw. The compounds to be evaluated were homogenized in Aqueous Vehicle (0.9% benzyl alcohol, 0.5% Tween 80 and 0.4% methylcellulose) and administered orally in a volume of 0.1 ml, 30 minutes prior to PAF.

Animals were tested 1, 2, 3 and 4 hours after PAF administration. The vocalization threshold, defined as the pressure (mmHg) needed to evoke a squeak response, was recorded for both the injected and contralateral paw. No animal was subjected to pressure greater than 60 mmHg. Hyperalgesia is defined as a decrease in vocalization threshold as compared to a normal paw. Percent inhibition of hyperalgesia was calculated as the proportion of animals with vocalization thresholds greater than 200% of controls.

Brewer's Yeast Hyperalgesia Assay

The standard method* for yeast hyperalgesia was used. Female Sprague-Dawley rats, 35—40 g were fasted overnight. A 5% solution (volume 0.1 ml) of Brewer's yeast was injected into the rat paw. The compound was homogenized in aqueous vehicle and given orally 2 hours after yeast. Vocalization thresholds were recorded 1 hours after drug (3 hours after yeast). Percent inhibition of hyperalgesia was determined by the proportion of animals with vocalization thresholds greater than 25 mmHg.

---

* Winter, C. A. *et al., J. Pharm. Exp. Ther. 150,* 165—171 (1965).

Following is data obtained using these various assays with representative compounds of Formula I.

TABLE 4

Assay Results

| Test No. | Compound | Macrophage Ic50 (µg/ml) | RBL-1 Ic50 (µg/ml) | In Vitro Antigen Challenge Test µg/ml and % Inhibition |
|---|---|---|---|---|
| 1 | 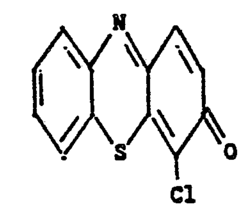 | 0.2 | 0.01 | 10 µg (100%)<br>1 µg (44%) |
| 2 | 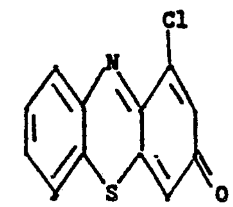 | 0.5 | 0.01 | 10 µg (68%) |
| 3 | 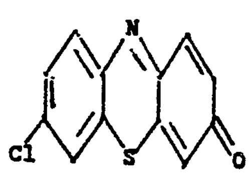 | 0.2 | — | 10 µg (25%) |
| 4 | 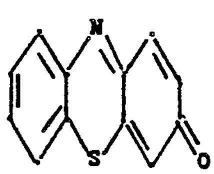 | 0.1 | 0.01 | 10 µg (100%)<br>1 µg (77%)<br>0.3 µg (42%) |

TABLE 4 continued

| Test No. | Compound | Macrophage Ic50 (µg/ml) | RBL-1 Ic50 (µg/ml) | In Vitro Antigen Challenge Test µg/ml and % Inhibition |
|---|---|---|---|---|
| 5 | 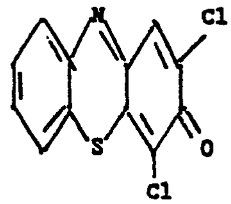 | 0.1—0.5 | 65% at 0.05 µ g/ml | 10 µg (45%) |
| 6 | 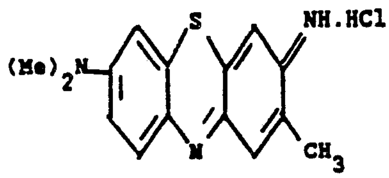 | 10% at 0,05 µg/ml | | 10 µg 24% |
| 7 | 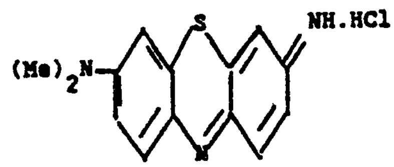 | 15% at 0.5 µg/ml | — | 10 µg 37% |
| 8 | 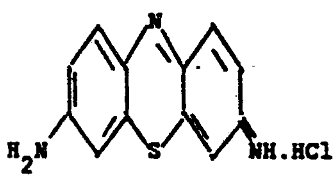 | 33% at 0.1 µg/ml | — | — |
| 9 | 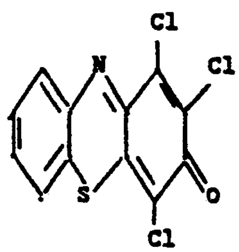 | 75% at 5 µg/ml | — | — |
| 10 | 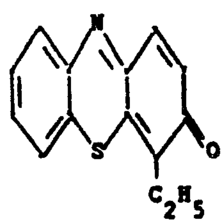 | — | — | 10 µg (100%) |
| 11 | 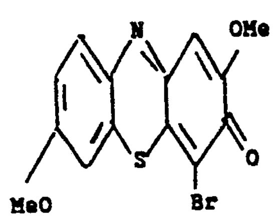 | 0.1 | — | 3 µg (23%) |

TABLE 4 continued

| Test No. | Compound | Macrophage Ic50 (µg/ml) | RBL-1 Ic50 (µg/ml) | In Vitro Antigen Challenge Test µg/ml and % Inhibition |
|---|---|---|---|---|
| 12 | 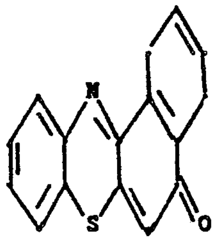 | 0.1 | — | 10 µg (55%) |
| 13 | 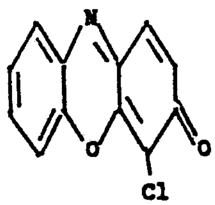 | — | — | 3 µg (54%) |
| 14 | 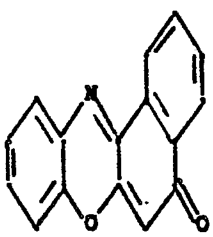 | 0.1 | — | — |

TABLE 5

Asthmatic Rat Assay Results

| Test No. | Compound | Method of Administration | $Ed_{50}$ |
|---|---|---|---|
| A | 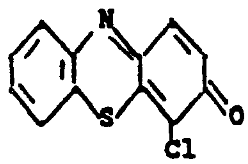 | i.p. | 0.5 mg/kg |
| B | 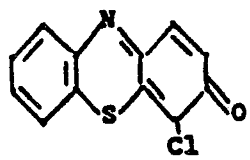 | p.o. | 1.5 mg/kg |
| C | 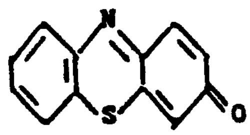 | i.p. | about 5.0 mg/kg |
| D | 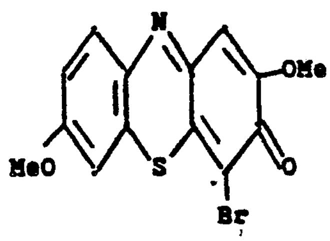 | p.o. | 1.0 mg/kg |
| E | 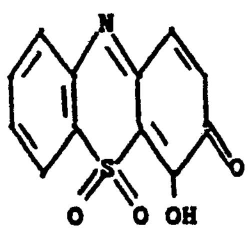 | p.o. | 37% inhibition at 1.5 mg/kg |
| F | 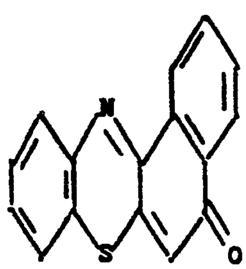 | p.o. | 36% inhibition at 5 mg/kg |

TABLE 6

PAF-induced Hyperalgesia Assay for Compound of Example 2

Experiment 1

| | Vocalization Threshold[a] | % Inhibition |
|---|---|---|
| 0.01 mg/kg p.o. | 28.2 ± 5.2 | 40 |
| 0.03 | 40.2 ± 5.0 | 80 |
| 0.1 | 37.4 ± 3.9 | 80 |
| 0.3 | 45.4 ± 3.7 | 90 |
| 3.0 | 46.0 ± 3.0 | 100 |
| Control | 14.4 ± 1.6 | |

Experiment 2

| | Vocalization Threshold[a] | % Inhibition |
|---|---|---|
| 0.001 mg/kg p.o. | 10.0 ± 1.6 | 0 |
| 0.003 | 16.4 ± 2.8 | 30 |
| 0.01 | 18.0 ± 2.9 | 30 |
| 0.1 | 29.6 ± 4.4 | 60 |
| Control | 11.2 ± 1.4 | |

[a] mmHg Mean ± S.E.M., n = 10 Reading taken 3 hr. after injection of PAF, (3.5 hr. after administration of compound).

TABLE 7

Rat Brewer's Yeast Hyperalgesia Assay for Example 2 Compound

| Dose | Vocalization Threshold[a] | % Inhibition |
|---|---|---|
| 0.3 mg/kg p.o. | 17.6 3.1 | 30 |
| 1.0 | 19.4 3.1 | 30 |
| 3.0 | 30.0 5.8 | 60 |
| 10.0 | 28.6 3.0 | 70 |
| 30.0 | 32.6 3.3 | 80 |
| Control | 10.2 1.3 | |

[a] mmHg: mean ± S.E.M. n = 10

The test results presented above show that representative compounds of Formula I inhibit the mammalian biosynthesis of leukotrienes especially via the 5-Lipoxygenase pathway of arachidonic acid metabolism and have representative pharmaceutical utility e.g., for asthma, pain and allergy.

The pharmaceutical compositions of the present invention will contain sufficient compound of Formula I in a dosage form suitable for inhibiting the mammalian biosynthesis of leukotrienes or, for the treatment desired. The effective concentration of the Formula I compound in the composition will vary as required by the mode of administration, dosage form and pharmacological effect and level desired. A general daily dosage of Formula I will range from 10 µg to 20 mg/kg of body weight. This dosage may be administered in single or divided individual doses. More or less of the general daily dosage may be necessary depending upon the individual needs of the patient.

For treating pulmonary conditions such as asthma, the mode of administration may be oral, parenteral, by inhalation, or by suppository. Suitable oral dosage forms are tablets, elixirs, emulsions, solutions and capsules, including delayed or sustained release capsules. Parenteral dosage forms include solutions and emulsions. Dosage forms for administration by inhalation include sprays and aerosols. These inhalation formulations may be administered in metered doses ranging from 0.1 µg to 200 µg, administered as needed.

For treating allergies or allergic reactions, such as allergic conjunctivitis and allergic rhinitis, the Formula I compound may be administered by any conventional mode, eg., orally, parenterally, topically, subcutaneously or by inhalation. The oral and parenteral dosage forms are the same type as for the pulmonary treatment. The topical application dosage forms include ointments, salves, controlled-release patches, emulsions, solutions, thixotropic formulations, powders and sprays. For topical application, the percent by weight active ingredient (Formula I compound) may vary from 0.001 to 10%.

For treating inflammation the mode of administration may be oral, parenteral or by suppository. The various dosage forms are the same as those described above.

For treating skin diseases such as psoriasis and atopic dermatitis, oral, topical or parenteral administration is useful. For topical application to the diseases area salves, patches, controlled release patches and emulsions are convenient dosage forms.

For use as an analgesic, i.e., for treating pain, any suitable mode of administration may be used, e.g., oral, parenteral, by insufflation or by suppository.

For treating cardiovascular conditions such as angina pectoris, any suitable mode of administration, e.g. oral, parenteral, topical or by insufflation, and dosage form, e.g. pills, liquid formulations, controlled release capsules or controlled release skin patches, may be used.

In addition to the common dosage forms set out above, the compound of Formula I may also be administered for the various utilities and indications or for inhibiting leukotriene synthesis by controlled release means and/or delivery devices such as those described in U.S. Patent Specifications US—A—3,845,770; 3,916,899; 3,536,809; 3,598,123; 3,630,200 and 4,008,719.

Dosage forms for application to treat the eye are also disclosed in U.S. Patent Specification US—A—4,348,398.

In preparing suitable dosage forms, conventional compounding procedures and ingredients e.g. diluents or carriers, may be used. The following are examples of representative pharmaceutical dosage forms:

| Injectible Suspension | mg/mL |
|---|---|
| Compound of Example C | 1—100 |
| Methylcellulose | 5.0 |
| Tween 80 | 0.5 |
| Benzyl alcohol | 9.0 |
| Methyl paraben | 1.8 |
| Propyl paraben | 0.2 |
| Water for injection to a total volume of 1 ml | |

| Aerosol for Oral Inhibition | mg/can (200 doses/can) |
|---|---|
| Compound of Formula I | 2—40 |
| Oleic Acid | 0.2—4.0 |
| Trichloromonofluoro methane | 5,000—8,000 To a total |
| Dichloromonofluoro methane | 15,000—12,400 of 20,400 |

| Cream | mg/g |
|---|---|
| Compound of Formula I | 1—100 |
| Cetyl alcohol | 130.0 |
| Sodium Lauryl Sulfate | 15.0 |
| Propylene Glycol | 100.0 |
| Methyl paraben | 1.8 |
| Propyl paraben | 1.2 |
| Purified Water of sufficient quantity to make total 1 g | |

| Ointment | mg/g |
|---|---|
| Compound of Formula I | 1—100 |
| Methyl paraben | 1.8 |
| Propyl paraben | 1.2 |
| Petrolatum of sufficient quantity to make total 1 g | |

| Tablet | mg/tablet |
|---|---|
| Compound of Formula I | 0.2—350 |
| Microcrystalline Cellulose | 0—349.8 |
| Povidone | 14.0 |
| Microcrystalline Cellulose | 90.0 |
| Pregelatinized Starch | 43.5 |
| Magnesium Stearate | 2.5 |
| | 500 |

| Capsule | mg/capsule |
|---|---|
| Compound of Formula I | 0.2—350 |
| Lactose Powder | 248.5—598.3 |
| Magnesium Stearate | 1.5 |
| | 600 |

In addition to the compounds of Formula I, the pharmaceutical compositions can also contain other active ingredients, such as non-steroidal anti-inflammatory agents e.g. indomethacin, ibuprofen, sulindac and fenbufen, peripheral analgesic agents such as zomepirac and diflunisal, or cyclooxygenase inhibitors. They may also contain leukotriene antagonists such as those disclosed in European Patent Specifications EP—A—0,106,565, EP—A—0,104,885, EP—A—0,056,172 and EP—A—0,061,800 and in U.K. Patent Specification GB—A—2,058,785. These pharmaceutical compositions may also contain as the second active ingredient, antihistaminic agents such as benadryl, dramamine, histadyl and phenergan. Alternatively, they may include prostaglandin antagonists such as those disclosed in EP—A—0,011,067 or thromboxane antagonists such as those disclosed in US—A—4,237,160. The compounds of Formula I may also be advantageously combined with an $H_1$ or $H_2$-receptor antagonist, such as for instance cimetidine, ranitidine or terfenadine, or those disclosed in US—A—4,283,408; 4,362,736 and 4,394,508; and EP—A—0,040,696. These pharmaceutical compositions containing Formula I compound and a second

active ingredient are another embodiment of the present invention. The weight ratio of the Formula I compound to the second active ingredient may be varied and may range from 10:1 to 1:10.

Another embodiment of the present invention are novel compounds encompassed by Formula I. These compounds have the Formula:

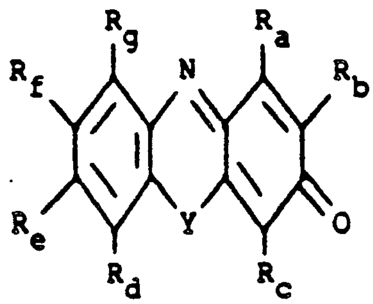

Tables 8 and 9 describe the novel compounds of the present invention:

TABLE 8

Novel Formula I Compound

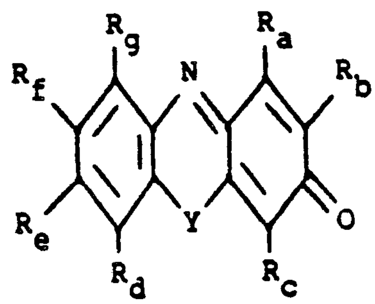

| Y | $R_a$ | $R_b$ | $R_c$ | $R_d$ | $R_e$ | $R_f$ | $R_g$ |
|---|---|---|---|---|---|---|---|
| S | H | $SCH_3$ | H | H | H | H | H |
| S | H | H | $SCF_3$ | H | H | H | H |
| S | H | H | CHO | H | H | H | H |
| S | H | H | $COCF_3$ | H | H | H | H |
| S | H | H | H | H | $SCH_3$ | H | H |
| S | H | H | H | H | $CO_2CH_3$ | H | H |
| S | H | H | H | H | $CO_2H$ | H | H |
| S | H | H | H | H | CHO | H | H |
| S | H | H | H | H | $CONH_2$ | H | H |
| S | H | H | H | H | $CH_2OH$ | H | H |
| S | H | H | Cl | H | $CO_2Me$ | H | H |
| S | H | H | Cl | H | $CO_2H$ | H | H |
| S | H | H | Cl | H | CHO | H | H |
| S | H | H | Cl | H | $CONH_2$ | H | H |
| S | H | H | Cl | H | $CH_2OH$ | H | H |
| S | H | O-benzyl | Cl | H | H | H | H |

TABLE 8 continued

| Y | $R_a$ | $R_b$ | $R_c$ | $R_d$ | $R_e$ | $R_f$ | $R_g$ |
|---|---|---|---|---|---|---|---|
| S | H | OEt | H | H | $CH_3$ | H | H |
| S | $CH_3$ | H | Cl | H | $CH_3$ | H | H |
| S | H | $CH_3$ | Cl | H | $CH_3$ | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | h | SMe | H | H |
| O | H | OMe | Br | H | OMe | H | H |
| O | H | OMe | Cl | H | OMe | H | H |
| S | H | $CH_3$ | I | H | H | H | h |
| $SO_2$ | H | H | OH | H | H | H | H |
| $SO_2$ | H | OMe | OH | H | OMe | H | H |
| $SO_2$ | OMe | OMe | Me | H | H | H | H |
| $SO_2$ | H | H | OMe | H | H | H | H |
| $SO_2$ | H | OMe | OM | H | OMe | H | H |
| S | $OCH_3$ | $OCH_3$ | Me | H | H | H | H |
| S | H | H | $COCH_3$ | H | H | H | H |
| S | $OCH_3$ | H | Br | H | $OCH_3$ | H | H |
| S | $OCH_3$ | Cl | Cl | H | $OCH_3$ | H | H |
| S | $OCH_3$ | H | Cl | H | $OCH_3H$ | H | H |
| S | H | 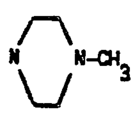 | H | H | $OCH_3$ | H | H |
| S | H | 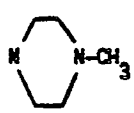 | Br | H | $OCH_3$ | H | H |
| $SO_2$ | H | $OCH_3$ | OH | H | $OCH_3$ | H | H |
| $SO_2$ | NHPr | H | NHPr | H | H | H | H |
| $SO_2$ | 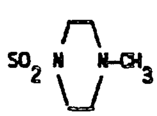 | H | 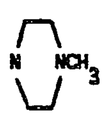 | H | H | H | H |

TABLE 8 continued

| Y | $R_a$ | $R_b$ | $R_c$ | $R_d$ | $R_e$ | $R_f$ | $R_g$ |
|---|---|---|---|---|---|---|---|
| $SO_2$ | H | $OCH_3$ | 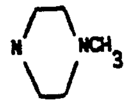 | H | $OCH_3$ | H | H |
| $SO_2$ | H | $OCH_3$ | Br | H | $OCH_3$ | H | H |
| S | NHPR | H | NHPr | H | H | H | H |
| S | NHPr | N | NHPr | H | $OCH_3$ | H | H |
| S | H | NHPr | NHPr | H | H | H | H |
| S | H | NHPr | NHPr | H | $OCH_3$ | H | H |
| S | H | $OCH_3$ | $NH_2$ | H | $OCH_3$ | H | H |
| S | H | $OCH_3$ | NHPr | H | $OCH_3$ | H | H |
| $SO_2$ | H | $OCH_3$ | NHPr | H | $OCH_3$ | H | H |
| O | $OCH_3$ | H | Cl | H | $OCH_3$ | H | H |
| O | $OCH_3$ | H | Br | H | $OCH_3$ | H | H |
| O | NHPr | H | NHPr | H | H | H | H |
| $SO_2$ | H | $OCH_3$ | CN | H | $OCH_3$ | H | H |
| $SO_2$ | H | $OCH_3$ | $NHCH_2CO_2R$* | H | $OCH_3$ | H | H |
| $SO_2$ | H | $OCH_3$ | S-N-Bu | H | $OCH_3$ | H | H |
| $SO_2$ | H | $OCH_3$ | $CH_2CO_2R$* | H | $OCH_3$ | H | H |
| $SO_2$ | H | $OCH_3$ | $SO_2CH_3$ | H | $OCH_3$ | H | H |
| S | H | S-n-Bu | H | H | H | H | H |
| S | H | H | S-n-Bu | H | H | H | H |
| S | H | $CH_3$ | S-n-Bu | H | H | H | H |
| S | H | OMe | Br | H | $CF_3$ | H | H |
| S | H | OMe | Br | H | F | H | H |
| S | H | OMe | Br | H | Cl | H | H |
| S | H | OMe | Br | H | Br | H | H |
| S | H | OMe | Br | H | $NMe_2$ | H | H |
| S | H | OMe | Br | H | SMe | H | H |
| S | H | OMe | Br | H | $SO_2Me$ | H | H |
| S | H | OMe | Br | H | Ph | H | H |
| S | H | H | H | Cl | OMe | H | H |
| S | H | OMe | Br | H | Me | H | H |

* R is H or $C_1$—$C_4$ alkyl.

Table 9 describes the novel compounds of the present invention having four rings.

TABLE 9

Novel Compounds of Formula II

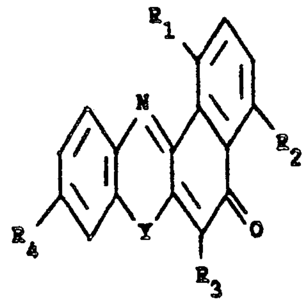

II

| Y | $R_1$ | $R_2$ | $R_3$ | $R_4$ |
|---|---|---|---|---|
| S | H | H | S-n-$CH_4H_9$ | H |
| S | OH | H | $CH_3$ | H |
| S | $OCH_3$ | H | $CH_3$ | H |
| S | H | H | F | H |
| S | H | H | $CF_3$ | H |
| S | H | H | Cl | $CF_3$ |
| S | H | H | Cl | $SCH_3$ |
| S | H | H | Br | Cl |
| S | H | H | $CH_3$ | Br |
| S | H | H | F | Br |
| S | H | H | $COCH_3$ | Cl |
| S | H | H | $CF_3$ | $CH_3$ |
| S | H | H | S-n-$C_4H_9$ | $CH_3$ |
| S | H | H | $CF_3$ | Cl |
| S | H | H | Cl | *$CH_2COOR$ |
| S | H | H | Cl | *$CH(Me)CO_2R$ |
| S | H | H | Cl | $COCH_3$ |
| S | H | H | H | Cl |
| S | H | H | H | Br |
| S | H | H | H | F |
| S | H | H | H | $CF_3$ |
| S | H | H | H | $CH_3$ |
| S | H | H | H | $CH_2OH$ |

TABLE 9 continued

| Y | $R_1$ | $R_2$ | $R_3$ | $R_4$ |
|---|---|---|---|---|
| S | H | H | H | $OCH_3$ |
| S | H | H | H | $SCH_3$ |
| S | H | H | H | *COOR |
| S | H | H | H | *$CH_2CO_2R$ |
| S | H | H | H | *$CH(Me)CO_2R$ |
| $SO_2$ | H | H | NHPr | H |
| $SO_2$ | H | H | 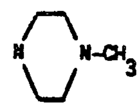 | H |
| $SO_2$ | H | H | $NH_2$ | H |
| $SO_2$ | H | H | NHPr | $OCH_3$ |
| S | -1,4-dihydro- | | H | |
| S | H | H | NHPr | $OCH_3$ |
| O | H | H | Cl | H |
| O | H | H | Br | H |
| O | H | H | Br | $OCH_3$ |
| O | H | H | NHPr | $OCH_3$ |

*R is H or $C_{1-4}$alkyl.

Table 10 describes additional novel compounds of the present invention.

TABLE 10

Novel Compounds of Formula III

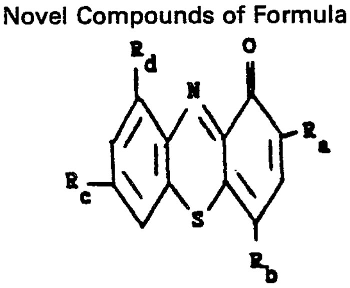

III

| $R_a$ | $R_b$ | $R_c$ | $R_d$ |
|---|---|---|---|
| t-Bu | t-Bu | H | h |
| t-Bu | t-Bu | F | H |
| t-Bu | t-Bu | Me | H |
| t-Bu | t-Bu | SMe | H |
| t-Bu | t-Bu | H | OMe |

Formula I includes both novel and known compounds. These compounds may be prepared by any process available to the skilled artisan.

One such process for compounds where X = 0 involves the oxidation of the appropriate phenothiazine as illustrated by the following equations.

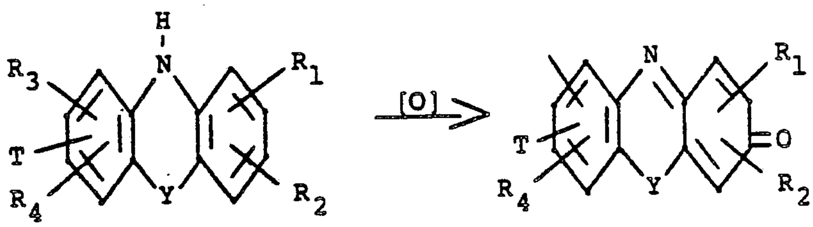


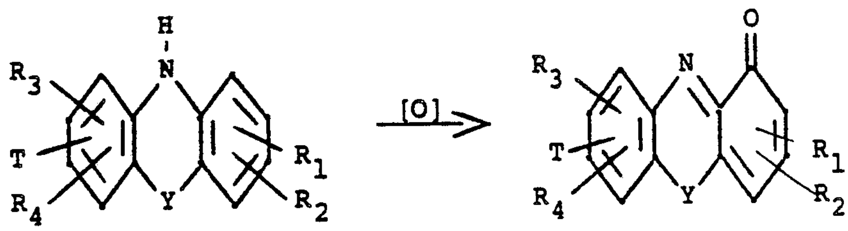


Various oxidizing agents and systems are taught in the art, e.g. $PbO_2$, $HNO_3$, $K_2Cr_2O_7$, $K_2Cr_2O_7$, iodine or $FeCl_3$.

Another process useful for preparing some Formula I compounds containing halogen substituents is by direct halogenation of an appropriate phenothiazone or analog thereof as illustrated by the following equation.

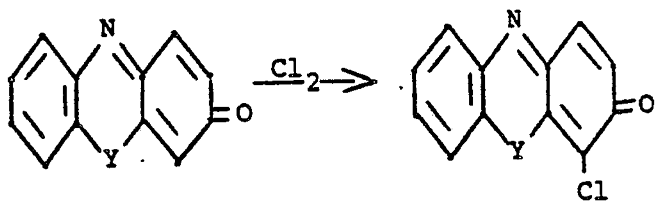


Still another process useful for preparing many of the Formula I compounds is by the reaction of an appropriate aniline with an appropriate quinone as illustrated by the following equation:

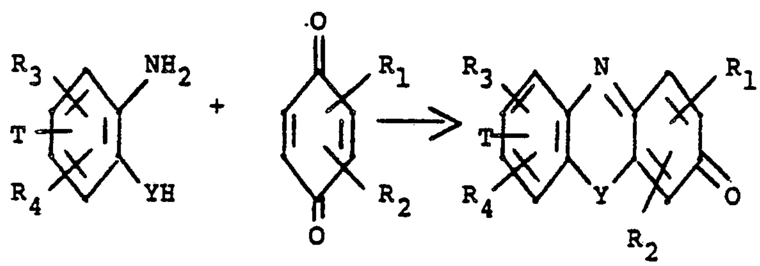

This general process is described in the literature.

A specific process for preparing the intermediate phenothiazin-3-one is illustrated by the following equation:

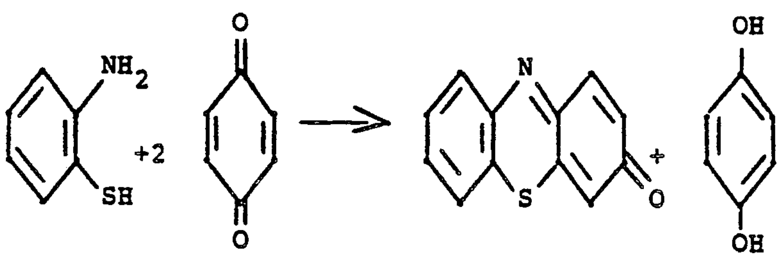

A B C D

The process requires the use of 2 moles of quinone per mole of aniline. Any suitable solvent may be used. Example of such solvents are acetic acid, lower alkanols, acetic acid/$H_2O$, loweralkanol/water or other polar solvents. A preferred solvent is one which will dissolve *A, B* and *D* and in which *C* is substantially insoluble.

The reaction is readily carried out at room temperature — lower temperatures, e.g. as low as −10°C, may be used — elevated temperatures may also be used but are not required. This process is more fully described in European Patent Specification EP—A—0,149,297.

Another useful process to prepare certain of the compounds of the present invention is the oxidation of certain phenothiazines or benzo[a]phenothiazines by standard oxidizing agents such as potassium dichromate, $NaClO_2$ and 2-3-dichloro-5,6-dicyano-1,4-benzoquinone (DDQ). Several of these benzo[a]phenothiazines and phenothiazines are described in EP—A—0,138,481 and EP—A—0,136,893.

Examples of the Formulae I and II compounds follow. These examples are illustrative and are provided as an aid to understand the invention. All temperatures are in degrees Celsius and are uncorrected.

Example 1

Method A:

3H-Phenothiazin-3-one

To a stirred suspension of 1.72 kg (16 mol) of *p*-benzoquinone in 13 litres MeOH at room temperature was added slowly a solution of 1.0 kg (8 mol) of 2-aminothiophenol in 600 mol MeOH over a period of 1 hour. The resulting red mixture was stirred at room temperature for another 2 hours and then the product 3H-phenothiaz-3-one was filtered off. This 3H-phenothiazin-3-one was washed thoroughly with methanol and dried to give 1.07 kg of 3H-phenothiazin-3-one (61.49% yield), m.p. 157—159°C.

Method B:

To a stirring solution of 1.1 kg of ceric ammonium nitrate in 12.5 liters of $H_2O$ and 1.25 liters of HOAc at 10°C was added dropwise a solution of 100 g of phenothiazine in 500 ml acetone over a period of 20 minutes. The resulting mixture was stirred for another 20 minutes and the product 3H-phenothiazin-3-one was then filtered off. The filtered 3H-phenothiazin-3-one was washed with water thoroughly and dried to give 92 g of crude 3H-phenothiazin-3-one. The crude 3H-phenothiazin-3-one was extracted with minimum volume of $CH_2Cl_2$. Upon dilution of the $CH_2Cl_2$ solution with 10 times the volume of cyclohexane, a precipitate was formed which was filtered and dried to afford 35 g of 3H-phenothiazin-3-one.

Example 2

4-Chloro-3H-phenothiazin-3-one

To a stirring solution of 500 g (2.34 mol) of 3H-phenothiazin-3-one in 12.5 liters of glacial acetic acid was added 1.25 kg of potassium dichromate. The mixture was stirred at room temperature for 1/2 hour. To this resulting mixture was then added 2.34 mol of a 1 M solution of chlorine in glacial acetic acid dropwise over a period of 4 hours. The progress of the reaction was monitored by tlc to ensure no excess chlorine was added. After addition of chlorine was completed the mixture was stirred at room temperature for another 1/2 hour and was then poured into 120 liters of $H_2O$ with vigorous stirring. The 4-chloro-3H-phenothiazin-3-one precipitated was allowed to settle overnight. The majority of the aqueous solution was siphoned and discarded and the rest was filtered. The filtered precipitate was washed thoroughly with water and then rinsed with methanol and was allowed to dry to give 504 g crude 4-chloro-3H-phenothiazin-3-one which was recrystallized from toluene, m.p. 221°.

Example 3

4-Chloro-2,7-dimethoxy-3H-phenothiazin-3-one

Following the procedure described in Example 2 but substituting 2,7-dimethoxy-3H-phenothiazin-3-one for 3H-phenothiazin-3-one, the title compound was obtained, m.p. 264°C.

| | | | | | |
|---|---|---|---|---|---|
| Analysis, calculated: | C, 54.63; | H, 3.27; | N, 4.55; | S, 10.42; | Cl, 11.52. |
| Observed: | C, 54.64; | H, 3.31; | N, 4.53; | S, 10.60; | Cl, 11.69. |

Example 4

4-Chloro-1,7-dimethyl-3H-phenothiazin-3-one

Following the procedure described in Example 2 but substituting 1,7-dimethyl-3H-phenothiazin-3-one for 3H-phenothiazin-3-one for 3H-phenothiazin-3-one the title compound was obtained, m.p. 215—218°C.

| | | | | | |
|---|---|---|---|---|---|
| Analysis, calculated: | C, 60.98; | H, 3.66; | N, 5.08; | S, 11.63; | Cl, 12.86. |
| Observed: | C, 60.78; | H, 3.75; | N, 4.99; | S, 11.79; | Cl, 13.01. |

Example 5

4-Chloro-2,7-dimethyl-3H-phenothiazin-3-one

Following the procedure described in Example 2 but substituting 2,7-dimethyl-3H-phenothiazin-3-one for 3H-phenothiazin-3-one the title compound was obtained, m.p. 193—195°C.

| | | | | | |
|---|---|---|---|---|---|
| Analysis, calculated: | C, 60.98; | H, 3.66; | N, 5.08; | S, 11.63; | Cl, 12.86. |
| Observed: | C, 60.89; | H, 3.79; | N, 5.22; | S, 11.63; | Cl, 12.50. |

Example 6

4-Chloro-2-methyl-3H-phenothiazin-3-one

Following the procedure described in Example 2 but substituting 2-methyl-3H-phenothiazin-3-one for 3H-phenothiazin-3-one, the title compound was obtained, m.p. 206°C.

| | | | | | |
|---|---|---|---|---|---|
| Analysis, calculated: | C, 59.66; | H, 3.08; | N, 5.35; | S, 12.25; | Cl, 13.55. |
| Observed: | C, 59.59; | H, 3.35; | N, 5.32; | S, 12.64; | Cl, 13.27. |

Example 7

4-Chloro-7-methyl-3H-phenothiazin-3-one

Following the procedure described in Example 2 but substituting 7-methyl-3H-phenothiazin-3-one for 3H-phenothiazin-3-one, the title compound was obtained, m.p. 218°C.

| | | | | | |
|---|---|---|---|---|---|
| Analysis, calculated: | C, 59.66; | H, 3.08; | N, 5.35; | S, 12.25; | Cl, 13.55. |
| Observed: | C, 59.48; | H, 3.17; | N, 5.27; | S, 12.40; | Cl, 13.63. |

Example 8

4-Chloro-7-ethoxy-2-methoxy-3H-phenothiazin-3-one

Following the procedure described in Example 2 but substituting 7-ethoxy-2-methoxy-3H-phenothiazin-3-one for 3H-phenothiazin-3-one, the title compound was obtained, m.p. 236—239°C.

| | | | | | |
|---|---|---|---|---|---|
| Analysis, calculated: | C, 55.99; | H, 3.76; | N, 4.35; | S, 9.96; | Cl, 11.02. |
| Observed: | C, 56.05; | H, 3.93; | N, 4.37; | S, 10.11; | Cl, 10.99. |

Example 9

4-Bromo-2-methyl-3H-phenothiazin-3-one

Following the procedure described in Example 2 but substituting 2-methyl-3H-phenothiazin-3-one for 3H-phenothiazin-3-one and substituting bromine for chlorine, the title compound was obtained, m.p. 190°C.

| | | | | | |
|---|---|---|---|---|---|
| Analysis, calculated: | C, 50.99; | H, 2.63; | N, 4.57; | S, 10.47; | Br, 26.09. |
| Observed: | C, 50.97; | H, 2.69; | N, 4.61; | S, 10.56; | Br, 26.24. |

Example 10

9-Methoxy-3H-phenothiazin-3-one

To a suspension of p-benzoquinone (3.0 g) in 15 ml methanol was added 2-amino-3-methoxythiophenol (2.2 g) dissolved in 10 ml methanol. The mixture was stirred at room temperature for 45 minutes and concentrated *in vacuo*. The residue was triturated with ether and filtered. The resulting dark solid was chromatographed on silica gel and eluted with EtOAc, to afford the desired compound, m.p. 206—207°.

| | | | | |
|---|---|---|---|---|
| Analysis, calculated: | C, 64.18; | H, 3.73; | N, 5.76; | S, 13.18. |
| Observed: | C, 64.10; | H, 3.83; | N, 5.69; | S, 13.40. |

Example 11

7-Fluoro-3H-phenothiazin-3-one

Following the procedure described in Example 10 but substituting 2-amino-5-fluorothiophenol for 2-amino-3-methoxythiophenol, the title compound was obtained, m.p. 240°C.

| | | | | | |
|---|---|---|---|---|---|
| Analysis, calculated: | C, 62.33; | H, 2.61; | N, 6.06; | S, 13.86; | F, 8.21. |
| Observed: | C, 62.26; | H, 2.70; | N, 6.05; | S, 14.04; | F, 8.06. |

Example 12

4-Chloro-7-fluoro-3H-phenothiazin-3-one

Following the procedure described in Example 2 but substituting 7-fluoro-3H-phenothiazin-3-one for 3H-phenothiazin-3-one, the title compound was obtained, m.p. 250—255°C.

| | | | | | | |
|---|---|---|---|---|---|---|
| Analysis, calculated: | C, 54.25; | H, 1.90; | N, 5.27; | S, 12.07; | F, 7.15; | Cl, 13.34. |
| Observed: | C, 54.10; | H, 2.01; | N, 5.35; | S, 12.20; | F, 7.20; | Cl, 13.50. |

Example 13

7-Fluoro-2-methoxy-3H-phenothiazin-3-one

Following the procedure described in Example 10 but substituting 2-amino-5-fluorothiophenol for 2-amino-3-methoxythiophenol and substituting 2-methoxy-p-benzoquinone for p-benzoquinone, the title compound was obtained, m.p. 252°C.

| | | | | | |
|---|---|---|---|---|---|
| Analysis, calculated: | C, 59.76; | H, 3.08; | N, 5.36; | S, 12.27; | F, 7.26. |
| Observed: | C, 59.60; | H, 3.11; | N, 5.20; | S, 12.17; | F, 7.33. |

Example 14

2,4-Dimethoxy-3H-phenothiazin-3-one

Following the procedure described in Example 10 but substituting 2-aminothiophenol for 2-amino-3-methoxythiophenol and substituting 2,6-dimethoxy-p-benzoquinone for p-benzoquinone, the title compound was obtained, m.p. 193°C.

Analysis, calculated: C, 61.52; H, 4.06; N, 5.12; S, 11.73.
Observed: C, 61.37; H, 4.14; N, 5.16; S, 12.90.

Example 15

1,2-Dimethoxy-4-methyl-3H-phenothiazin-3-one

Following the procedure described in Example 10 but substituting 2-aminothiophenol for 2-amino-3-methoxythiophenol and substituting 2,3-dimethoxy-5-methyl-p-benzoquinone for p-benzoquinone, the title compound was obtained, m.p. 138°C.

Analysis, calculated: C, 62.70; H, 4.56; N, 4.87; S, 11.16.
Observed: C, 62.72; H, 4.74; N, 4.92; S, 11.28.

Example 16

1,7-Dimethyl-3H-phenothiazin-3-one and 2,7-dimethyl-3H-phenothiazin-3-one

Following the procedure described in Example 10 but substituting 2-amino-5-methylthiophenol for 2-amino-3-methoxythiophenol and substituting 2-methyl-p-benzoquinone for p-benzoquinone, a mixture of the title compounds were obtained. Chromatography on silica gel eluting with 10% EtOAc in $CH_2Cl_2$ afforded firstly 2,7-dimethyl-3H-phenothiazin-3-one, m.p. 177°C.

Analysis, calculated: C, 69.70; H, 4.60; N, 5.81; S, 13.29.
Observed: C, 69.51; H, 4.82; N, 5.78; S, 12.27,

and secondly, 1,7-dimethyl-3H-phenothiazin-3-one. m.p. 168—170°C.

Analysis, calculated: C, 69.70; H, 4.60; N, 5.81; S, 13.29.
Observed: C, 69.59; H, 4.63; N, 5.80; S, 13.40.

Example 17

2,4-Dichloro-7-fluoro-3H-phenothiazin-3-one

Following the procedure described in Example 10 but substituting 2-amino-5-fluorothiophenol for 2-amino-3-methoxythiophenol and substituting 2,6-dichloro-p-benzoquinone for p-benzoquinone, the title compound was obtained, m.p. 256—258°C.

Analysis, calculated: C, 48.02; H, 1.34; N, 4.68; S, 10.68; F, 6.33; Cl, 23.62.
Observed: C, 47.93; H, 1.42; N, 4.63; S, 10.75; F, 6.42; Cl, 23.80.

Example 18

1,4-Dichloro-7-fluoro-3H-phenothiazin-3-one

Following the procedure described in Example 10 but substituting 2-amino-5-fluorothiophenol for 2-amino-3-methoxythiophenol and substituting 2,5-dichloro-p-benzoquinone for p-benzoquinone, the title compound was obtained, m.p. 245—247°C.

Analysis, calculated: C, 48.02; H, 1.34; N, 6.33.
Observed: C, 48.20; H, 1.14; N, 6.20.

Example 19

2-Methoxy-7-methylthio-3H-phenothiazin-3-one

Following the procedure described in Example 10 but substituting 2-amino-5-methylthio thiophenol for 2-amino-3-methoxythiophenol and substituting 2-methoxy-p-benzoquinone for p-benzoquinone, the title compound was obtained, m.p. 222—224°C.

Analysis, calculated: C, 58.11; H, 3.83; N, 4.84; S, 22.16.
Observed: C, 58.28; H, 4.24; N, 4.62; S, 22.02.

Example 20

4-Trifluoromethyl-3H-phenothiazin-3-one and 2,4-bis(trifluoromethyl)-3H-phenothiazin-3-one

A solution of 3H-phenothiazin-3-one (10 g), trifluoromethyl iodide (50 g) and pyridine (40 ml) in acetonitrile (140 ml) was irradiated with a 450 watt lamp for 3 days. The volatiles were removed under vacuum and the resulting residue chromatographed on a silica gel column eluting with 5% EtOAc/$CH_2Cl_2$ to afford firstly, 2,4-bis(trifluromethyl)-3H-phenothiazin-3-one (650 mg) m.p. 173—175°C.

Analysis, calculated: C, 48.14; H, 1.44; N, 4.01; S, 9.18; F, 32.64.
Observed: C, 48.25; H, 1.72; N, 4.00; S, 9.28; F, 32.51.

Secondly, 4-trifluoromethyl-3H-phenothiazin-3-one (1.76 g), m.p. 184—185°C.

Analysis, calculated: C, 55.51; H, 2.15; N, 4.98; S, 11.40; F, 20.27.
Observed: C, 55.60; H, 2.14; N, 5.22; S, 11.43; F, 20.41.

Example 21

4-Acetyl-3H-phenothiazin-3-one

A solution of 3H-phenothiazin-3-one (2 g) and acetaldehyde (32 ml) in benzene (240 ml) was irradiated with a 450 watt lamp for 2 days. The volatiles were removed under vacuum and the residue chromatographed on a silica gel column eluting with 25% EtOAc/hexane to afford the desired compound, m.p. 222°C.

Analysis, calculated: C, 65.87; H, 3.55; N, 5.49; S, 12.56.
Observed: C, 65.88; H, 3.61; N, 5.30; S, 12.70.

Example 22

4-Bromo-2,7-dimethoxy-3H-phenothiazin-3-one

Step 1:

2-Methoxy-p-benzoquinone

Vanillin (2.432 kg) was added to a solution of sodium hydroxide (640 g) in water (8 l) and cooled to 10°C with an ice-bath. Then a solution of hydrogen peroxide (30%) (2.4 l) was added at a rate to keep the temperature of the reaction mixture below 30°C. The addition completed (about 2 hours), the reaction mixture was added over a period of 3 hours to a suspension of sodium periodate (880 g) in water (4 l) and acetic acid (640 ml) cooled with an ice-bath to 10°C (the temperature of the reaction mixture was kept below 35°C). The precipitate was filtered, washed with cold water followed by ethanol/hexane (1:1) mixture and air-dried to afford the title compound (1.9 kg), m.p. 144—147°C.

Step 2:

2-Amino-5-methoxythiophenol

To a solution of potassium hydroxide 8N (1.3 l) was added 2-amino-6-methoxybenzothiazole (750 g) and the mixture was refluxed for 18 hours. The resulting solution was neutralized by the addition of concentrated HCl, to pH 8.0, then acetic acid to pH 6.0. The precipitate which formed was filtered and washed with water to afford the title compound which was used immediately in Step 3.

Step 3:

2,7-Dimethoxy-3H-phenothiazin-3-one

To a suspension of 2-methoxy-p-benzoquinone, (1.15 kg) (Step 1) in methanol (8 l) was added portionwise a suspension of 2-amino-5-methoxythiophenol (from Step 2) in methanol (6 l). The reaction mixture was stirred for 15 minutes at room temperature, filtered and the collected solid washed with methanol (8 l). The product isolated was swished with DMF (16 l) for 2 hours, filtered and air-dried. The crude material was dissolved in hot DMF (16 l) (130°—140°C), filtered through Celite® and the filtrate cooled to room temperature. The crystals were filtered, washed with methanol (8 l) and air-dried to afford the title compound (703 g), m.p. 237—238°C.

Step 4:

4-Bromo-2,7-dimethoxy-3H-phenothiazin-3-one

A solution of bromine (280 g) in acetic acid (2.8 l) was added over a period of 30 minutes to a suspension of 2,7-dimethoxy-3H-phenothiazin-3-one (250 g) (Step 3) in acetic acid (7.5 l) and stirred for 2 hours. Methanol (12 l) was added and the mixture was stirred until the black suspension became an orange suspension. Then, the precipitate was filtered, washed with methanol and air-dried to afford the desired compound (312 g), m.p. 260—261°C.

Analysis, calculated: C, 47.74; H, 2.86; N, 3.98; S, 9.10; Br, 22.69.
Observed: C, 47.74; H, 2.81; N, 3.90; S, 9.02; Br, 22.37.

Example 23

4-Chloro-2-ethoxy-3H-phenothiazin-3-one and 4-chloro-2,7-diethoxy-3H-phenothiazin-3-one

Metallic sodium (506 mg) was dissolved in absorbed ethanol (75 ml) and 4-chloro-3H-phenothiazin-3-one (4.95 g) was added and stirred overnight at room temperature. The solvent was removed in vacuo, the resulting residue stirred in acetone (500 ml) for 1 hour and filtered. The filtrate was evaporated to dryness and the residue was chromatographed on a silica gel column eluting with 5% EtOAc/toluene to afford firstly, 4-chloro-2-ethoxy-3H-phenothiazin-3-one (1.33 g), m.p. 188—189°C.

Analysis, calculated: C, 57.63; H, 3.45; N, 4.80; S, 10.99; Cl, 12.15.
Observed: C, 57.66; H, 3.54; N, 4.81; S, 11.16; Cl, 12.02.

and secondly, 4-chloro-2,7-diethoxy-3H-phenothiazin-3-one (110 mg), m.p. 227—228°C.

Analysis, calculated: C, 57.22; H, 4.20; N, 4.17; S, 9.55; Cl, 10.56.
Observed: C, 57.19; H, 4.35; N, 4.07; S, 9.62; Cl, 10.61.

Example 24

2-(n-Butylthio)-3H-phenothiazin-3-one

To a solution of 3H-phenothiazin-3-one (0.64 g) in 75 ml methanol was added thiethylamine (1.0 ml) and n-butanethiol (0.58 ml). The mixture was stirred at room temperature for 48 hours. Then 2,3-dichloro-5,6-dicyano-1,4-benzoquinone (0.67 g) was added. The mixture was stirred at room temperature for 2 hours. The solvent was removed *in vacuo*. The residue was chromatographed on neutral alumina (Act III) and eluted with 15% EtOAc/hexane to afford the title compound (0.4 g), m.p. 133°C.

Analysis, calculated: C, 63.78; H, 5.02; N, 4.65.
Observed: C, 63.61; H, 5.04; N, 4.51.

Example 25

4-(n-Butylthio)-3H-phenothiazin-3-one

To a solution of phenothiazin-3-one (0.21 g) in 20 ml THF was added triethylamine (0.28 ml) and n-butanethiol (0.2 ml). The mixture was refluxed for 15 hours and then cooled to room temperature. 2,3-Dichloro-5,6-dicyano-1,4-benzoquinone (DDQ) (0.22 g) was added and the reaction was stirred for 2 hours at 25°. The solvent was removed *in vacuo*. The residue was chromatographed on neutral alumina (Act III) and eluted with 15% EtOAc/hexane to afford the title compound, m.p. 72°C.

| | | | | |
|---|---|---|---|---|
| Analysis, calculated: | C, 63.78; | H, 5.02; | N, 4.05; | S, 21.24. |
| Observed: | C, 63.83; | H, 5.07; | N, 4.86; | S, 21.06. |

Example 26

4-(n-Butylthio)-2-methyl-3H-phenothiazin-3-one

To a solution of 2-methyl-phenothiazin-3-one (0.23 g) in 12 ml dichloroethane was added triethylamine (0.8 ml) and n-butanethiol (0.7 ml). The mixture was stirred at room temperature for 72 hours. 2,3-Dichloro-5,6-dicyano-1,4-benzoquinone (0.22 g) was added. The mixture was stirred at room temperature for 2 hours. The mixture was stirred at room temperature for 2 hours. The solvent was removed *in vacuo* and the residue was chromatographed on neutral alumina (Act III) eluting with 15% EtOAc/hexane to give the title compound (120 mg), m.p. 97°C.

| | | | |
|---|---|---|---|
| Analysis, calculated: | C, 64.75; | H, 5.43; | N, 4.44. |
| Observed: | C, 64.62; | H, 5.53; | N, 4.43. |

Example 27

2-S-Glutathionyl-3H-phenothiazin-3-one

A mixture of phenothiazin-3-one (0.22 g), triethylamine (0.41 ml) and glutathione (0.3 g) in 1,2-dichloroethane (12 ml) was stirred at room temperature for 5 days. The solvent was removed *in vacuo*. The residue was dissolved in water and filtered. The filtrate was concentrated *in vacuo* and then chromatographed on XAD resin and eluted with water to give the title compound.

Example 28

4-Chloro-2-S-glutathionylphenothiazin-3-one

Following the procedure of Example 27, but substituting 4-chloro-3H-phenothiazine-3-one for 3H-phenothiazin-3-one, the title compound was obtained.

Example 29

5H-Benzo[a]phenothiazin-5-one

Following the procedure described in Example 10 but substituting 2-aminothiophenyl for 2-amino-3-methoxythiophenyl and substituting 1,4-naphthoquinone for p-benzoquinone the title compound was obtained, m.p. 176—177°C.

Example 30

6-Chloro-5H-benzo[a]phenothiazin-5-one

Following the procedure described in Example 2, but substituting 5H-benzo[a]phenothiazin-5-one for 3H-phenothiazin-3-one, the title compound was obtained, m.p. 230—231°C.

Example 31

6-Methyl-5H-benzo[a]phenothiazin-5-one

Following the procedure described in Example 10, but substituting 2-aminothiophenol for 2-amino-3-methoxythiophenol and substituting 2-methyl-1,4-naphthoquinone for p-benzoquinone, the title compound was obtained, m.p. 181°C.

| | | | | |
|---|---|---|---|---|
| Analysis, calculated: | C, 73.62; | H, 4.00; | N, 5.05; | S, 11.56. |
| Observed: | C, 73.77; | H, 4.16; | N, 4.99; | S, 11.69. |

Example 32

1-Hydroxy-6-methyl-5H-phenothiazin-5-one

Following the procedure described in Example 10 but substituting 2-aminothiophenol for 2-amino-3-methoxythiophenol and substituting 5-hydroxy-2-methyl-1,4-naphthoquinone for p-benzoquinone, the title compound was obtained, m.p. 226—228°C.

| | | | | |
|---|---|---|---|---|
| Analysis, calculated: | C, 69.61; | H, 3.78; | N, 4.77; | S, 10.93. |
| Observed: | C, 69.66; | H, 3.90; | N, 4.66; | S, 10.77. |

Example 33

1-Methoxy-6-methyl-5H-phenothiazin-5-one

Potassium *tert.*-butoxide (500 mg) was added to a suspension of 1-hydroxy-6-methyl-5H-benzo[a]phenothiazin-5-one (from Example 32) (500 mg) and methyl iodide (2 ml) in DMF (20 ml). After 30 minutes at room temperature, EtOAc (250 ml) was added followed by water (200 ml). The aqueous layer

was decanted and the organic layer was dried and evaporated to dryness. The residue was treated with ether, filtered and air-dried to afford the desired product (420 mg), m.p. 170—171°C.

Analysis, calculated: C, 70.34; H, 4.26; N, 4.56; S, 10.43.
Observed: C, 70.37; H, 4.44; N, 4.45; S, 10.52.

Example 34

4-Hydroxy-3H-phenothiazin-3-one-5,5-dioxide

To a suspension of 3-hydroxy-10H-phenothiazine-5,5-dioxide (1.75 g, 7 mmoles) in 2% aqueous sulfuric acid (25 ml) there was added, at room temperature, a solution of 80% sodium chlorite (3.17 g, 28 mmoles) in water (25 ml). The mixture was stirred for 15 minutes, then the red-orange precipitate was filtered to afford crude product (1.73 g). Purification was achieved by crystallization from DMF-methanol, m.p. 266° (dec.).

Analysis, calculated: C, 55.16; H, 2.70; N, 5.36; S, 12.27.
Observed: C, 54.68; H, 2.76; N, 5.38; S, 12.47.

Example 35

4-Chloro-3H-phenoxazin-3-one

To a solution of 1.2 g of 3H-phenoxazin-3-one in acetic acid (25 ml) was added $K_2Cr_2O_7$ (3.7 g). A solution of chlorine in acetic acid was added dropwise to the resulting suspension. After disappearance of the starting material, as monitored by TLC, the reaction mixture was poured into 200 ml of $H_2O$ and the resulting precipitate was filtered (1.2 g) and chromatographed on silica gel to yield the title compound.

Analysis, calculated: C, 62.22; H, 2.61; Cl, 15.30.
Observed: C, 62.10; H, 2.75; Cl, 15.24.

Example 36

2,4-Di-t-butyl-1H-phenothiazin-1-one

To a solution of 4.4 gm of 3,5-di-t-butyl-1,2-benzoquinone in 20 ml of ether was added a solution of 1.25 g of 2-aminothiophenol in 5 ml of ether. After stirring for 1 hour at 25°, the reaction mixture was evaporated. The residue was purified by flash chromatography on silica gel using 2% ethyl acetate in benzene as eluent. There was thus obtained 860 mg of the title compound as dark blue plates, m.p. 137—141°.

Analysis, calculated: C, 73.81; H, 7.12; N, 4.30; S, 9.85.
Observed: C, 73.77; H, 7.33; N, 4.33; S, 9.85.

Example 37

4-Bromo-1,7-dimethoxy-3-H-phenothiazin-3-one

To a suspension of 1,7-dimethoxy-3H-phenothiazin-3-one (300 mg) in acetic acid (9 ml) was added a 0.63 M solution of $Br_2$ in acetic acid (1.92 ml). After 15 minutes, methanol was added and the solid filtered, washed with ether and air dried to afford the title compound (353 mg), m.p. 267—270°C (dec).

Example 38

4-Chloro-1,7-dimethoxy-3H-phenothiazin-3-one and 2,4-dichloro-1,7-dimethoxy-2-H-phenothiazin-3-one

To a suspension of 1,7-dimethoxy-3H-phenothiazin-3-one (800 mg) in acetic acid (24 ml) was added a 1.15 M solution of $Cl_2$ in acetic acid (3.1 ml). After 15 minutes, methanol was added and the mixture was filtered, washed with ether and air dried to afford a mixture of the two title compounds (700 mg), which were separated on a silica gel column (EtOAc:$CH_2Cl_2$, 1:9), affording 4-chloro-1,7-dimethoxy-2H-phenothiazin-3-one, m.p. 278—280°C (dec.)

Analysis, Calculated: C, 54.64; H, 3.28; N, 4.55; S, 10.42; Cl, 11.52.
Observed: C, 54.44; H, 3.26; N, 4.62; S, 10.54; Cl, 11.48.

and 2,4-dichloro-1,7-dimethoxy-3H-phenothiazin-3-one, m.p. 259—260°C (dec.) m/e 341.

Example 39

7-Methoxy-2-(4-methylpiperazin-1-yl)-3H-phenothiazin-3-one

A mixture of 7-methoxy-3H-phenothiazin-3-one (1.2 g) and N-methyl piperazine HCl (3.4 g) in DMF (20 ml) was heated at 100°C for 3 hours. Then $NaIO_4$ (1 g) was added and the reaction mixture was heated at 100°C for 1 hour. Ice-water was added to the reaction mixture followed by ethyl acetate. The aqueous layer was decanted, filtered and the filtrate basified with $K_2CO_3$ and extracted with ethyl acetate. The organic layer was evaporated to dryness, the resulting residue was dissolved in $CH_2Cl_2$, dried and evaporated to dryness to afford the crude final product (1.2 g) which was purified by chromatography on silica gel column eluting with 10% MEOH/$CH_2Cl_2$ to give the title compound, m.p. 208—209°.

Example 40

4-Bromo-7-methoxy-2-(4-methylpiperazin-1-yl)-3H-phenothiazin-3-one

To a suspension of 7-methoxy-2-(4-methylpiperazin-1-yl)-3H-phenothiazin-3-one (500 mg) in acetic acid (10 ml) was added a solution of bromine in acetic acid (0.5 M) (6 ml) and stirred for 5 minutes. Hexane

(100 ml) was added and the resulting precipitate was filtered. The solid was suspended in a mixture of aqueous $K_2CO_3$ (50 ml), EtOAc (100 ml) and methanol (20 ml) and stirred for 15 minutes. After filtration and decantation, the organic layer was washed with brine, dried and evaporated to dryness to afford the title compound (190 mg) m.p. 209—210° (dec.).

Example 41

4-Bromo-2,7-dimethoxy-3H-phenothiazin-3-one-5,5-dioxide

Step 1:

4-Bromo-3-hydroxy-2,7-dimethoxy-10-H-phenothiazine

To a suspension of 4-bromo-2,7-dimethoxy-3H-phenothiazin-3-one (100 g) in a mixture of ethylacetate (2 l) and water (1 l) was added sodium hydrosulfite (200 g) in one batch with mechanical stirring. The orange reaction mixture was stirred for 15 hours under a nitrogen atmosphere. The resulting white reaction mixture was filtered and the precipitate washed with water under a nitrogen atmosphere to prevent air oxidation of the compound. The title compound (130 g) was obtained as a wet material and was used as such in the next step (Step 2). An analytical sample was air dried, m.p. 185°C.

| | | | | | |
|---|---|---|---|---|---|
| Analysis, Calculated: | C, 47.74; | H, 3.42; | N, 3.95; | S, 9.05; | Br, 22.56. |
| Observed: | C, 47.21; | H, 3.39; | N, 3.74; | S, 8.76; | Br, 22.44. |

Step 2:

3-Acetoxy-4-bromo-2,7-dimethoxy-10-H-phenothiazine

Wet 4-bromo-3-hydroxy-2,7-dimethoxy-10-H-phenothiazine (130 g) (from step 1) was suspended in pyridine (230 ml). The mixture was cooled to 0°C in an ice-water bath. Acetic anhydride (195 ml) was then slowly added. The solution was left stirring at room temperature for 1/2 hour. The mixture was then concentrated under reduced pressure to approximately 1/3 of the original volume. Then a mixture of ether:hexane, 1:1 (700 ml) was added, causing a large amount of crystals to appear. These crystals were filtered, washed with ether, and air dried, giving 51.4 g of pure 3-acetoxy-4-bromo-2,7-dimethoxy-10-phenothiazine. The mother liquors were reevaporated, and ether and hexane were added again, giving 36.37 of crude 3-acetoxy-4-bromo-2,7-dimethoxy-10H-phenothiazine m.p. 201—203°C.

| | | | | |
|---|---|---|---|---|
| Analysis, Calculated: | C, 48.50; | H, 3.56; | N, 3.53; | S, 8.09. |
| Observed: | C, 48.31; | H, 3.47; | N, 3.47; | S, 8.00. |

Step 3:

3-Acetoxy-4-bromo-2,7-dimethoxy-10H-phenothiazin-5,5-dioxide

To 3-acetoxy-4-bromo-2,7-dimethoxy-10H-phenothiazin (20 g) (from Step 2) in suspension in $CH_2Cl_2$:MeOH, (1:1) (500 ml), was added m-chloroperoxybenzoic acid (26.0 g). The reaction mixture rapidly became deep brown with the formation of a yellowish precipitate which corresponded to the intermediate sulfoxide on the 5-position. The mixture was heated at reflux for 18 hours. The solid was then filtered and washed with ether. Since there was still some sulfoxide remaining, the solid was suspended in ethanol:1,2-dichloroethane (500 ml) with 1.35 g of m-chloroperoxybenzoic acid and heated at reflux overnight (15 hours). The solid was then filtered and washed with ether and air dried giving 13.0 g of 3-acetoxy-4-bromo-2,7-dimethoxy-10H-phenothiazine-5,5-dioxide, m.p. 260°C.

| | | | | |
|---|---|---|---|---|
| Analysis, Calculated: | C, 44.87; | H, 3.29; | N, 3.27; | S, 7.49. |
| Observed: | C, 44.82; | H, 3.21; | N, 3.18; | S, 7.67. |

Step 4:

4-Bromo-3-hydroxy-2,7-dimethoxy-10H-phenothiazin-5,5-dioxide

To a suspension of 3-acetoxy-4-bromo-2,7-dimethoxy-10H-phenothiazine-5,5-dioxide (10.0 g) in methanol (105 ml) was added a solution of 2N aqueous sodium hydroxide (74 ml) under a nitrogen atmosphere. After 20 minutes, the mixture was acidified with 10% v/v aqueous acetic acid (250 ml), causing a large amount of compound to precipitate. The mixture was then diluted with water (105 ml) and the solid filtered, washed with water and ether and dried in a desiccator to afford quantitatively the title compound, m.p. 252—260°C (dec).

Step 5:

4-Bromo-2,7-dimethoxy-3H-phenothiazin-3-one-5,5-dioxide

To a stirred suspension of 4-bromo-3-hydroxy-2,7-dimethoxy-10H-phenothiazin-5,5-dioxide (from Step 4) (1 g) in THF (10 ml) was added 2,3-dichloro-5,6-dicyano-1,4-benzoquinone (1.17 g). After 15 minutes, the mixture was filtered, the solid washed with ether and air dried. The solid was filtered through a silica gel pad with $CH_2Cl_2$:EtOAc, 1:1, to afford the title compound (300 mg), m.p. 228—230°C (dec), m/e 383.

Example 42

2,7-Dimethoxy-4-(4-methylpiperazin-1-yl)-3H-phenothazin-3-one-5,5-dioxide

To a stirred suspension of 4-bromo-3-hydroxy-2,7-dimethoxy-10H-phenothiazine-5,5-dioxide (1 g) in THF (10 ml) was added 2,3-dichloro-5,5-dicyano-1,4-benzoquinone (1.17 g). After a period of 20 minutes, N-methyl piperazine (1.44 ml) was slowly added. After 20 minutes, hexane was added to the mixture and the resulting precipitate was filtered, washed with ether and air dried. The compound was chromatographed using $CH_2Cl_2$:MeOH (9.5:0.5) as eluant, to afford the title compound (242 mg), m.p. 261°C (dec.).

| | | | | |
|---|---|---|---|---|
| Analysis; Calculated: | C, 58.67; | H, 5.83; | N, 10.80; | S, 8.24. |
| Observed: | C, 58.73; | H, 5.67; | N, 10.83; | S, 8.56. |

Example 43

4-Hydroxy-2,7-dimethoxy-3H-phenothiazin-3-one-5,5-dioxide

To a stirred suspension of 4-bromo-3-hydroxy-2,7-dimethoxy-10H-phenothiazine-5,5-dioxide (100 mg) in THF (10 ml) was added water (0.1 ml) and 2,3-dichloro-5,6-dicyano-1,4-benzoquinone (0.12 g). After 20 minutes, hexane was added and the solid was filtered, washed with ether and air dried to afford the title compound, m.p. 333—335°C (dec.).

Example 44

1,4-Bis(1-propylamino)-3H-phenothiazin-3-one-5,5-dioxide

To a solution of 3-hydroxy-10H-phenothiazine-5,5-dioxide (989 mg) in THF (50 ml) was added 2,3-dichloro-5,6-dicyano-1,4-benzoquinone (1.82 g). The resulting green mixture was stirred at room temperature for 3 minutes, then there was added n-propyl amine (2.36 g). The mixture was stirred for 20 minutes and then filtered. The filtrate was evaporated to dryness and the residue chromatographed on a column of silica gel eluting with a 1:20 mixture of ethyl acetate and dichloromethane to afford the title compound as a purple solid (850 mg). Crystallization from methane afforded purple crystals (606 mg), m.p. 174—176°C.

| | | | | |
|---|---|---|---|---|
| Analysis, Calc'd: | C, 60.14; | H, 5.89; | N, 11.69; | S, 8.92. |
| Found: | C, 60.08; | H, 5.93; | N, 11.80; | S, 8.71. |

Example 45

1,4-Bis(4-methylpiperazin-1-yl)-3H-phenothiazin-3-one-5,5-dioxide

The procedure of Example 44 was used, substituting N-methyl piperazine for n-propyl amine to afford the title compound. It was crystallized from a toluene-hexane mixture to afford red crystals, m.p.: 247—249°C (dec.).

| | | | | |
|---|---|---|---|---|
| Analysis, Calc'd: | C, 59.84; | H, 6.16; | N, 15.86; | S, 7.26 |
| Found: | C, 59.98; | H, 6.35; | N, 15.58; | S, 7.1. |

Example 46

6-(1-Propylamino)-5H-benzo[a]phenothiazin-5-one-7,7-dioxide

Step 1: 5-acetoxy-12H-benzo[a]phenothiazine

To a stirred solution of 5-hydroxy-12H-benzo[a]phenothiazine (50 g) in pyridine (115 ml) was added acetic anhydride (48 ml). The reaction was exothermic stirring was continued without cooling for 30 minutes, then the mixture was cooled to 10°C using an ice bath. The yellow crystalline solid was filtered and washed with ether to afford the title compound (23.4 g). The product was crystallized from ethyl acetate, m.p. 185—186°C.

Step 2: 5-acetoxy-12H-benzo[a]phenothiazine-7,7-dioxide

To a suspension of 5-acetoxy-12H-benzo[a]phenothiazine (10 g) in dichloromethane (125 ml), was added a solution of m-chloroperoxybenzoic acid (18 g) in methanol (125 ml). The mixture was refluxed for 2.5 hours, then after cooling to room temperature the insoluble solid was filtered to afford the desired sulfone (8.6 g). The solid was recrystallized from THF m.p. 284—287°C.

| | | | | |
|---|---|---|---|---|
| Analysis, Calc'd: | C, 63.70; | H, 3.86; | N, 4.13; | S, 9.45. |
| Found: | C, 63.67; | H, 3.82; | N, 4.20; | S, 9.44. |

Step 3: 5-hydroxy-12H-benzo[a]phenothiazine-7,7-dioxide

To a suspension of 5-acetoxy-12H-benzo[a]phenothiazine-7,7-dioxide (6.6 g) in methanol (200 ml), kept under a nitrogen atmosphere was added 2N aqueous sodium hydroxide solution (132 ml). The mixture was stirred at room temperature for 7 minutes, then there was added 10% acetic acid (200 ml) and water (300 ml). After 10 minutes of stirring the mixture was filtered to afford the title compound (5.68 g) as a pink solid. The solid was recrystallized from THF, m.p. 334°C (dec).

| | | | | |
|---|---|---|---|---|
| Analysis, Calc'd: | C, 63.70; | H, 3.86; | N, 4.13; | S, 9.45. |
| Found: | C, 63.67; | H, 3.82; | N, 4.20; | S, 9.44. |

Step 4: 6-(1-Propyl amino)-5H-benzo[a]phenothiazin-5-one-7,7-dioxide

To a suspension of 5-hydroxy-12H-benzo[a]phenothiazine-7,7-dioxide (594 mg) in THF (10 ml) was added 2,3-dichloro 5,6-dicyano-1,4-benzoquinone (1.021 gram). The mixture was stirred at room temperature for 2 minutes, then there was added n-propyl amine (0.2 ml). The stirring was continued for 0.5 hour, then the mixture was evaporated to dryness. To the residue was added 50 ml dichloromethane and the mixture was stirred for 15 minutes and filtered. The filtrate was evaporated to dryness and the residue crystallized from a mixture of toluene and hexane to afford the title compound (442 mg) as a red-brown crystalline solid, m.p. 149—151°C (dec.).

| | | | | |
|---|---|---|---|---|
| Analysis, Calc'd: | C, 64.75; | H, 4.58; | N, 7.95; | S, 9.10. |
| Found: | C, 64.66; | H, 4.48; | N, 7.95; | S, 9.04. |

Example 47

6-(4-Methyl piperazin-1-yl)-5H-benzo[a]phenothiazin-5-one-7,7-dioxide

The procedure of Example 46, Step 4 was used, substituting N-methyl piperazine for n-propyl amine, to afford the title compound, m.p. slow dec. from 183°C.

| | | | | |
|---|---|---|---|---|
| Analysis, Calc'd: | C, 64.10; | H, 4.87; | N, 10.68; | S, 8.15. |
| Found: | C, 63.89; | H, 4.90; | N, 10.56; | S, 8.10. |

Example 48

6-Amino-5H-benzo[a]phenothiazin-5-one-7,7-dioxide

The procedure of Example 46, Step 4 was used, substituting 28% aqueous ammonium hydroxide solution for n-propyl amine, to afford the title compound, m.p. 264—266°C.

Certain of the compounds herein disclosed contain one or more centers of asymmetry. The present invention is meant to include the various diastereomers of such compounds as well as their racemic and optically active resolved forms.

Some of the compounds described may exist in one or more tautomeric forms. All such tautomeric forms are included within the present invention.

## Claims

1. The use, for the manufacture of a medicament for inhibiting mammalian leukotriene biosynthesis or action, of a compound of the Formula I, a compound that is a salt of a compound of the Formula I, or a pharmaceutical composition containing such a compound:

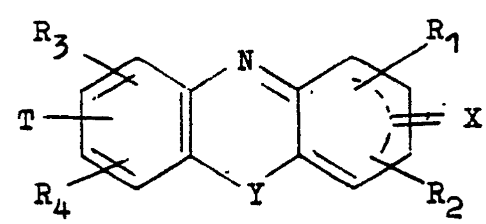

I

in which

X is in the 1 or 3 position and is O, S or NR;

R is H, $C_{1-6}$ branched or linear alkyl, CN or phenyl;

Y is O, Se, S, SO, $SO_2$ or NR; and the broken line represents a double bond between the 1 and 2 or 2 and 3 positions;

each of $R_1$, $R_2$, $R_3$ and $R_4$ independently of the others, is

(1) hydrogen,

(2) $C_{1-6}$ alkyl,

(3) $C_{2-6}$ alkenyl,

(4) $-(CH_2)_nM$

where n is 0 or an integer from 1 to 6 and M is

(a) $OR_5$,

(b) halogen,

(c) $CF_3$,

(d) $SR_5$ where $R_5$ is H; $C_1$—$C_6$ alkyl; benzyl; phenyl or substituted phenyl where the substituents are $C_{1-3}$ alkyl, halogen, CN, $CF_3$, $COOR_6$, $CH_2COOR_6$, $(CH_2)_nNR_8R_9$ where n is 0, 1 or 2, $C_{1-3}$ alkoxy, OH or $C_{1-6}$ haloalkyl; $-(CH_2)_mCOOR_6$, where m is 0 or an integer from 1 to 6 and $R_6$ is H, phenyl or $C_{1-6}$ alkyl; CN, formyl, $CF_3$ or $CH_2$—$R_{12}$, where $R_{12}$ is $C_{1-5}$ alkyl, phenyl or dimethylamino;

(e) phenyl or substituted phenyl as defined above for $R_5$;

(f) $COOR_6$;

(g) 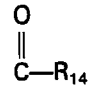

where $R_{15}$ is H, $(CH_2)_n$ $COOR_6$ where n is 0 or an integer from 1 to 4, $C_{1-6}$ alkyl, $CF_3$, phenyl, or substituted phenyl as defined above for $R_5$;

(h) tetrazole;

(i) $$-NH-\overset{O}{\overset{\|}{C}}-R_7$$

where $R_7$ is $C_{1-6}$ alkyl, benzyl or phenyl;

(j) $-NR_8R_9$ where $R_8$ and $R_9$ are independently selected from H, phenyl or substituted phenyl as defined above for $R_5$ or $C_{1-4}$ alkyl, $C_{1-4}$ alkylaminoalkyl, or may be joined through the N to form a 4-methyl piperazinyl radical;

(k) $-NHSO_2R_{10}$ where $R_{10}$ is OH, $C_{1-6}$ alkyl, $C_{1-6}$ alkoxy, phenyl or $CF_3$;

(l) $$-\overset{O}{\overset{\|}{C}}-CH_2OH;$$

(m) $-SOR_{11}$ where $R_{11}$ is $C_{1-6}$ alkyl, phenyl or substituted phenyl as defined above for $R_5$, $(CH_2)_mCOOR_6$ where m is 1 to 6, CN, formyl or $CF_3$;

(n) $-CONR_8R_9$;

(o) $-SO_2NR_8R_9$;

(p) $-SO_2R_{13}$ where $R_{13}$ is OH, H, $C_{1-6}$ alkyl, phenyl or substituted phenyl as defined above for $R_5$, $(CH_2)_mCOOR_6$ where m is 1 to 6, CN or $CF_3$;

(q) $NO_2$;

(r) $$-O-\overset{O}{\overset{\|}{C}}-R_{14};$$

(s) $$-O-\overset{O}{\overset{\|}{C}}-NR_8R_9;$$

(t) $$-O-\overset{O}{\overset{\|}{C}}-OR_7;$$

(u) $-CN$; or

(v) $NR_{15}R_{16}$ where $R_{15}$ and $R_{16}$ are such that $NHR_{15}R_{16}$ is an essential amino acid;

or any two of $R_1$, $R_2$, $R_3$ and $R_4$ are joined to form a fourth saturated or unsaturated $C_{5-6}$ ring; and T is H, halogen or $CF_3$.

2. The use claimed in Claim 1, in which, in Formula I, X is in the 1-position.

3. The use claimed in Claim 2, in which X is O or NR.

4. The use claimed in Claim 3, in which, in Formula I, Y is S, SO, $SO_2$, NR or O and, in the structural unit $(CH_2)_nM$, n is 0 or 1.

5. The use claimed in Claim 4, in which, in Formula I, X is O and Y is S.

6. The use claimed in Claim 1, in which, in Formula I X is in the 3-position.

7. The use claimed in Claim 6, in which X is O or NR.

8. The use claimed in Claim 7, in which in Formula I Y is S, SO, $SO_2$, NR or O and in the structural unit $(CH_2)_nM$, n is 0 or 1.

9. The use claimed in Claim 8, in which Y is S or O.

10. The use claimed in Claim 9, in which X is O and Y is S.

11. The use, for the manufacture of a medicament for inhibiting mammalian leukotriene biosynthesis or action, of a compound of the Formula I, a compound that is a salt of a compound of the Formula I, or a pharmaceutical composition containing such a compound, in which, in Formula I, the variables are as defined as follows, X being in the 1-position:

| Y | X | $R_1$ | $R_2$ | $R_3$ | $R_4$ | T |
|---|---|---|---|---|---|---|
| O | O | 2-t-Bu | 8-t-Bu | 4-t-Bu | 6-t-Bu | H |
| O | O | 2-t-Bu | H | 4-Me | H | H |
| S | S | 2-t-Bu | 4-t-Bu | H | H | H |
| N—$CH_3$, S, O, Se, SO or $SO_2$ | O | 2-Cl | H | H | H | H |
| " | O | 2-$SCF_3$ | H | H | H | H |
| " | O | 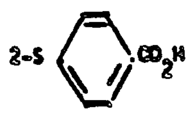 | H | H | H | H |
| S | O | 2-t-Bu | 4-t-Bu | H | H | H |
| N—$CH_3$, S, O, Se, SO or $SO_2$ | O | 2-CN | H | H | H | H |
| " | O | H | 3-$CO_2$Et | H | H | H |
| " | O | H | 3-Cl | H | H | H |
| " | O | H | H | 4-Cl | H | H |
| " | O | H | H | 4-$SO_2CH_3$ | H | H |
| " | O | 2-Cl | H | 4-Cl | H | H |
| " | NH | 2-Cl | H | 4-Cl | H | H |
| " | NH | H | H | H | H | H |
| N—CN | O | 2-Cl | H | 4-Cl | H | H |
| S | O | H | H | H | H | H |
| S | O | 2-Cl | 3-Cl | 4-Cl | 7-Cl | 9-Cl |
| S | O | 2-Br | 3-Br | 4-Br | 7-Br | 9-Br |
| S | O | H | H | H | 7-$SO_2CH_3$ | H |
| S | O | 2-Cl | H | 4-$SO_2CH_3$ | H | H |
| S | O | 2-F | H | 4-Cl | H | H |
| S | O | 2-Br | H | H | H | H |
| S | O | 2-$CF_3$ | H | H | H | H |
| S | O | 2-$SCF_3$ | H | H | H | H |
| S | O | 2-$SO_2CF_3$ | H | H | H | H |
| S | O | H | 3-Cl | H | H | H |

| Y | X | $R_1$ | $R_2$ | $R_3$ | $R_4$ | T |
|---|---|---|---|---|---|---|
| S | O | H | 3-$CO_2$Et | H | H | H |
| S | O | H | 3-$CO_2H$ | H | H | H |
| S | O | H | 3-CN | H | H | H |
| S | O | H | 3-$SCF_3$ | H | H | H |
| S | O | H | H | 4-Cl | H | H |
| S | O | H | H | 4-$SCF_3$ | H | H |
| S | O | H | H | 4-Cl | H | H |
| S | O | 2-Br | H | 4-Br | H | H |
| S | O | 2-Cl | H | H | 8-CN | H |
| S | O | 2-Cl | H | H | 8-$CO_2$Et | H |
| S | O | 2-Cl | H | H | 8-$CO_2H$ | H |
| S | O | 2-Cl | H | H | 8-$CF_3$ | H |
| S | O | 2-Cl | H | H | 7-$SO_2CH_3$ | H |
| S | O | H | 3-$CONMe_2$ | H | H | H |
| S | O | 2-Cl | H | H | 7-$OCH_3$ | H |
| S | O | 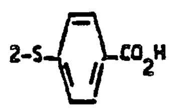 | H | H | H | H |
| S | O | 2-$SO_2CH_3$ | H | H | H | H |
| S | O | 2-$CH_2CH{=}CH_2$ | H | 4-$CH_2CH{=}CH_2$ | H | H |
| S | O | H | 3-$N(CH_3)_2$ | H | H | H |
| S | O | H | H | 4-Cl | 7-S—$C_6H_6$ | H |
| S | O | 2-$CHCO_2$ | H | H | H | H |
| S | O | 2-Cl | H | 4-$SCH_2CO_2H$ | H | H |
| S | O | 2-$COC_3$ | H | H | 7-$OCH_3$ | H |
| S | O | H | H | 4-CO—$C_6H_6$ | 7-$OCH_3$ | H |
| S | NH | 2-Cl | H | 4-Cl | H | H |
| S | NH | H | 3-$N(CH_3)_2$ | H | H | H |
| S | NH | 2-$SCH_3$ | H | 4-$SCH_3$ | H | H |
| S | O | H | H | H | H | H |
| S | NH | H | H | H | H | H |
| S | O | 2-$COCH_3$ | H | H | 7-$OCH_3$ | H |
| S | O | H | H | 4-$COC_6H_5$ | 7-$OCH_3$ | H |

| Y | X | $R_1$ | $R_2$ | $R_3$ | $R_4$ | T |
|---|---|---|---|---|---|---|
| S | NH.HCl | H | H | H | H | H |
| S | O | H | H | H | H | H |
| O | O | H | H | H | H | H |
| O | NH | H | H | H | H | H |
| O | S | H | H | H | H | H |
| O | NH.HCl | H | H | H | H | H |
| Se | O | H | H | H | H | H |
| Se | NH | H | H | H | H | H |
| Se | S | H | H | H | H | H |
| NH | NH.HCl | H | H | H | H | H |
| NH | S | H | H | H | H | H |
| O | O | 4-Cl | H | H | H | H |
| O | O | 4-Cl | H | 7-OMe | H | H |
| O | O | 4-Me | H | H | H | H |
| O | O | H | 2-Cl | H | H | H |
| O | O | 4-Cl | 2-S-pPAA* | H | H | H |
| Se | O | 4-Cl | H | H | H | H |
| Se | O | 4-Cl | H | 7-OMe | H | H |
| Se | O | 4-Me | H | H | H | H |
| Se | O | 4-Cl | 2-S-pPAA* | H | H | H |
| N—$CH_3$ | O | 4-Cl | H | H | H | H |
| N—$C_6H_6$ | O | 4-Cl | H | 7-OMe | H | H |
| N—H | O | 4-Cl | 2-S-pPAA* | H | H | H |
| S | O | 4-Cl | H | H | H | H |
| SO | O | H | H | H | H | H |
| $SO_2$ | O | H | H | H | H | H |
| $SO_2$ | O | 4-Cl | H | H | H | H |
| N—Me | O | H | H | H | H | H |
| N—Me | O | 4-Cl | H | H | H | H |
| N—Me | O | 4-Cl | H | 7-OMe | H | H |
| NCN | O | 4-Cl | H | H | H | H |
| NH | O | 4-Cl | H | H | H | H |

| Y | X | $R_1$ | $R_2$ | $R_3$ | $R_4$ | T |
|---|---|---|---|---|---|---|
| NH | O | 4-Cl | H | H | H | H |
| S | O | 2-t-Bu | 9-t-Bu | 4-OMe | H | H |
| S | O | 2-t-Bu | 7-t-Bu | 4-F | H | H |
| S | O | 2-t-Bu | 7-t-Bu | 4-Me | H | H |
| S | O | 2-t-Bu | 7-t-Bu | 4-SMe | H | H |

*p-PAA = para-Phenylacetic acid

12. The use, for the manufacture of a medicament for inhibiting mammalian leukotriene biosynthesis or action, of a compound of the Formula I, a compound that is a salt of a compound of the Formula I, or a pharmaceutical composition containing such a compound, in which, in Formula I, the variables are as defined as follows, X being in the 3-position:

| $R_1$ | $R_2$ | $R_3$ | $R_4$ | T |
|---|---|---|---|---|
| H | H | H | H | H |
| 2-Cl | H | H | H | H |
| H | H | 6-Cl | H | H |
| H | H | 7-Cl | H | H |
| H | H | 8-Cl | H | H |
| H | H | 9-Cl | H | H |
| 1-Cl | H | H | H | H |
| 1-Cl | 4-Cl | H | H | H |
| 2-Cl | 4-Cl | H | H | 1-Cl |
| 2-$N(Me)_2$ | H | H | H | H |
| 2-SMe | H | H | H | H |
| 2-S-pPAA | H | H | H | H |
| 2-$C(O)CH_3$ | H | H | H | H |
| 2-OMe | H | H | H | H |
| H | H | H | 7-$CH_2CO_2H$ | H |
| H | H | H | 8-$CH_2COOH$ | H |
| H | 2-$SO_3H$ | H | H | H |
| 2-$N(Me)_2$ | H | H | H | H |
| 2-SMe | H | H | H | H |
| 2-$C(O)CH_3$ | H | H | H | H |
| 2-OMe | H | H | H | 7-I |
| 2-$CH_2CO_2H$ | H | H | H | H |

| $R_1$ | $R_2$ | $R_3$ | $R_4$ | T |
|---|---|---|---|---|
| 2-$CH(CH_3)CO_2H$ | H | H | H | H |
| 4-$CH_2COOH$ | H | H | H | H |
| 4-$CH(CH_3)CO_2H$ | H | H | H | H |
| H | H | 7-OH | 6-propyl | H |
| 4-Cl | H | H | H | H |
| 4-F | H | H | H | H |
| 4-F | H | 7-Cl | H | H |
| 4-Et | H | H | H | H |
| 4-Et | H | 7-OMe | H | H |
| 4-Et | H | 7-Cl | H | H |
| 4-Cl | H | 7-OMe | H | H |
| 4-OMe | H | 7-Cl | H | H |
| 4-Cl | H | 6-Cl | H | H |
| 4-Cl | H | 8-Cl | H | H |
| 4-Cl | H | 9-Cl | H | H |
| 4-Cl | H | 6-OMe | H | H |
| 4-Cl | H | 8-OMe | H | H |
| 4-Cl | H | 9-Et | H | h |
| 4-Cl | H | 6-Et | H | H |
| 4-Cl | H | 7-Et | H | H |
| 4-Cl | H | 8-Et | H | H |
| 4-Cl | 1-Et | H | H | H |
| 4-Cl | 2-Et | H | H | H |
| 4-Cl | 1-$CH_2COOH$ | H | H | H |
| 4-Cl | 2-$CH_2COOH$ | H | H | H |
| 4-OH | 2-OMe | 7-OMe | H | H |
| 4-OH | H | H | H | H |
| 4-Me | 1-OMe | 2-OMe | H | H |
| 4-Cl | H | 6-$CH_2COOH$ | H | H |
| 4-Cl | H | 7-$CH_2COOH$ | H | H |
| 4-Cl | H | 8-$CH_2COOH$ | H | H |

| $R_1$ | $R_2$ | $R_3$ | $R_4$ | T |
|---|---|---|---|---|
| 4-Cl | 2-N(Me)$_2$ | H | H | H |
| 4-Cl | 1-N(Me)$_2$ | H | H | H |
| 4-Cl | 2-N(Me)$_2$ | 7-OMe | H | H |
| 4-Cl | 2-N(Me)$_2$ | 7-Cl | H | H |
| 4-Cl | 2-SMe | H | H | H |
| 4-Cl | 2-SCH$_2$COOH | H | H | H |
| 4-Cl | 2-S-pPAA | H | H | H |
| 4-Cl | 1-S-pPAA | H | H | H |
| 4-Cl | 2-S-pPAA | 7-OMe | H | H |
| 4-Cl | 2-SO$_3$H | H | H | H |
| 4-Cl | 2-OMe | H | H | H |
| 4-Cl | 2-OMe | 7-Cl | H | H |
| 4-Cl | H | 7F | H | H |
| 4-OMe | H | 7-OMe | H | H |
| 4-OMe | H | 7-Me | H | H |
| 4-OMe | 2-SMe | H | H | H |
| 4-SMe | H | H | H | H |
| 4-Br | H | H | H | H |
| 4-I | H | H | H | H |
| 4-Br | H | 7-OMe | H | H |
| 4-I | H | 7-OMe | H | H |
| 4-Br | 2-Me | H | H | H |
| 4-I | 2-Me | H | H | H |
| 4-Cl | H | 7/8-(CH$_2$)$_4$— | | H |
| 4-Cl | H | 7/8-(CH$_2$)$_3$— | | H |
| 4-Br | 2-OMe | 7-OMe | H | H |
| 7-F | H | H | H | H |
| 7-NH$_2$ | H | H | H | H |
| 2-Me | 7-N(Me)$_2$ | H | H | H |
| 7-N(Me)$_2$ | H | H | H | H |
| 1-CO$_2$H | 4-OH | 7-NMe$_2$ | H | H |

| $R_1$ | $R_2$ | $R_3$ | $R_4$ | T |
|---|---|---|---|---|
| 1-Cl | 2-Cl | 4-Cl | H | 7-Cl |
| 1-Me | 7-Me | H | H | 4-Cl |
| 2-Me | 7-Me | H | H | 4-Cl |
| 2-Me | H | H | H | 4-Cl |
| 7-Me | H | H | H | 4-Cl |
| 9-OMe | H | H | H | H |
| 2-OMe | H | H | H | 7-F |
| 2-OMe | 4-OMe | H | H | H |
| 1-OMe | 2-OMe | 7-Me | H | H |
| 1-Me | 7-Me | H | H | H |
| 2-Me | 7-Me | H | H | H |
| 2-Cl | 4-Cl | H | H | 7-F |
| 1-Cl | 4-Cl | H | H | 7-F |
| 2-OMe | 7-SMe | H | H | H |
| H | H | H | H | 4-$CF_3$ |
| 2-$CF_3$ | H | H | H | 4-$CF_3$ |
| 4-COMe | H | H | H | H |
| 2-OEt | H | H | H | 4-Cl |
| 2-S-n-Bu | H | H | H | H |
| 4-S-n-Bu | H | H | H | H |
| 2-Me | 4-S-n-Bu | H | H | H |
| 9-OMe | H | H | H | H |
| 2-OMe | H | H | H | H |
| 2-OMe | 4-OMe | H | H | H |
| 1-OMe | 2-OMe | 4-Me | H | H |
| 4-OMe | H | H | H | H |
| 1-OMe | 7-OMe | H | H | 4-Br |
| 1-OMe | 7-OMe | 2-Cl | H | 4-Cl |
| 1-OMe | 7-OMe | H | H | 4-Cl |
| 2- 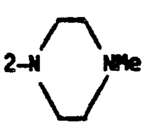 | 7-OMe | H | H | H |

| $R_1$ | $R_2$ | $R_3$ | $R_4$ | T |
|---|---|---|---|---|
| 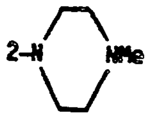 | 7-OMe | H | H | 4-Br |
| 2-OMe | 4-OH | 7-OMe | H | H |
| 1-NHPr | 4-NHPr | H | H | H |
| 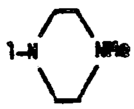 | 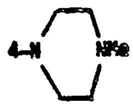 | H | H | H |
| 4-COMe | H | H | H | H |
| 2-NHPr | 4-NHPr | H | H | H |
| 2-OME | 4-CN | 7-OMe | H | H |
| 2-OMe | 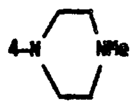 | 7-OMe | H | H |
| 2-OMe | 7-OMe | H | H | H |
| 2-OMe | 4-NHPr | 7-OMe | H | H |
| 1-NHPr | 4-NHPr | H | H | H |
| 1-NHPr | 4-NHPr | 7-OMe | H | H |
| 1-NHPr | 4-NHPr | H | H | H |
| 2-NHPr | 4-NHPr | 7-OMe | H | H |
| 2-OMe | 4-$NH_2$ | 7-OMe | H | H |
| 2-OMe | 4-NHPr | 7-OMe | H | H |
| 1-OMe | 4-Cl | 7-OMe | H | H |
| 1-OMe | 4-Br | 7-OMe | H | H |
| 1-NHPr | 4-NHPr | H | H | H |
| 1-OMe | 4-CN | 7-OMe | H | H |
| 2-OMe | 4-$NHCH_2CO_2R$* | 7-OMe | H | H |
| 2-OMe | 4-S-7-Bu | 7-OMe | H | H |
| 2-OMe | 4-$CH_2CO_2R$* | 7-OMe | H | H |
| 2-OMe | 4-$SO_2Me$ | 7-OMe | H | H |
| 2-S-n-Bu | H | H | H | H |
| 4-S-n-Bu | H | H | H | H |
| 2-Me | 4-S-n-Bu | H | H | H |
| 2-OMe | 7-Me | H | H | 4-Br |

| $R_1$ | $R_2$ | $R_3$ | $R_4$ | T |
|---|---|---|---|---|
| 2-OMe | 7-$CF_3$ | H | H | 4-Br |
| 2-OMe | 7-F | H | H | 4-Br |
| 2-OMe | 7-Cl | H | H | 4-Br |
| 2-OMe | 7-Br | H | H | 4-Br |
| 2-OMe | 7-$NMe_2$ | H | H | 4-Br |
| 2-OMe | 7-SMe | H | H | 4-Br |
| 2-OMe | 7-$SO_2$Me | H | H | 4-Br |
| 2-OMe | 7-Ph | H | H | 4-Br |
| 1-Me | H | H | H | 4-Br |
| 2-Me | H | H | H | H |
| 2-OEt | H | H | H | H |
| 7-Cl | H | H | H | H |
| 9-Cl | H | H | H | H |
| 7-F | H | H | H | H |
| 7-Me | H | H | H | H |
| 7-OMe | H | H | H | H |
| 2-Cl | H | H | H | 4-Cl |
| 1-Me | 7-Me | H | H | 4-Cl |
| 1-Me | 7-Me | H | H | H |
| 2-OMe | 7-OEt | H | H | H |
| 2-$NH_2$ | H | H | H | H |
| 7-OH | H | H | H | H |

* pPAA = para-Phenylacetic Acid

and in which R is H or $C_1$ to $C_4$ alkyl.

13. The use, for the manufacture of a medicament for inhibiting mammalian leukotriene biosynthesis or action, of a compound of the Formula I, a compound that is a salt of a compound of the Formula I, or a pharmaceutical composition containing such a compound, in which, in Formula I, the variables are as defined as follows, X being in the 3-position:

| Y | X | $R_1$ | $R_2$ | $R_3$ | $R_4$ | T |
|---|---|---|---|---|---|---|
| S | O | 2-OMe | 7-OMe | H | H | H |
| S | O | 1-OMe | 7-OMe | H | H | H |
| S | O | 2-OMe | 7-OMe | H | H | 1-Br |
| S | O | 1-OMe | 7-OMe | H | H | 2-Br |
| S | O | 1-OMe | 7-OMe | H | H | 4-Br |
| S | O | 1-OMe | 7-OMe | H | H | 2-Cl |
| S | O | 1-OMe | 7-OMe | H | H | 4-Cl |
| S | O | 2-OMe | 7-OMe | H | H | 1-Cl |
| S | O | 2-OMe | 7-OMe | H | H | 4-Cl |
| S | O | 2-OEt | 7-OEt | H | H | 1-Br |
| S | O | 2-OEt | 7-OEt | H | H | 4-Br |
| S | O | 2-OEt | 7-OEt | H | H | 1-Cl |
| S | O | 2-OEt | 7-OEt | H | H | 4-Cl |
| S | O | 2-OMe | 7-OMe | 8-OMe | H | 1-Br |
| S | O | 2-OMe | 7-OMe | 8-OMe | H | 4-Br |
| S | O | 2-OMe | 7-OMe | H | H | 4-F |
| S | O | 2-OMe | 7-OMe | H | H | 4-$CF_3$ |
| S | O | 2-OMe | 7-OEt | H | H | 4-Be |
| S | O | 2-OMe | 7-OEt | H | H | 4-Cl |
| S | O | 2-OMe | 7-OEt | H | H | 4-F |
| S | O | 2-OMe | 7-OEt | H | H | 4-$CF_3$ |
| S | O | 2-OEt | 7-OMe | H | H | 4-Br |
| S | O | 2-OEt | 7-OMe | H | H | 4-Cl |
| S | O | 2-OEt | 7-OMe | H | H | 4-F |
| S | O | 2-OEt | 7-OMe | H | H | 4-$CF_3$ |
| S | O | 2-OMe | 7-OMe | H | H | 4-Br |

14. The use, for the manufacture of a medicament for inhibiting mammalian leukotriene biosynthesis or action, of a compound of the Formula I, a compound that is a salt of a compound of the Formula II:

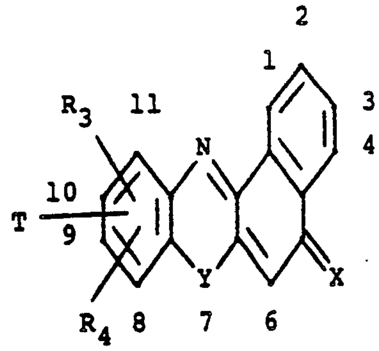

II

where the substituents are:

| Y | X | $R_3$ | $R_4$ | T |
|---|---|---|---|---|
| O | O | H | H | H |
| S | O | H | H | H |
| SO | O | H | H | H |
| $SO_2$ | O | H | H | H |
| SO | O | H | H | 6-Cl |
| S | O | 6-$COCH_3$ | H | H |
| S | O | 6-$CH_3$ | H | H |
| $SO_2$ | O | 6-OH | H | H |
| $SO_2$ | O | 6-OMe | H | H |
| S | O | 9-OMe | H, | H |
| S | O | 6-OH | H | H |
| S | O | 6-OMe | H | H |
| S | O | 6-NHCOMe | H | H |
| S | O | 6-NHPh | H | H |
| S | O | H | H | 6-Br |
| S | O | 6-NHMe | H | H |
| S | O | 6-NH-t-Bu | H | H |
| S | O | 6-NH-COMe | H | 9-Cl |
| S | O | 6-NH-COMe | 9-Ome | H |
| S | O | 6-NHPh-p-Br | H | 9-Cl |
| O | O | H | H | 6-Cl |
| O | O | H | H | 6-Br |
| O | O | 9-OMe | H | 6-Br |

| Y | X | $R_3$ | $R_4$ | T |
|---|---|---|---|---|
| O | O | 9-OMe | 6-NHPr | H |
| S | O | 6-$CF_3$ | H | H |
| S | O | 6-S-n-Bu | H | H |
| S | O | 6-OMe | H | 9-Cl |
| S | O | 9-OMe | H | 6-Cl |
| S | O | 6-OMe | 9-OMe | H |
| S | O | 6-Cl | 9-Me | 11-Br |
| S | O | 6-NHPh | 9-Me | 11-Br |
| S | O | 6-Me | H | H |
| O | NH | 9-$NMe_2$ | 10-Me | H |
| O | NH | 9-$N(Et)_2$ | H | H |
| S | O | 6-Cl | H | H |

15. A use as claimed in any one of Claims 1 to 14, applied to treatment of (1) pulmonary conditions, (2) inflammation, (3) allergies, (4) pain, (5) cardiovascular conditions or (6) skin conditions.

16. The compounds of the Formula:

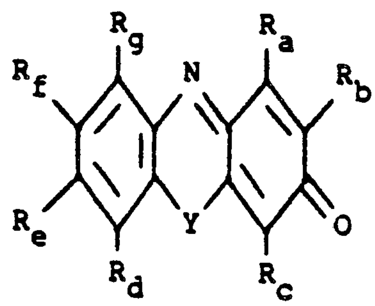

in which the substituents are:

| Y | $R_a$ | $R_b$ | $R_c$ | $R_d$ | $R_e$ | $R_f$ | $R_g$ |
|---|---|---|---|---|---|---|---|
| S | H | $SCH_3$ | H | H | H | H | H |
| S | H | H | $SCF_3$ | H | H | H | H |
| S | H | H | CHO | H | H | H | H |
| S | H | H | $COCF_3$ | H | H | H | H |
| S | H | H | H | H | $SCH_3$ | H | H |
| S | H | H | H | H | $CO_2CH_3$ | H | H |
| S | H | H | H | H | $CO_2H$ | H | H |
| S | H | H | H | H | CHO | H | H |
| S | H | H | H | H | $CONH_2$ | H | H |
| S | H | H | H | H | $CH_2OH$ | H | H |

| Y | $R_a$ | $R_b$ | $R_c$ | $R_d$ | $R_e$ | $R_f$ | $R_g$ |
|---|---|---|---|---|---|---|---|
| S | H | H | Cl | H | $CO_2Me$ | H | H |
| S | H | H | Cl | H | $CO_2H$ | H | H |
| S | H | H | Cl | H | CHO | H | H |
| S | H | H | Cl | H | $CONH_2$ | H | H |
| S | H | H | Cl | H | $CH_2OH$ | H | H |
| S | H | O-benzyl | Cl | H | H | H | H |
| S | H | OEt | H | H | $CH_3$ | H | H |
| S | $CH_3$ | H | Cl | H | $CH_3$ | H | H |
| S | H | $CH_3$ | Cl | H | $CH_3$ | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| S | H | OMe | Br | H | Me | H | H |
| S | H | H | CHO | H | H | H | H |
| S | H | H | $COCF_3$ | H | H | H | H |
| S | H | H | H | H | $SCH_3$ | H | H |
| S | H | H | H | H | $OCH_3$ | H | H |
| S | H | H | H | H | $CO_2CH_3$ | H | H |
| S | H | H | H | H | $CO_2H$ | H | H |
| S | H | H | H | H | CN | H | H |
| S | H | H | H | H | CHO | H | H |
| S | H | H | H | H | $CONH_2$ | H | H |
| S | H | H | H | H | $CH_2OH$ | H | H |
| S | H | H | H | H | $CF_3$ | H | H |
| S | $CH_3$ | H | Cl | H | H | H | H |
| S | H | $CH_3$ | Cl | H | H | H | H |

| Y | $R_a$ | $R_b$ | $R_c$ | $R_d$ | $R_e$ | $R_f$ | $R_g$ |
|---|---|---|---|---|---|---|---|
| S | H | H | Cl | H | F | H | H |
| S | H | Cl | Cl | H | F | H | H |
| S | H | $CH_3$ | H | H | F | H | H |
| S | $CH_3$ | H | H | H | F | H | H |
| S | H | H | Cl | H | OMe | H | H |
| S | H | H | Cl | H | $CF_3$ | H | H |
| S | H | H | Cl | H | $CO_2Me$ | H | H |
| S | H | H | Cl | H | $CO_2H$ | H | H |
| S | H | H | Cl | H | CN | H | H |
| S | H | H | Cl | H | CHO | H | H |
| S | H | H | Cl | H | $CONH_2$ | H | H |
| S | H | H | Cl | H | $CH_2OH$ | H | H |
| S | H | H | $OCH_3$ | H | Cl | H | H |
| S | H | H | $CF_3$ | H | Cl | H | H |
| S | H | OEt | Cl | H | H | H | H |
| S | H | OiPr | H | H | H | H | H |
| S | OMe | H | Cl | H | H | H | H |
| S | OEt | H | Cl | H | H | H | H |
| S | H | OiPr | Cl | H | H | H | H |
| S | H | O-benzyl | Cl | H | H | H | H |
| S | H | OEt | H | H | F | H | H |
| S | H | $OCH_3$ | Cl | H | H | H | H |
| S | H | OEt | H | H | $CH_3$ | H | H |
| S | H | OEt | Cl | H | F | H | H |
| S | H | OEt | Cl | H | $CH_3$ | H | H |
| S | H | H | Cl | H | $CH_3$ | H | H |
| S | H | $CH_3$ | Cl | H | H | H | H |
| S | H | $CH_3$ | Br | H | H | H | H |
| S | $CH_3$ | H | H | H | $CH_3$ | H | H |
| S | H | $CH_3$ | H | H | $CH_3$ | H | H |
| S | $CH_3$ | H | Cl | H | $CH_3$ | H | H |
| S | H | $CH_3$ | Cl | H | $CH_3$ | H | H |

| Y | $R_a$ | $R_b$ | $R_c$ | $R_d$ | $R_e$ | $R_f$ | $R_g$ |
|---|---|---|---|---|---|---|---|
| S | Cl | H | Cl | H | F | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| O | H | H | Cl | H | H | H | H |
| S | H | OMe | Br | H | Me | H | H |
| $SO_2$ | H | H | OH | H | H | H | H |
| $SO_2$ | H | OMe | OH | H | OMe | H | H |
| $SO_2$ | OMe | OMe | Me | H | H | H | H |
| $SO_2$ | H | H | OMe | H | H | H | H |
| $SO_2$ | H | OMe | OMe | H | OMe | H | H |
| S | H | H | H | H | H | H | $OCH_3$ |
| S | H | $OCH_3$ | H | H | F | H | H |
| S | H | $OCH_3H$ | $OCH_3$ | H | H | H | H |
| S | $OCH_3$ | $OCH_3$ | Me | H | H | H | H |
| S | H | H | $COCH_3$ | H | H | H | H |
| S | $OCH_3$ | H | Br | H | $OCH_3$ | H | H |
| S | $OCH_3$ | Cl | Cl | H | $OCH_3$ | H | H |
| S | $OCH_3$ | H | Cl | H | $OCH_3H$ | H | H |
| S | H | 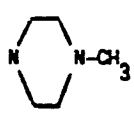 | H | H | $OCH_3$ | H | H |
| S | H | 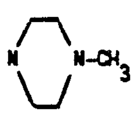 | Br | H | $OCH_3$ | H | H |
| $SO_2$ | H | $OCH_3$ | OH | H | $OCH_3$ | H | H |
| $SO_2$ | NHPr | H | NHPr | H | H | H | H |
| $SO_2$ 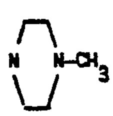 | | H | 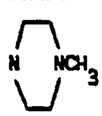 | H | H | H | H |

| Y | $R_a$ | $R_b$ | $R_c$ | $R_d$ | $R_e$ | $R_f$ | $R_g$ |
|---|---|---|---|---|---|---|---|
| $SO_2$ | H | $OCH_3$ | 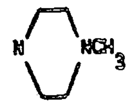 | H | $OCH_3$ | H | H |
| $SO_2$ | H | $OCH_3$ | Br | H | $OCH_3$ | H | H |
| S | NHPR | H | NHPr | H | H | H | H |
| S | NHPr | H | NHPr | H | $OCH_3$ | H | H |
| S | H | NHPr | NHPr | H | H | H | H |
| S | H | NHPr | NHPr | H | $OCH_3$ | H | H |
| S | H | $OCH_3$ | $NH_2$ | H | $OCH_3$ | H | H |
| S | H | $OCH_3$ | NHPr | H | $OCH_3$ | H | H |
| $SO_2$ | H | $OCH_3$ | NHPr | H | $OCH_3$ | H | H |
| O | $OCH_3$ | H | Cl | H | $OCH_3$ | H | H |
| O | $OCH_3$ | H | Br | H | $OCH_3$ | H | H |
| O | NHPr | H | NHPr | H | H | H | H |
| $SO_2$ | H | $OCH_3$ | CN | H | $OCH_3$ | H | H |
| $SO_2$ | H | $OCH_3$ | $NHCH_2CO_2R$* | H | $OCH_3$ | H | H |
| $SO_2$ | H | $OCH_3$ | S-N-Bu | H | $OCH_3$ | H | H |
| $SO_2$ | H | $OCH_3$ | $CH_2CO_2R$* | H | $OCH_3$ | H | H |
| $SO_2$ | H | $OCH_3$ | $SO_2CH_3$ | H | $OCH_3$ | H | H |
| S | H | S-n-Bu | H | H | H | H | H |
| S | H | H | S-n-Bu | H | H | H | H |
| S | H | $CH_3$ | S-n-Bu | H | H | H | H |
| S | H | OMe | Br | H | $CF_3$ | H | H |
| S | H | OMe | Br | H | F | H | H |
| S | H | OMe | Br | H | Cl | H | H |
| S | H | OMe | Br | H | Br | H | H |
| S | H | OMe | Br | H | $NMe_2$ | H | H |
| S | H | OMe | Br | H | SMe | H | H |
| S | H | OMe | Br | H | $SO_2Me$ | H | H |
| S | H | OMe | Br | H | Ph | H | H |
| S | H | H | H | Cl | OMe | H | H |

and R is H or $C_2$ to $C_4$ alkyl.

17. The compounds of the Formula:

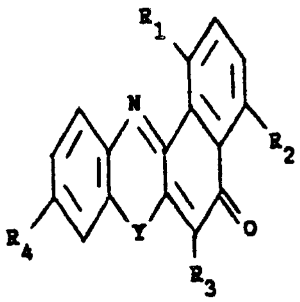

wherein the substituents are:

| Y | $R_1$ | $R_2$ | $R_3$ | $R_4$ |
|---|---|---|---|---|
| S | H | H | S-n-$CH_4H_9$ | H |
| S | OH | H | $CH_3$ | H |
| S | $OCH_3$ | H | $CH_3$ | H |
| S | H | H | F | H |
| S | H | H | $CF_3$ | H |
| S | H | H | Cl | $CF_3$ |
| S | H | H | Cl | $SCH_3$ |
| S | H | H | Br | Cl |
| S | H | H | $CH_3$ | Br |
| S | H | H | F | Br |
| S | H | H | $COCH_3$ | Cl |
| S | H | H | $CF_3$ | $CH_3$ |
| S | H | H | $SC_4H_9$ | $CH_3$ |
| S | H | H | $CF_3$ | Cl |
| S | H | H | Cl | $CH_2COOR$ |
| S | H | H | Cl | $CH(Me)CO_2R$ |
| S | H | H | Cl | $COCH_3$ |
| S | H | H | H | Cl |
| S | H | H | H | Br |
| S | H | H | H | F |
| S | H | H | H | $CF_3$ |
| S | H | H | H | $CH_3$ |
| S | H | H | H | $CH_2OH$ |
| S | H | H | H | $OCH_3$ |
| S | H | H | H | $SCH_3$ |

| Y | $R_1$ | $R_2$ | $R_3$ | $R_4$ |
|---|---|---|---|---|
| S | H | H | H | COOR |
| S | H | H | H | $CH_2CO_2R$ |
| S | H | H | H | $CH(Me)CO_2R$ |
| $SO_2$ | H | H | NHPr | H |
| $SO_2$ | H | H | 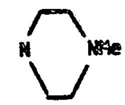 | H |
| $SO_2$ | H | H | $NH_2$ | H |
| $SO_2$ | H | H | NHPr | $OCH_3$ |
| S | -1,4-dihydro- | | H | H |
| S | H | H | NHPr | $OCH_3$ |
| O | H | H | Cl | H |
| O | H | H | Br | H |
| O | H | H | Br | $OCH_3$ |
| O | H | H | NHPr | $OCH_3$ |

and wherein R is H or $C_1$ to $C_4$ alkyl.

18. The compounds of the formula:

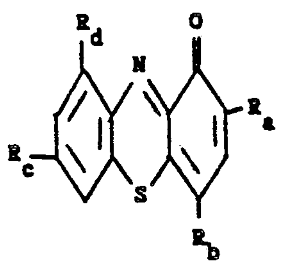

III

where the substituents are:

| $R_a$ | $R_b$ | $R_c$ | $R_d$ |
|---|---|---|---|
| t-Bu | t-Bu | H | H |
| t-Bu | t-Bu | F | H |
| t-Bu | t-Bu | Me | H |
| t-Bu | t-Bu | SMe | H |
| t-Bu | t-Bu | H | OMe |

19. The compounds 2-S-glutathionyl-3H-phenothiazin-3-one and 4-chloro-2-S-glutathionyl-phenothiazin-3-one.

20. The use claimed in Claim 1 of the manufacture of a medicament that additionally contains a second active ingredient that is a non-steroidal antiinflammatory agent; a peripheral analgesic agent; a cyclooxygenase inhibitor; a leukotriene antagonist, an antihistaminic agent; a prostaglandin antagonist; or a thromboxane antagonist, in which the weight ratio of the Formula I compound to the second active ingredient ranges from 10:1 to 1:10.

**Patentansprüche**

1. Verwendung, zur Herstellung eines Medikaments zur Inhibierung der Leukotrienbiosynthese oder -wirkung in Säugetieren, einer Verbindung der Formel I, einer Verbindung, die ein Salz einer Verbindung der Formel I ist, oder einer eine solche Verbindung enthaltenden pharmazeutischen Zusammensetzung:

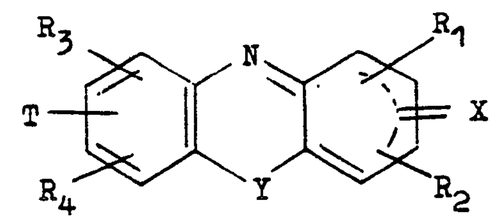

I

worin

X in der 1- oder 3-Stellung ist und O, S oder NR ist;

R H, verzweigtes oder lineares $C_{1-6}$-Alkyl, CN oder Phenyl ist;

Y O, Se, S, SO, $SO_2$ oder NR ist; und die gestrichelte Linie eine Doppelbindung zwischen der 1- und 2- oder 2- und 3-Stellung darstellt;

jedes von $R_1$, $R_2$, $R_3$ und $R_4$ unabhängig von den anderen

(1) Wasserstoff,

(2) $_{1-6}$-Alkyl,

(3) $_{2-6}$-Alkenyl,

(4) —$(CH_2)_nM$ ist,

worin n 0 oder eine ganze Zahl von 1 bis 6 ist und M

(a) $OR_5$,

(b) Halogen,

(c) $CF_3$,

(d) $SR_5$, worin $R_5$ H; $C_{1-6}$-Alkyl; Benzyl; Phenyl oder substituiertes Phenyl, worin die Substituenten $C_{1-3}$-Alkyl, Halogen, CN, $CF_3$, $COOR_6$, $CN_2COOR_6$, $(CH_2)_nNR_8R_9$ worin n 0, 1 oder 2 ist, $C_{1-3}$-Alkoxy, OH oder $C_{1-6}$-Halogenalkyl; sind; —$(CH_2)_mCOOR_6$, worin m 0 oder eine ganze Zahl von 1 bis 6 ist und $R_6$ H, Phenyl oder $C_{1-6}$-Alkyl; CN, Formyl, $CF_3$ oder $CH_2$—$R_{12}$, worin $R_{12}$ $C_{1-5}$-Alkyl, Phenyl oder Dimethylamino ist; ist,

(e) Phenyl oder substituiertes Phenyl, wie oben für $R_5$ definiert;

(f) $COOR_6$;

(g) 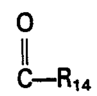

worin $R_{15}$ H, $(CH_2)_n$ $COOR_6$, worin n 0 oder eine ganze Zahl von 1 bis 4 ist, $C_{1-6}$-Alkyl, $CF_3$, Phenyl oder substituiertes Phenyl, wie oben für $R_5$ definiert;

(h) Tetrazol;

(i) 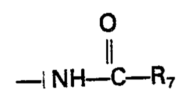

worin $R_7$ $C_{1-6}$-Alkyl, Benzyl oder Phenyl ist;

(j) —$NR_8R_9$ worin $R_8$ und $R_9$ unabhängig aus H, Phenyl oder substituiertem Phenyl, wie oben für $R_5$ definiert, oder $C_{1-4}$-Alkyl, $C_{1-4}$-Alkylaminoalkyl ausgewählt sind, oder über das N unter Bildung eines 4-Methylpiperazinylrestes verbunden sein können;

(k) —$NHSO_2R_{10}$, worin $R_{10}$ OH, $C_{1-6}$-Alkyl, $C_{1-6}$-Alkoxy, Phenyl oder $CF_2$ ist;

(l) 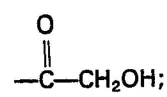

(m) —$SOR_{11}$, worin $R_{11}$ $C_{1-6}$-Alkyl, Phenyl oder substituiertes Phenyl, wie oben für $R_5$ definiert, $(CH_2)_mCOOR_6$, worin m 1 bis 6 ist, CN, Formyl oder $CF_3$ ist;

(n) —$CONR_8R_9$;

(o) —$SO_2NR_8R_9$;

(p) —$SO_2R_{13}$, worin $R_{13}$ OH, H, $C_{1-6}$-Alkyl, Phenyl oder substituiertes Phenyl, wie oben für $R_5$ definiert, $(CH_2)_mCOOR_6$, worin m 1 bis 6 ist, CN oder $CF_3$ ist;

(q) $NO_2$;

(r) 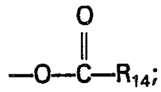

(s) 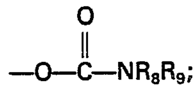

(t) 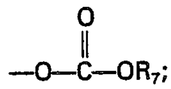

(u) —CN; oder

(v) $NR_{15}R_{16}$ worin $R_{15}$ und $R_{16}$ so sind, daß $HNR_{15}R_{16}$ eine essentielle Aminosäure darstellt; oder jeweils zwei von $R_1$, $R_2$, $R_3$ und $R_4$ unter Bildung eines vierten gesättigten oder ungesättigten $C_5$—$C_6$-Rings zusammengenommen werden; und T H, Halogen oder $CF_3$ ist.

2. Verwendung, wie in Anspruch 1 beansprucht, worin in Formel I X in der 1-Stellung ist.

3. Verwendung, wie in Anspruch 2 beansprucht, worin X O oder NR ist.

4. Verwendung, wie in Anspruch 3 beansprucht, worin in Formel I Y S, SO, $SO_2$, NR oder O ist und in der Struktureinheit $(CH_2)_nM$ n 0 oder 1 ist.

5. Verwendung, wie in Anspruch 4 beansprucht, worin in Formel I X O ist und Y S ist.

6. Verwendung, wie in Anspruch 1 beansprucht, worin in Formel I X in der 3-Stellung ist.

7. Verwendung, wie in Anspruch 6 beansprucht, worin X O oder NR ist.

8. Verwendung, wie in Anspruch 7 beansprucht, worin in Formel I Y S, SO, $SO_2$, NR oder O ist und in der Struktureinheikt $(CH_2)_nM$ n 0 oder 1 ist.

9. Verwendung, wie in Anspruch 8 beansprucht, worin Y S oder O ist.

10. Verwendung, wie in Anspruch 9 beansprucht, worin X O ist und Y S ist.

11. Verwendung, zur Herstellung eines Medikaments zur Ihhibierung der Leukotrienbiosynthese oder -wirkung in Säugetieren, einer Verbindung der Formel I, einer Verbindung, die ein Salz einer Verbindung der Formel I ist, oder einer eine solche Verbindung enthaltenden pharmazeutischen Zusammensetzung, worin in der Formel I die Variablen wie nachstehend definiert sind und X in der 1-Stellung ist.

| Y | X | $R_1$ | $R_2$ | $R_3$ | $R_4$ | T |
|---|---|---|---|---|---|---|
| O | O | 2-t-Bu | 8-t-Bu | 4-t-Bu | 6-t-Bu | H |
| O | O | 2-t-Bu | H | 4-Me | H | H |
| S | S | 2-t-Bu | 4-t-Bu | H | H | H |
| N—$CH_3$, S, O, Se, SO oder $SO_2$ | O | 2-Cl | H | H | H | H |
| " | O | 2-$SCF_3$ | H | H | H | H |
| " | O | 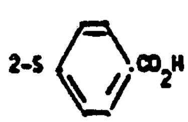 | H | H | H | H |
| S | O | 2-t-Bu | 4-t-Bu | H | H | H |
| N—$CH_3$, S, O, Se, SO oder $SO_2$ | O | 2-CN | H | H | H | H |
| " | O | H | 3-$CO_2$Et | H | H | H |
| " | O | H | 3-Cl | H | H | H |
| " | O | H | H | 4-Cl | H | H |
| " | O | H | H | 4-$SO_2CH_3$ | H | H |

| Y | X | $R_1$ | $R_2$ | $R_3$ | $R_4$ | T |
|---|---|---|---|---|---|---|
| $SO_2$ | O | 2-Cl | H | 4-Cl | H | H |
| " | NH | 2-Cl | H | 4-Cl | H | H |
| " | NH | H | H | H | H | H |
| N—CN | O | 2-Cl | H | 4-Cl | H | H |
| S | O | H | H | H | H | H |
| S | O | 2-Cl | 3-Cl | 4-Cl | 7-Cl | 9-Cl |
| S | O | 2-Br | 3-Br | 4-Br | 7-Br | 9-Br |
| S | O | H | H | H | 7-$SO_2CH_3$ | H |
| S | O | 2-Cl | H | 4-$SO_2CH_3$ | H | H |
| S | O | 2-F | H | 4-Cl | H | H |
| S | O | 2-Br | H | H | H | H |
| S | O | 2-$CF_3$ | H | H | H | H |
| S | O | 2-$SCF_3$ | H | H | H | H |
| S | O | 2-$SO_2CF_3$ | H | H | H | H |
| S | O | H | 3-Cl | H | H | H |
| S | O | H | 3-$CO_2$Et | H | H | H |
| S | O | H | 3-$CO_2$H | H | H | H |
| S | O | H | 3-CN | H | H | H |
| S | O | H | 3-$SCF_3$ | H | H | H |
| S | O | H | H | 4-Cl | H | H |
| S | O | H | H | 4-$SCF_3$ | H | H |
| S | O | H | H | 4-Cl | H | H |
| S | O | 2-Br | H | 4-Br | H | H |
| S | O | 2-Cl | H | H | 8-CN | H |
| S | O | 2-Cl | H | H | 8-$CO_2$Et | H |
| S | O | 2-Cl | H | H | 8-$CO_2$H | H |
| S | O | 2-Cl | H | H | 8-$CF_3$ | H |
| S | O | 2-Cl | H | H | 7-$SO_2CH_3$ | H |
| S | O | H | 3-$CONMe_2$ | H | H | H |
| S | O | 2-Cl | H | H | 7-$OCH_3$ | H |

| Y | X | $R_1$ | $R_2$ | $R_3$ | $R_4$ | T |
|---|---|---|---|---|---|---|
| S | O | 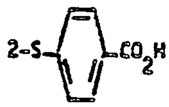 | H | H | H | H |
| S | O | 2-$SO_2CH_3$ | H | H | H | H |
| S | O | 2-$CH_2CH{=}CH_2$ | H | 4-$CH_2CH{=}CH_2$ | H | H |
| S | O | H | 3-$N(CH_3)_2$ | H | H | H |
| S | O | H | H | 4-Cl | 7-S—$C_6H_6$ | H |
| S | O | 2-$CHCO_2$ | H | H | H | H |
| S | O | 2-Cl | H | 4-$SCH_2CO_2H$ | H | H |
| S | O | 2-$COC_3$ | H | H | 7-$OCH_3$ | H |
| S | O | H | H | 4-CO—$C_6H_6$ | 7-$OCH_3$ | H |
| S | NH | 2-Cl | H | 4-Cl | H | H |
| S | NH | H | 3-$N(CH_3)_2$ | H | H | H |
| S | NH | 2-$SCH_3$ | H | 4-$SCH_3$ | H | H |
| S | O | H | H | H | H | H |
| S | NH | H | H | H | H | H |
| S | O | 2-$COCH_3$ | H | H | 7-$OCH_3$ | H |
| S | O | H | H | 4-$COC_6H_5$ | 7-$OCH_3$ | H |
| S | NH.HCl | H | H | H | H | H |
| S | O | H | H | H | H | H |
| O | O | H | H | H | H | H |
| O | NH | H | H | H | H | H |
| O | S | H | H | H | H | H |
| O | NH.HCl | H | H | H | H | H |
| Se | O | H | H | H | H | H |
| Se | NH | H | H | H | H | H |
| Se | S | H | H | H | H | H |
| NH | NH.HCl | H | H | H | H | H |
| NH | S | H | H | H | H | H |
| O | O | 4-Cl | H | H | H | H |
| O | O | 4-Cl | H | 7-OMe | H | H |
| O | O | 4-Me | H | H | H | H |

| Y | X | $R_1$ | $R_2$ | $R_3$ | $R_4$ | T |
|---|---|---|---|---|---|---|
| O | O | H | 2-Cl | H | H | H |
| O | O | 4-Cl | 2-S-pPAA* | H | H | H |
| Se | O | 4-Cl | H | H | H | H |
| Se | O | 4-Cl | H | 7-OMe | H | H |
| Se | O | 4-Me | H | H | H | H |
| Se | O | 4-Cl | 2-S-pPAA* | H | H | H |
| N—$CH_3$ | O | 4-Cl | H | H | H | H |
| N—$C_6H_6$ | O | 4-Cl | H | 7-OMe | H | H |
| N—H | O | 4-Cl | 2-S-pPAA* | H | H | H |
| S | O | 4-Cl | H | H | H | H |
| SO | O | H | H | H | H | H |
| $SO_2$ | O | H | H | H | H | H |
| $SO_2$ | O | 4-Cl | H | H | H | H |
| N—Me | O | H | H | H | H | H |
| N—Me | O | 4-Cl | H | H | H | H |
| N—Me | O | 4-Cl | H | 7-OMe | H | H |
| NCN | O | 4-Cl | H | H | H | H |
| NH | O | 4-Cl | H | H | H | H |
| NH | O | 4-Cl | H | H | H | H |
| S | O | 2-t-Bu | 9-t-Bu | 4-OMe | H | H |
| S | O | 2-t-Bu | 7-t-Bu | 4-F | H | H |
| S | O | 2-t-Bu | 7-t-Bu | 4-Me | H | H |
| S | O | 2-t-Bu | 7-t-Bu | 4-SMe | H | H |

*pPAA = Paraphenylessigsäure

12. Verwendung, zur Herstellung eines Medikaments zur Inhibierung der Leukotrienbiosynthese oder -wirkung in Säugetieren, einer Verbindung der Formel I, einer Verbindung, die ein Salz einer Verbindung der Formel I ist, oder einer eine solche Verbindung enthaltenden pharmazeutischen Zusammensetzung, worin in Formel I die Variablen wie nachstehend definiert sind und X in der 3-Stellung ist.

| $R_1$ | $R_2$ | $R_3$ | $R_4$ | T |
|---|---|---|---|---|
| H | H | H | H | H |
| 2-Cl | H | H | H | H |
| H | H | 6-Cl | H | H |
| H | H | 7-Cl | H | H |

| $R_1$ | $R_2$ | $R_3$ | $R_4$ | T |
|---|---|---|---|---|
| H | H | 8-Cl | H | H |
| H | H | 9-Cl | H | H |
| 1-Cl | H | H | H | H |
| 1-Cl | 4-Cl | H | H | H |
| 2-Cl | 4-Cl | H | H | 1-Cl |
| 2-$N(Me)_2$ | H | H | H | H |
| 2-SMe | H | H | H | H |
| 2-S-pPAA | H | H | H | H |
| 2-$C(O)CH_3$ | H | H | H | H |
| 2-OMe | H | H | H | H |
| H | H | H | 7-$CH_2CO_2H$ | H |
| H | H | H | 8-$CH_2COOH$ | H |
| H | 2-$SO_3H$ | H | H | H |
| 2-$N(Me)_2$ | H | H | H | H |
| 2-SMe | H | H | H | H |
| 2-$C(O)CH_3$ | H | H | H | H |
| 2-OMe | H | H | H | 7-I |
| 2-$CH_2CO_2H$ | H | H | H | H |
| 2-$CH(CH_3)CO_2H$ | H | H | H | H |
| 4-$CH_2COOH$ | H | H | H | H |
| 4-$CH(CH_3)CO_2H$ | H | H | H | H |
| H | H | 7-OH | 6-propyl | H |
| 4-Cl | H | H | H | H |
| 4-F | H | H | H | H |
| 4-F | H | 7-Cl | H | H |
| 4-Et | H | H | H | H |
| 4-Et | H | 7-OMe | H | H |
| 4-Et | H | 7-Cl | H | H |
| 4-Cl | H | 7-OMe | H | H |
| 4-OMe | H | 7-Cl | H | H |
| 4-Cl | H | 6-Cl | H | H |
| 4-Cl | H | 8-Cl | H | H |

| $R_1$ | $R_2$ | $R_3$ | $R_4$ | T |
|---|---|---|---|---|
| 4-Cl | H | 9-Cl | H | H |
| 4-Cl | H | 6-OMe | H | H |
| 4-Cl | H | 8-OMe | H | H |
| 4-Cl | H | 9-Et | H | h |
| 4-Cl | H | 6-Et | H | H |
| 4-Cl | H | 7-Et | H | H |
| 4-Cl | H | 8-Et | H | H |
| 4-Cl | 1-Et | H | H | H |
| 4-Cl | 2-Et | H | H | H |
| 4-Cl | 1-$CH_2COOH$ | H | H | H |
| 4-Cl | 2-$CH_2COOH$ | H | H | H |
| 4-OH | 2-OMe | 7-OMe | H | H |
| 4-OH | H | H | H | H |
| 4-Me | 1-OMe | 2-OMe | H | H |
| 4-Cl | H | 6-$CH_2COOH$ | H | H |
| 4-Cl | H | 7-$CH_2COOH$ | H | H |
| 4-Cl | H | 8-$CH_2COOH$ | H | H |
| 4-Cl | 2-$N(Me)_2$ | H | H | H |
| 4-Cl | 1-$N(Me)_2$ | H | H | H |
| 4-Cl | 2-$N(Me)_2$ | 7-OMe | H | H |
| 4-Cl | 2-$N(Me)_2$ | 7-Cl | H | H |
| 4-Cl | 2-SMe | H | H | H |
| 4-Cl | 2-$SCH_2COOH$ | H | H | H |
| 4-Cl | 2-S-pPAA | H | H | H |
| 4-Cl | 1-S-pPAA | H | H | H |
| 4-Cl | 2-S-pPAA | 7-OMe | H | H |
| 4-Cl | 2-$SO_3H$ | H | H | H |
| 4-Cl | 2-OMe | H | H | H |
| 4-Cl | 2-OMe | 7-Cl | H | H |
| 4-Cl | H | 7F | H | H |
| 4-OMe | H | 7-OMe | H | H |

| $R_1$ | $R_2$ | $R_3$ | $R_4$ | T |
|---|---|---|---|---|
| 4-OMe | H | 7-Me | H | H |
| 4-OMe | 2-SMe | H | H | H |
| 4-SMe | H | H | H | H |
| 4-Br | H | H | H | H |
| 4-I | H | H | H | H |
| 4-Br | H | 7-OMe | H | H |
| 4-I | H | 7-OMe | H | H |
| 4-Br | 2-Me | H | H | H |
| 4-I | 2-Me | H | H | H |
| 4-Cl | H | 7/8-$(CH_2)_4$— | | H |
| 4-Cl | H | 7/8-$(CH_2)_3$— | | H |
| 4-Br | 2-OMe | 7-OMe | H | H |
| 7-F | H | H | H | H |
| 7-$NH_2$ | H | H | H | H |
| 2-Me | 7-$N(Me)_2$ | H | H | H |
| 7-$N(Me)_2$ | H | H | H | H |
| 1-$CO_2H$ | 4-OH | 7-$NMe_2$ | H | H |
| 1-Cl | 2-Cl | 4-Cl | H | 7-Cl |
| 1-Me | 7-Me | H | H | 4-Cl |
| 2-Me | 7-Me | H | H | 4-Cl |
| 2-Me | H | H | H | 4-Cl |
| 7-Me | H | H | H | 4-Cl |
| 9-OMe | H | H | H | H |
| 2-OMe | H | H | H | 7-F |
| 2-OMe | 4-OMe | H | H | H |
| 1-OMe | 2-OMe | 7-Me | H | H |
| 1-Me | 7-Me | H | H | H |
| 2-Me | 7-Me | H | H | H |
| 2-Cl | 4-Cl | H | H | 7-F |
| 1-Cl | 4-Cl | H | H | 7-F |
| 2-OMe | 7-SMe | H | H | H |

| $R_1$ | $R_2$ | $R_3$ | $R_4$ | T |
|---|---|---|---|---|
| H | H | H | H | 4-$CF_3$ |
| 2-$CF_3$ | H | H | H | 4-$CF_3$ |
| 4-COMe | H | H | H | H |
| 2-OEt | H | H | H | 4-Cl |
| 2-S-n-Bu | H | H | H | H |
| 4-S-n-Bu | H | H | H | H |
| 2-Me | 4-S-n-Bu | H | H | H |
| 9-OMe | H | H | H | H |
| 2-OMe | H | H | H | H |
| 2-OMe | 4-OMe | H | H | H |
| 1-OMe | 2-OMe | 4-Me | H | H |
| 4-OMe | H | H | H | H |
| 1-OMe | 7-OMe | H | H | 4-Br |
| 1-OMe | 7-OMe | 2-Cl | H | 4-Cl |
| 1-OMe | 7-OMe | H | H | 4-Cl |
| 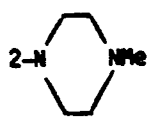 | 7-OMe | H | H | H |
| 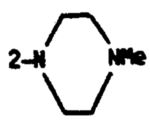 | 7-OMe | H | H | 4-Br |
| 2-OMe | 4-OH | 7-OMe | H | H |
| 1-NHPr | 4-NHPr | H | H | H |
| 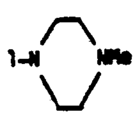 | 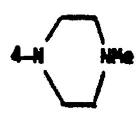 | H | H | H |
| 4-COMe | H | H | H | H |
| 2-NHPr | 4-NHPr | H | H | H |
| 2-OME | 4-CN | 7-OMe | H | H |
| 2-OMe | 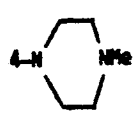 | 7-OMe | H | H |
| 2-OMe | 7-OMe | H | H | H |
| 2-OMe | 4-NHPr | 7-OMe | H | H |
| 1-NHPr | 4-NHPr | H | H | H |

| $R_1$ | $R_2$ | $R_3$ | $R_4$ | T |
|---|---|---|---|---|
| 1-NHPr | 4-NHPr | 7-OMe | H | H |
| 1-NHPr | 4-NHPr | H | H | H |
| 2-NHPr | 4-NHPr | 7-OMe | H | H |
| 2-OMe | 4-$NH_2$ | 7-OMe | H | H |
| 2-OMe | 4-NHPr | 7-OMe | H | H |
| 1-OMe | 4-Cl | 7-OMe | H | H |
| 1-OMe | 4-Br | 7-OMe | H | H |
| 1-NHPr | 4-NHPr | H | H | H |
| 1-OMe | 4-CN | 7-OMe | H | H |
| 2-OMe | 4-$NHCH_2CO_2R$* | 7-OMe | H | H |
| 2-OMe | 4-S-7-Bu | 7-OMe | H | H |
| 2-OMe | 4-$CH_2CO_2R$* | 7-OMe | H | H |
| 2-OMe | 4-$SO_2$Me | 7-OMe | H | H |
| 2-S-n-Bu | H | H | H | H |
| 4-S-n-Bu | H | H | H | H |
| 2-Me | 4-S-n-Bu | H | H | H , |
| 2-OMe | 7-Me | H | H | 4-Br |
| 2-OMe | 7-$CF_3$ | H | H | 4-Br |
| 2-OMe | 7-F | H | H | 4-Br |
| 2-OMe | 7-Cl | H | H | 4-Br |
| 2-OMe | 7-Br | H | H | 4-Br |
| 2-OMe | 7-$NMe_2$ | H | H | 4-Br |
| 2-OMe | 7-SMe | H | H | 4-Br |
| 2-OMe | 7-$SO_2$Me | H | H | 4-Br |
| 2-OMe | 7-Ph | H | H | 4-Br |
| 1-Me | H | H | H | 4-Br |
| 2-Me | H | H | H | H |
| 2-OEt | H | H | H | H |
| 7-Cl | H | H | H | H |
| 9-Cl | H | H | H | H |
| 7-F | H | H | H | H |
| 7-Me | H | H | H | H |

| $R_1$ | $R_2$ | $R_3$ | $R_4$ | T |
|---|---|---|---|---|
| 7-OMe | H | H | H | H |
| 2-Cl | H | H | H | 4-Cl |
| 1-Me | 7-Me | H | H | 4-Cl |
| 1-Me | 7-Me | H | H | H |
| 2-OMe | 7-OEt | H | H | H |
| $2-NH_2$ | H | H | H | H |
| 7-OH | H | H | H | H |

pPAA = Paraphenylessigsäure und worin R H oder $C_1$—$C_4$-Alkyl ist.

13. Verwendung, zur Herstellung eines Medikaments zur Inhibierung der Leukotrienbiosynthese oder -wirkung in Säugetieren, einer Verbindung der Formel I, einer Verbindung, die ein Salz einer Verbindung der Formel I ist, oder einer eine solche Verbindung enthaltenden pharmazeutischen Zusammensetzung, worin in Formel I die Variablen wie nachstehend definiert sind und X in der 3-Stellung ist:

| Y | X | $R_1$ | $R_2$ | $R_3$ | $R_4$ | T |
|---|---|---|---|---|---|---|
| S | O | 2-OMe | 7-OMe | H | H | H |
| S | O | 1-OMe | 7-OMe | H | H | H |
| S | O | 2-OMe | 7-OMe | H | H | 1-Br |
| S | O | 1-OMe | 7-OMe | H | H | 2-Br |
| S | O | 1-OMe | 7-OMe | H | H | 4-Br |
| S | O | 1-OMe | 7-OMe | H | H | 2-Cl |
| S | O | 1-OMe | 7-OMe | H | H | 4-Cl |
| S | O | 2-OMe | 7-OMe | H | H | 1-Cl |
| S | O | 2-OMe | 7-OMe | H | H | 4-Cl |
| S | O | 2-OEt | 7-OEt | H | H | 1-Br |
| S | O | 2-OEt | 7-OEt | H | H | 4-Br |
| S | O | 2-OEt | 7-OEt | H | H | 1-Cl |
| S | O | 2-OEt | 7-OEt | H | H | 4-Cl |
| S | O | 2-OMe | 7-OMe | 8-OMe | H | 1-Br |
| S | O | 2-OMe | 7-OMe | 8-OMe | H | 4-Br |
| S | O | 2-OMe | 7-OMe | H | H | 4-F |
| S | O | 2-OMe | 7-OMe | H | H | $4-CF_3$ |
| S | O | 2-OMe | 7-OEt | H | H | 4-Be |
| S | O | 2-OMe | 7-OEt | H | H | 4-Cl |

| Y | X | $R_1$ | $R_2$ | $R_3$ | $R_4$ | T |
|---|---|---|---|---|---|---|
| S | O | 2-OMe | 7-OEt | H | H | 4-F |
| S | O | 2-OMe | 7-OEt | H | H | 4-$CF_3$ |
| S | O | 2-OEt | 7-OMe | H | H | 4-Br |
| S | O | 2-OEt | 7-OMe | H | H | 4-Cl |
| S | O | 2-OEt | 7-OMe | H | H | 4-F |
| S | O | 2-OEt | 7-OMe | H | H | 4-$CF_3$ |
| S | O | 2-OMe | 7-OMe | H | H | 4-Br |

14. Verwendung, zur Herstellung eines Medikaments zur Inhibierung der Leukotrienbiosynthese oder -wirkung in Säugetieren, einer Verbindung der Formel I, einer Verbindung, die ein Salz einer Verbindung der Formel II ist:

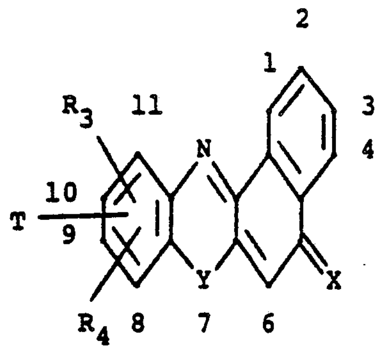

II

worin die Substituenten wie folgt sind:

| Y | X | $R_3$ | $R_4$ | T |
|---|---|---|---|---|
| O | O | H | H | H |
| S | O | H | H | H |
| SO | O | H | H | H |
| $SO_2$ | O | H | H | H |
| SO | O | H | H | 6-Cl |
| S | O | 6-$COCH_3$ | H | H |
| S | O | 6-$CH_3$ | H | H |
| $SO_2$ | O | 6-OH | H | H |
| $SO_2$ | O | 6-OMe | H | H |
| S | O | 9-OMe | H | H |
| S | O | 6-OH | H | H |
| S | O | 6-OMe | H | H |
| S | O | 6-NHCOMe | H | H |
| S | O | 6-NHPh | H | H |

| Y | X | $R_3$ | $R_4$ | T |
|---|---|---|---|---|
| S | O | H | H | 6-Br |
| S | O | 6-NHMe | H | H |
| S | O | 6-NH-t-Bu | H | H |
| S | O | 6-NH-COMe | H | 9-Cl |
| S | O | 6-NH-COMe | 9-Ome | H |
| S | O | 6-NHPh-p-Br | H | 9-Cl |
| O | O | H | H | 6-Cl |
| O | O | H | H | 6-Br |
| O | O | 9-OMe | H | 6-Br |
| O | O | 9-OMe | 6-NHPr | H |
| S | O | 6-$CF_3$ | H | H |
| S | O | 6-S-n-Bu | H | H |
| S | O | 6-OMe | H | 9-Cl |
| S | O | 9-OMe | H | 6-Cl |
| S | O | 6-OMe | 9-OMe | H |
| S | O | 6-Cl | 9-Me | 11-Br |
| S | O | 6-NHPh | 9-Me | 11-Br |
| S | O | 6-Me | H | H |
| O | NH | 9-$NMe_2$ | 10-Me | H |
| O | NH | 9-$N(Et)_2$ | H | H |
| S | O | 6-Cl | H | H |

15. Verwendung, wie in einem der Ansprüche 1 bis 14 beansprucht, angewandt auf die Behandlung von (1) Lungenzuständen, (2) Entzündungen, (3) Allergien, (4) Schmerz, (5) cardiovaskuläre Zustände oder (6) Hautzustände.

16. Verbindungen der Formel

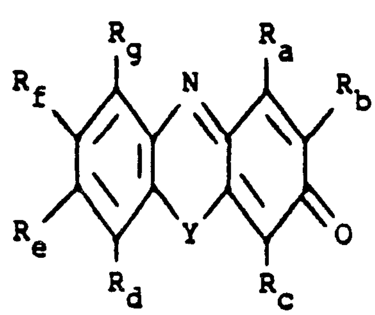

worin die Substituenten wie folgt sind:

| Y | $R_a$ | $R_b$ | $R_c$ | $R_d$ | $R_e$ | $R_f$ | $R_g$ |
|---|---|---|---|---|---|---|---|
| S | H | $SCH_3$ | H | H | H | H | H |
| S | H | H | $SCF_3$ | H | H | H | H |
| S | H | H | CHO | H | H | H | H |
| S | H | H | $COCF_3$ | H | H | H | H |
| S | H | H | H | H | $SCH_3$ | H | H |
| S | H | H | H | H | $CO_2CH_3$ | H | H |
| S | H | H | H | H | $CO_2H$ | H | H |
| S | H | H | H | H | CHO | H | H |
| S | H | H | H | H | $CONH_2$ | H | H |
| S | H | H | H | H | $CH_2OH$ | H | H |
| S | H | H | Cl | H | $CO_2Me$ | H | H |
| S | H | H | Cl | H | $CO_2H$ | H | H |
| S | H | H | Cl | H | CHO | H | H |
| S | H | H | Cl | H | $CONH_2$ | H | H |
| S | H | H | Cl | H | $CH_2OH$ | H | H |
| S | H | O-benzyl | Cl | H | H | H | H |
| S | H | OEt | H | H | $CH_3$ | H | H |
| S | $CH_3$ | H | Cl. | H | $CH_3$ | H | H |
| S | H | $CH_3$ | Cl | H | $CH_3$ | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| S | H | OMe | Br | H | Me | H | H |
| S | H | H | CHO | H | H | H | H |
| S | H | H | $COCF_3$ | H | H | H | H |
| S | H | H | H | H | $SCH_3$ | H | H |
| S | H | H | H | H | $OCH_3$ | H | H |

| Y | $R_a$ | $R_b$ | $R_c$ | $R_d$ | $R_e$ | $R_f$ | $R_g$ |
|---|---|---|---|---|---|---|---|
| S | H | H | H | H | $CO_2CH_3$ | H | H |
| S | H | H | H | H | $CO_2H$ | H | H |
| S | H | H | H | H | CN | H | H |
| S | H | H | H | H | CHO | H | H |
| S | H | H | H | H | $CONH_2$ | H | H |
| S | H | H | H | H | $CH_2OH$ | H | H |
| S | H | H | H | H | $CF_3$ | H | H |
| S | $CH_3$ | H | Cl | H | H | H | H |
| S | H | $CH_3$ | Cl | H | H | H | H |
| S | H | H | Cl | H | F | H | H |
| S | H | Cl | Cl | H | F | H | H |
| S | H | $CH_3$ | H | H | F | H | H |
| S | $CH_3$ | H | H | H | F | H | H |
| S | H | H | Cl | H | OMe | H | H |
| S | H | H | Cl | H | $CF_3$ | H | H |
| S | H | H | Cl | H | $CO_2Me$ | H | H |
| S | H | H | Cl | H | $CO_2H$ | H | H |
| S | H | H | Cl | H | CN | H | H |
| S | H | H | Cl | H | CHO | H | H |
| S | H | H | Cl | H | $CONH_2$ | H | H |
| S | H | H | Cl | H | $CH_2OH$ | H | H |
| S | H | H | $OCH_3$ | H | Cl | H | H |
| S | H | H | $CF_3$ | H | Cl | H | H |
| S | H | OEt | Cl | H | H | H | H |
| S | H | OiPr | H | H | H | H | H |
| S | OMe | H | Cl | H | H | H | H |
| S | OEt | H | Cl | H | H | H | H |
| S | H | OiPr | Cl | H | H | H | H |
| S | H | O-benzyl | Cl | H | H | H | H |
| S | H | OEt | H | H | F | H | H |

| Y | $R_a$ | $R_b$ | $R_c$ | $R_d$ | $R_e$ | $R_f$ | $R_g$ |
|---|---|---|---|---|---|---|---|
| S | H | $OCH_3$ | Cl | H | H | H | H |
| S | H | OEt | H | H | $CH_3$ | H | H |
| S | H | OEt | Cl | H | F | H | H |
| S | H | OEt | Cl | H | $CH_3$ | H | H |
| S | H | H | Cl | H | $CH_3$ | H | H |
| S | H | $CH_3$ | Cl | H | H | H | H |
| S | H | $CH_3$ | Br | H | H | H | H |
| S | $CH_3$ | H | H | H | $CH_3$ | H | H |
| S | H | $CH_3$ | H | H | $CH_3$ | H | H |
| S | $CH_3$ | H | Cl | H | $CH_3$ | H | H |
| S | H | $CH_3$ | Cl | H | $CH_3$ | H | H |
| S | Cl | H | Cl | H | F | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| O | H | H | Cl | H | H | H | H |
| S | H | OMe | Br | H | Me | H | H |
| $SO_2$ | H | H | OH | H | H | H | H |
| $SO_2$ | H | OMe | OH | H | OMe | H | H |
| $SO_2$ | OMe | OMe | Me | H | H | H | H |
| $SO_2$ | H | H | OMe | H | H | H | H |
| $SO_2$ | H | OMe | OMe | H | OMe | H | H |
| S | H | H | H | H | H | H | $OCH_3$ |
| S | H | $OCH_3$ | H | H | F | H | H |
| S | H | $OCH_3H$ | $OCH_3$ | H | H | H | H |
| S | $OCH_3$ | $OCH_3$ | Me | H | H | H | H |
| S | H | H | $COCH_3$ | H | H | H | H |

| Y | $R_a$ | $R_b$ | $R_c$ | $R_d$ | $R_e$ | $R_f$ | $R_g$ |
|---|---|---|---|---|---|---|---|
| S | $OCH_3$ | H | Br | H | $OCH_3$ | H | H |
| S | $OCH_3$ | Cl | Cl | H | $OCH_3$ | H | H |
| S | $OCH_3$ | H | Cl | H | $OCH_3H$ | H | H |
| S | H | 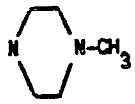 | H | H | $OCH_3$ | H | H |
| S | H | 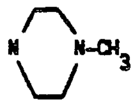 | Br | H | $OCH_3$ | H | H |
| $SO_2$ | H | $OCH_3$ | OH | H | $OCH_3$ | H | H |
| $SO_2$ | NHPr | H | NHPr | H | H | H | H |
| $SO_2$ | 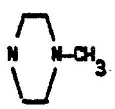 | H | 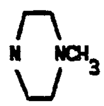 | H | H | H | H |
| $SO_2$ | H | $OCH_3$ | 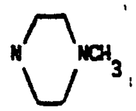 | H | $OCH_3$ | H | H |
| $SO_2$ | H | $OCH_3$ | Br | H | $OCH_3$ | H | H |
| S | NHPR | H | NHPr | H | H | H | H |
| S | NHPr | H | NHPr | H | $OCH_3$ | H | H |
| S | H | NHPr | NHPr | H | H | H | H |
| S | H | NHPr | NHPr | H | $OCH_3$ | H | H |
| S | H | $OCH_3$ | $NH_2$ | H | $OCH_3$ | H | H |
| S | H | $OCH_3$ | NHPr | H | $OCH_3$ | H | H |
| $SO_2$ | H | $OCH_3$ | NHPr | H | $OCH_3$ | H | H |
| O | $OCH_3$ | H | Cl | H | $OCH_3$ | H | H |
| O | $OCH_3$ | H | Br | H | $OCH_3$ | H | H |
| O | NHPr | H | NHPr | H | H | H | H |
| $SO_2$ | H | $OCH_3$ | CN | H | $OCH_3$ | H | H |
| $SO_2$ | H | $OCH_3$ | $NHCH_2CO_2R^*$ | H | $OCH_3$ | H | H |
| $SO_2$ | H | $OCH_3$ | S-N-Bu | H | $OCH_3$ | H | H |
| $SO_2$ | H | $OCH_3$ | $CH_2CO_2R^*$ | H | $OCH_3$ | H | H |
| $SO_2$ | H | $OCH_3$ | $SO_2CH_3$ | H | $OCH_3$ | H | H |
| S | H | S-n-Bu | H | H | H | H | H |
| S | H | H | S-n-Bu | H | H | H | H |

| Y | $R_a$ | $R_b$ | $R_c$ | $R_d$ | $R_e$ | $R_f$ | $R_g$ |
|---|---|---|---|---|---|---|---|
| S | H | $CH_3$ | S-n-Bu | H | H | H | H |
| S | H | OMe | Br | H | $CF_3$ | H | H |
| S | H | OMe | Br | H | F | H | H |
| S | H | OMe | Br | H | Cl | H | H |
| S | H | OMe | Br | H | Br | H | H |
| S | H | OMe | Br | H | $NMe_2$ | H | H |
| S | H | OMe | Br | H | SMe | H | H |
| S | H | OMe | Br | H | $SO_2Me$ | H | H |
| S | H | OMe | Br | H | Ph | H | H |
| S | H | H | H | Cl | OMe | H | H |

und R H oder $C_2$—$C_4$-Alkyl ist.

17. Verbindungen der Formel

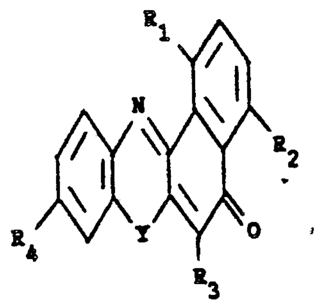

worin die Substituenten wie folgt sind:

| Y | $R_1$ | $R_2$ | $R_3$ | $R_4$ |
|---|---|---|---|---|
| S | H | H | S-n-$CH_4H_9$ | H |
| S | OH | H | $CH_3$ | H |
| S | $OCH_3$ | H | $CH_3$ | H |
| S | H | H | F | H |
| S | H | H | $CF_3$ | H |
| S | H | H | Cl | $CF_3$ |
| S | H | H | Cl | $SCH_3$ |
| S | H | H | Br | Cl |
| S | H | H | $CH_3$ | Br |
| S | H | H | F | Br |
| S | H | H | $COCH_3$ | Cl |
| S | H | H | $CF_3$ | $CH_3$ |
| S | H | H | $SC_4H_9$ | $CH_3$ |

| Y | $R_1$ | $R_2$ | $R_3$ | $R_4$ |
|---|---|---|---|---|
| S | H | H | $CF_3$ | Cl |
| S | H | H | Cl | $CH_2COOR$ |
| S | H | H | Cl | $CH(Me)CO_2R$ |
| S | H | H | Cl | $COCH_3$ |
| S | H | H | H | Cl |
| S | H | H | H | Br |
| S | H | H | H | F |
| S | H | H | H | $CF_3$ |
| S | H | H | H | $CH_3$ |
| S | H | H | H | $CH_2OH$ |
| S | H | H | H | $OCH_3$ |
| S | H | H | H | $SCH_3$ |
| S | H | H | H | COOR |
| S | H | H | H | $CH_2CO_2R$ |
| S | H | H | H | $CH(Me)CO_2R$ |
| $SO_2$ | H | H | NHPr | H |
| $SO_2$ | H | H | 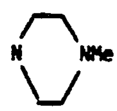 | H |
| $SO_2$ | H | H | $NH_2$ | H |
| $SO_2$ | H | H | NHPr | $OCH_3$ |
| S | -1,4-dihydro- | | H | H |
| S | H | H | NHPr | $OCH_3$ |
| O | H | H | Cl | H |
| O | H | H | Br | H |
| O | H | H | Br | $OCH_3$ |
| O | H | H | NHPr | $OCH_3$ |

und worin R H oder $C_1$—$C_4$-Alkyl ist.

18. Verbindungen der Formel

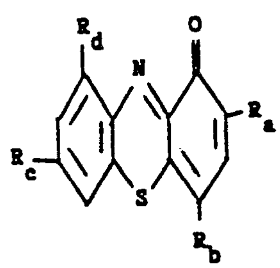

III

worin die Substituenten wie folgt sind:

| $R_a$ | $R_b$ | $R_c$ | $R_d$ |
|---|---|---|---|
| t-Bu | t-Bu | H | H |
| t-Bu | t-Bu | F | H |
| t-Bu | t-Bu | Me | H |
| t-Bu | t-Bu | SMe | H |
| t-Bu | t-Bu | H | OMe |

19. Die Verbindung 2-S-Glutathionyl-3H-phenothiazin-3-on und 4-Chlor-2-S-glutathionylphenothiazin-3-on.

20. Die in Anspruch 1 beansprucht Verwendung zur Herstellung eines Medikaments, die zusätzlich einen zweiten aktiven Bestandteil enthält, der ein nicht-steroides entzündungshemmendes Mittel ist; ein peripheres Analgetikum, ein Cyclooxygenaseinhibitor; ein Leukotrienantagonist; ein Antihistaminikum; ein Prostaglandinantagonist; oder ein Thromboxanantagonist, wobei das Gewichtsverhältnis der Verbindung der Formel I zum zweiten aktiven Bsestandteil im Bereich von 10:1 bis 1:10 liegt.

**Revendications**

1. L'utilisation, pour la fabrication d'un médicament pour l'inhibition de la biosynthèse ou de l'action des leucotriènes chez les mammifères, d'un composé de formule I, un composé qui est un sel d'un composé de formule I ou une composition pharmaceutique contenant un tel composé:

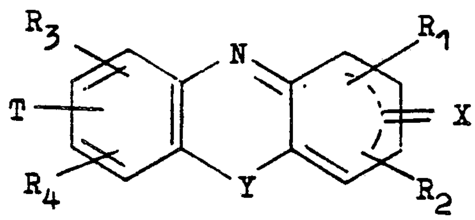

I

dans laquelle

X est dans la position 1 ou 3 et est O, S ou NR;

R est H, un alkyle ramifié ou linéaire en $C_{1-6}$, CN ou un phényle;

Y est O, Se, S, SO, $SO_2$ ou NR; et le pointillé représente une double liaison entre les positions 1 et 2 ou 2 et 3;

chacun de $R_1$, $R_2$, $R_3$ et $R_4$ indépendamment des autres est:

(1) un hydrogène,

(2) un alkyle en $C_{1-6}$,

(3) un alcényle en $C_{2-6}$,

(4) $-(CH_2)_nM$

où n est 0 ou un entier de 1 à 6 et M est

(a) $OR_5$,

(b) un halogène,

(c) $CF_3$,

(d) $SR_5$, où $R_5$ est H; un alkyle en $C_1$—$C_6$; un benzyle; un phényle ou un phényle substitué dont les substituants sont un alkyle en $C_{1-3}$, un halogène, CN, $CF_3$, $COOR_6$, $CH_2COOR_6$, $(CH_2)_nNR_8R_9$ où n est 0, 1 ou 2, un alcoxy en $C_{1-3}$, OH ou un halogénoalkylene en $C_{1-6}$; $-(CH_2)_mCOOR_6$, où m est 0 ou un entier de 1 à 6 et $R_6$ est H, un phényle ou un alkyle en $C_{1-6}$; CN, un formyle, $CF_3$ ou $CH_2-R_{12}$, où $R_{12}$ est un alkyle en $C_{1-5}$, un phényle ou un diméthylamino;

(e) un phényle ou un phényle substitué comme défini ci-dessus pour $R_5$;

(f) $COOR_6$;

(g) 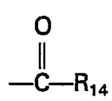

où $R_{14}$ est H, $(CH_2)_nCOOR_6$ où n est 0 ou un entier de 1 à 4, un alkyle en $C_{1-6}$, $CF_3$, un phényle ou un phényle substitué comme défini ci-dessus pour $R_5$;

(h) un tétrazole;

(i) $$-NH-\overset{O}{\overset{\|}{C}}-R_7$$

où $R_7$ est un alkyle en $C_{1-6}$, un benzyle ou un phényle;

(j) $-NR_8R_9$ où $R_8$ et $R_9$ sont indépendamment choisis parmi H, un phényle ou un phényle substitué comme défini ci-dessus pour $R_5$ ou un alkyle en $C_{1-4}$, un alkyl($C_{1-4}$)aminoalkyle ou peuvent être unis par N pour former un radical 4-méthylpipérazinyle;

(k) $-NHSO_2R_{10}$ où $R_{10}$ est OH, un alkyle en $C_{1-6}$, un alcoxy en $C_{1-6}$, un phényle ou $CF_3$;

(l) $$-\overset{O}{\overset{\|}{C}}-CH_2OH;$$

(m) $-SOR_{11}$ où $R_{11}$ est un alkyle en $C_{1-6}$, un phényle ou un phényle substitué comme défini ci-dessus pour $R^5$, $(CH_2)_mCOOR_6$ où m est 1 à 6, CN, un formyle ou $CF_3$;

(n) $-CONR_8R_9$;

(o) $-SO_2NR_8R_9$;

(p) $-SO_2R_{13}$ où $R_{13}$ est OH, H, un alkyle en $C_{1-6}$, un phényle ou un phényle substitué comme défini ci-dessus pour $R_5$, $(CH_2)_mCOOR_6$ où m est 1 à 6, CN ou $CF_3$;

(q) $NO_2$;

(r) $$-O-\overset{O}{\overset{\|}{C}}-R_{14};$$

(s) $$-O-\overset{O}{\overset{\|}{C}}-NR_8R_9;$$

(t) $$-O-\overset{O}{\overset{\|}{C}}-OR_7;$$

(u) —CN; ou

(v) $NR_{15}R_{16}$ où $R_{15}$ et $R_{16}$ sont chacun tels que $HNR_{15}R_{16}$ soit un amino-acide essentiel;

ou deux quelconques de $R_1$, $R_2$, $R_3$ et $R_4$ sont unis pour former un quatrième cycle saturé ou insaturé en $C_{5-6}$; et

T est H, un halogène ou $CF_3$.

2. L'utilisation selon la revendication 1, dans laquelle, dans la formule I, X est en position 1.

3. L'utilisation selon la revendication 2, dans laquelle X est O ou NR.

4. L'utilisation selon la revendication 3, dans laquelle, dans la formule I, Y est S, SO, $SO_2$, NR ou O et dans l'unité structurale $(CH_2)_nM$, n est 0 ou 1.

5. L'utilisation selon la revendication 4, dans laquelle, dans la formule I, X est O et Y est S.

6. L'utilisation selon la revendication 1, dans laquelle, dans la formule I, X est en la position 3.

7. L'utilisation selon la revendication 6, dans laquelle X est O ou NR.

8. L'utilisation selon la revendication 7, dans laquelle, dans la formule I, Y est S, SO, $SO_2$, NR ou O et dans l'unité structurale $(CH_2)_nM$, n est 0 ou 1.

9. L'utilisation selon la revendication 8, dans laquelle Y est S ou O.

10. L'utilisation selon la revendication 9, dans laquelle X est O et Y est S.

11. L'utilisation, pour la fabrication d'un médicament pour l'inhibition de la biosynthèse ou de l'action des leucotriènes chez les mammifères, d'un composé de formule I, un composé qui est un sel d'un composé de formule I ou une composition pharmaceutique contenant un tel composé, dans laquelle, dans la formule I, les variables sont comme défini ci-après, X étant en position 1:

| Y | X | $R_1$ | $R_2$ | $R_3$ | $R_4$ | T |
|---|---|---|---|---|---|---|
| O | O | 2-t-Bu | 8-t-Bu | 4-t-Bu | 6-t-Bu | H |
| O | O | 2-t-Bu | H | 4-Me | H | H |
| S | S | 2-t-Bu | 4-t-Bu | H | H | H |
| N—$CH_3$, S, O, Se, SO ou $SO_2$ | O | 2-Cl | H | H | H | H |
| 〃 | O | 2-$SCF_3$ | H | H | H | |
| 〃 | O | 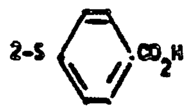 | H | H | H | H |
| S | O | 2-t-Bu | 4-t-Bu | H | H | H |
| N—$CH_3$, S, O, Se, SO ou $SO_2$ | O | 2-CN | H | H | H | H |
| 〃 | O | H | 3-$CO_2$Et | H | H | H |
| 〃 | O | H | 3-Cl | H | H | H |
| 〃 | O | H | H | 4-Cl | H | H |
| 〃 | O | H | H | 4-$SO_2CH_3$ | H | H |
| 〃 | O | 2-Cl | H | 4-Cl | H | H |
| 〃 | NH | 2-Cl | H | 4-Cl | H | H |
| 〃 | NH | H | H | H | H | H |
| N—CN | O | 2-Cl | H | 4-Cl | H | H |
| S | O | H | H | H | H | H |
| S | O | 2-Cl | 3-Cl | 4-Cl | 7-Cl | 9-Cl |
| S | O | 2-Br | 3-Br | 4-Br | 7-Br | 9-Br |
| S | O | H | H | H | 7-$SO_2CH_3$ | H |
| S | O | 2-Cl | H | 4-$SO_2CH_3$ | H | H |
| S | O | 2-F | H | 4-Cl | H | H |
| S | O | 2-Br | H | H | H | H |
| S | O | 2-$CF_3$ | H | H | H | H |
| S | O | 2-$SCF_3$ | H | H | H | H |
| S | O | 2-$SO_2CF_3$ | H | H | H | H |
| S | O | H | 3-Cl | H | H | H |
| S | O | H | 3-$CO_2$Et | H | H | H |
| S | O | H | 3-$CO_2$H | H | H | H |

| Y | X | $R_1$ | $R_2$ | $R_3$ | $R_4$ | T |
|---|---|---|---|---|---|---|
| S | O | H | 3-CN | H | H | H |
| S | O | H | 3-$SCF_3$ | H | H | H |
| S | O | H | H | 4-Cl | H | H |
| S | O | H | H | 4-$SCF_3$ | H | H |
| S | O | H | H | 4-Cl | H | H |
| S | O | 2-Br | H | 4-Br | H | H |
| S | O | 2-Cl | H | H | 8-CN | H |
| S | O | 2-Cl | H | H | 8-$CO_2Et$ | H |
| S | O | 2-Cl | H | H | 8-$CO_2H$ | H |
| S | O | 2-Cl | H | H | 8-$CF_3$ | H |
| S | O | 2-Cl | H | H | 7-$SO_2CH_3$ | H |
| S | O | H | 3-$CONMe_2$ | H | H | H |
| S | O | 2-Cl | H | H | 7-$OCH_3$ | H |
| S | O | 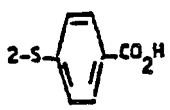 | H | H | H | H |
| S | O | 2-$SO_2CH_3$ | H | H | H | H |
| S | O | 2-$CH_2CH{=}CH_2$ | H | 4-$CH_2CH{=}CH_2$ | H | H |
| S | O | H | 3-$N(CH_3)_2$ | H | H | H |
| S | O | H | H | 4-Cl | 7-S—$C_6H_6$ | H |
| S | O | 2-$CHCO_2$ | H | H | H | H |
| S | O | 2-Cl | H | 4-$SCH_2CO_2H$ | H | H |
| S | O | 2-$COC_3$ | H | H | 7-$OCH_3$ | H |
| S | O | H | H | 4-CO—$C_6H_6$ | 7-$OCH_3$ | H |
| S | NH | 2-Cl | H | 4-Cl | H | H |
| S | NH | H | 3-$N(CH_3)_2$ | H | H | H |
| S | NH | 2-$SCH_3$ | H | 4-$SCH_3$ | H | H |
| S | O | H | H | H | H | H |
| S | NH | H | H | H | H | H |
| S | O | 2-$COCH_3$ | H | H | 7-$OCH_3$ | H |
| S | O | H | H | 4-$COC_6H_5$ | 7-$OCH_3$ | H |
| S | NH.HCl | H | H | H | H | H |
| S | O | H | H | H | H | H |

| Y | X | $R_1$ | $R_2$ | $R_3$ | $R_4$ | T |
|---|---|---|---|---|---|---|
| O | O | H | H | H | H | H |
| O | NH | H | H | H | H | H |
| O | S | H | H | H | H | H |
| O | NH.HCl | H | H | H | H | H |
| Se | O | H | H | H | H | H |
| Se | NH | H | H | H | H | H |
| Se | S | H | H | H | H | H |
| NH | NH.HCl | H | H | H | H | H |
| NH | S | H | H | H | H | H |
| O | O | 4-Cl | H | H | H | H |
| O | O | 4-Cl | H | 7-OMe | H | H |
| O | O | 4-Me | H | H | H | H |
| O | O | H | 2-Cl | H | H | H |
| O | O | 4-Cl | 2-S-pPAA* | H | H | H |
| Se | O | 4-Cl | H | H | H | H |
| Se | O | 4-Cl | H | 7-OMe | H | H |
| Se | O | 4-Me | H | H | H | H |
| Se | O | 4-Cl | 2-S-pPAA* | H | H | H |
| N—$CH_3$ | O | 4-Cl | H | H | H | H |
| N—$C_6H_6$ | O | 4-Cl | H | 7-OMe | H | H |
| N—H | O | 4-Cl | 2-S-pPAA* | H | H | H |
| S | O | 4-Cl | H | H | H | H |
| SO | O | H | H | H | H | H |
| $SO_2$ | O | H | H | H | H | H |
| $SO_2$ | O | 4-Cl | H | H | H | H |
| N—Me | O | H | H | H | H | H |
| N—Me | O | 4-Cl | H | H | H | H |
| N—Me | O | 4-Cl | H | 7-OMe | H | H |
| NCN | O | 4-Cl | H | H | H | H |
| NH | O | 4-Cl | H | H | H | H |
| NH | O | 4-Cl | H | H | H | H |

| Y | X | $R_1$ | $R_2$ | $R_3$ | $R_4$ | T |
|---|---|---|---|---|---|---|
| S | O | 2-t-Bu | 9-t-Bu | 4-OMe | H | H |
| S | O | 2-t-Bu | 7-t-Bu | 4-F | H | H |
| S | O | 2-t-Bu | 7-t-Bu | 4-Me | H | H |
| S | O | 2-t-Bu | 7-t-Bu | 4-SMe | H | H |

*pPAA = acide paraphénylacétique.

12. L'utilisation, pour la fabrication d'un médicament pour l'inhibition de la biosynthèse ou de l'action des leucotriènes chez les mammifères, d'un composé de formule I, un composé qui est un sel d'un composé de formule I ou une composition pharmaceutique contenant un tel composé, dans laquelle, dans la formule I, les variables sont comme défini ci-après, X étant en position 3:

| $R_1$ | $R_2$ | $R_3$ | $R_4$ | T |
|---|---|---|---|---|
| H | H | H | H | H |
| 2-Cl | H | H | H | H |
| H | H | 6-Cl | H | H |
| H | H | 7-Cl | H | H |
| H | H | 8-Cl | H | H |
| H | H | 9-Cl | H | H |
| 1-Cl | H | H | H | H |
| 1-Cl | 4-Cl | H | H | H |
| 2-Cl | 4-Cl | H | H | 1-Cl |
| $2\text{-}N(Me)_2$ | H | H | H | H |
| 2-SMe | H | H | H | H |
| 2-S-pPAA | H | H | H | H |
| $2\text{-}C(O)CH_3$ | H | H | H | H |
| 2-OMe | H | H | H | H |
| H | H | H | $7\text{-}CH_2CO_2H$ | H |
| H | H | H | $8\text{-}CH_2COOH$ | H |
| H | $2\text{-}SO_3H$ | H | H | H |
| $2\text{-}N(Me)_2$ | H | H | H | H |
| 2-SMe | H | H | H | H |
| $2\text{-}C(O)CH_3$ | H | H | H | H |
| 2-OMe | H | H | H | 7-I |
| $2\text{-}CH_2CO_2H$ | H | H | H | H |
| $2\text{-}CH(CH_3)CO_2H$ | H | H | H | H |

| $R_1$ | $R_2$ | $R_3$ | $R_4$ | T |
|---|---|---|---|---|
| 4-$CH_2COOH$ | H | H | H | H |
| 4-$CH(CH_3)CO_2H$ | H | H | H | H |
| H | H | 7-OH | 6-propyl | H |
| 4-Cl | H | H | H | H |
| 4-F | H | H | H | H |
| 4-F | H | 7-Cl | H | H |
| 4-Et | H | H | H | H |
| 4-Et | H | 7-OMe | H | H |
| 4-Et | H | 7-Cl | H | H |
| 4-Cl | H | 7-OMe | H | H |
| 4-OMe | H | 7-Cl | H | H |
| 4-Cl | H | 6-Cl | H | H |
| 4-Cl | H | 8-Cl | H | H |
| 4-Cl | H | 9-Cl | H | H |
| 4-Cl | H | 6-OMe | H | H |
| 4-Cl | H | 8-OMe | H | H |
| 4-Cl | H | 9-Et | H | h |
| 4-Cl | H | 6-Et | H | H |
| 4-Cl | H | 7-Et | H | H |
| 4-Cl | H | 8-Et | H | H |
| 4-Cl | 1-Et | H | H | H |
| 4-Cl | 2-Et | H | H | H |
| 4-Cl | 1-$CH_2COOH$ | H | H | H |
| 4-Cl | 2-$CH_2COOH$ | H | H | H |
| 4-OH | 2-OMe | 7-OMe | H | H |
| 4-OH | H | H | H | H |
| 4-Me | 1-OMe | 2-OMe | H | H |
| 4-Cl | H | 6-$CH_2COOH$ | H | H |
| 4-Cl | H | 7-$CH_2COOH$ | H | H |
| 4-Cl | H | 8-$CH_2COOH$ | H | H |
| 4-Cl | 2-$N(Me)_2$ | H | H | H |

| $R_1$ | $R_2$ | $R_3$ | $R_4$ | T |
|---|---|---|---|---|
| 4-Cl | 1-N(Me)$_2$ | H | H | H |
| 4-Cl | 2-N(Me)$_2$ | 7-OMe | H | H |
| 4-Cl | 2-N(Me)$_2$ | 7-Cl | H | H |
| 4-Cl | 2-SMe | H | H | H |
| 4-Cl | 2-SCH$_2$COOH | H | H | H |
| 4-Cl | 2-S-pPAA | H | H | H |
| 4-Cl | 1-S-pPAA | H | H | H |
| 4-Cl | 2-S-pPAA | 7-OMe | H | H |
| 4-Cl | 2-SO$_3$H | H | H | H |
| 4-Cl | 2-OMe | H | H | H |
| 4-Cl | 2-OMe | 7-Cl | H | H |
| 4-Cl | H | 7F | H | H |
| 4-OMe | H | 7-OMe | H | H |
| 4-OMe | H | 7-Me | H | H |
| 4-OMe | 2-SMe | H | H | H |
| 4-SMe | H | H | H | H |
| 4-Br | H | H | H | H |
| 4-I | H | H | H | H |
| 4-Br | H | 7-OMe | H | H |
| 4-I | H | 7-OMe | H | H |
| 4-Br | 2-Me | H | H | H |
| 4-I | 2-Me | H | H | H |
| 4-Cl | H | 7/8-(CH$_2$)$_4$— | | H |
| 4-Cl | H | 7/8-(CH$_2$)$_3$— | | H |
| 4-Br | 2-OMe | 7-OMe | H | H |
| 7-F | H | H | H | H |
| 7-NH$_2$ | H | H | H | H |
| 2-Me | 7-N(Me)$_2$ | H | H | H |
| 7-N(Me)$_2$ | H | H | H | H |
| 1-CO$_2$H | 4-OH | 7-NMe$_2$ | H | H |
| 1-Cl | 2-Cl | 4-Cl | H | 7-Cl |

| $R_1$ | $R_2$ | $R_3$ | $R_4$ | T |
|---|---|---|---|---|
| 1-Me | 7-Me | H | H | 4-Cl |
| 2-Me | 7-Me | H | H | 4-Cl |
| 2-Me | H | H | H | 4-Cl |
| 7-Me | H | H | H | 4-Cl |
| 9-OMe | H | H | H | H |
| 2-OMe | H | H | H | 7-F |
| 2-OMe | 4-OMe | H | H | H |
| 1-OMe | 2-OMe | 7-Me | H | H |
| 1-Me | 7-Me | H | H | H |
| 2-Me | 7-Me | H | H | H |
| 2-Cl | 4-Cl | H | H | 7-F |
| 1-Cl | 4-Cl | H | H | 7-F |
| 2-OMe | 7-SMe | H | H | H |
| H | H | H | H | 4-$CF_3$ |
| 2-$CF_3$ | H | H | H | 4-$CF_3$ |
| 4-COMe | H | H | H | H |
| 2-OEt | H | H | H | 4-Cl |
| 2-S-n-Bu | H | H | H | H |
| 4-S-n-Bu | H | H | H | H |
| 2-Me | 4-S-n-Bu | H | H | H |
| 9-OMe | H | H | H | H |
| 2-OMe | H | H | H | H |
| 2-OMe | 4-OMe | H | H | H |
| 1-OMe | 2-OMe | 4-Me | H | H |
| 4-OMe | H | H | H | H |
| 1-OMe | 7-OMe | H | H | 4-Br |
| 1-OMe | 7-OMe | 2-Cl | H | 4-Cl |
| 1-OMe | 7-OMe | H | H | 4-Cl |
| 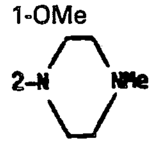 | 7-OMe | H | H | H |
| 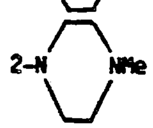 | 7-OMe | H | H | 4-Br |

| $R_1$ | $R_2$ | $R_3$ | $R_4$ | T |
|---|---|---|---|---|
| 2-OMe | 4-OH | 7-OMe | H | H |
| 1-NHPr | 4-NHPr | H | H | H |
| 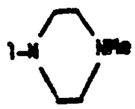 | 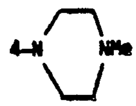 | H | H | H |
| 4-COMe | H | H | H | H |
| 2-NHPr | 4-NHPr | H | H | H |
| 2-OME | 4-CN | 7-OMe | H | H |
| 2-OMe | 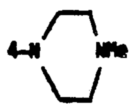 | 7-OMe | H | H |
| 2-OMe | 7-OMe | H | H | H |
| 2-OMe | 4-NHPr | 7-OMe | H | H |
| 1-NHPr | 4-NHPr | H | H | H |
| 1-NHPr | 4-NHPr | 7-OMe | H | H |
| 1-NHPr | 4-NHPr | H | H | H |
| 2-NHPr | 4-NHPr | 7-OMe | H | H |
| 2-OMe | $4-NH_2$ | 7-OMe | H | H |
| 2-OMe | 4-NHPr | 7-OMe | H | H |
| 1-OMe | 4-Cl | 7-OMe | H | H |
| 1-OMe | 4-Br | 7-OMe | H | H |
| 1-NHPr | 4-NHPr | H | H | H |
| 1-OMe | 4-CN | 7-OMe | H | H |
| 2-OMe | $4-NHCH_2CO_2R^*$ | 7-OMe | H | H |
| 2-OMe | 4-S-7-Bu | 7-OMe | H | H |
| 2-OMe | $4-CH_2CO_2R^*$ | 7-OMe | H | H |
| 2-OMe | $4-SO_2Me$ | 7-OMe | H | H |
| 2-S-n-Bu | H | H | H | H |
| 4-S-n-Bu | H | H | H | H |
| 2-Me | 4-S-n-Bu | H | H | H |
| 2-OMe | 7-Me | H | H | 4-Br |
| 2-OMe | $7-CF_3$ | H | H | 4-Br |
| 2-OMe | 7-F | H | H | 4-Br |
| 2-OMe | 7-Cl | H | H | 4-Br |

| $R_1$ | $R_2$ | $R_3$ | $R_4$ | T |
|---|---|---|---|---|
| 2-OMe | 7-Br | H | H | 4-Br |
| 2-OMe | 7-$NMe_2$ | H | H | 4-Br |
| 2-OMe | 7-SMe | H | H | 4-Br |
| 2-OMe | 7-$SO_2Me$ | H | H | 4-Br |
| 2-OMe | 7-Ph | H | H | 4-Br |
| 1-Me | H | H | H | 4-Br |
| 2-Me | H | H | H | H |
| 2-OEt | H | H | H | H |
| 7-Cl | H | H | H | H |
| 9-Cl | H | H | H | H |
| 7-F | H | H | H | H |
| 7-Me | H | H | H | H |
| 7-OMe | H | H | H | H |
| 2-Cl | H | H | H | 4-Cl |
| 1-Me | 7-Me | H | H | 4-Cl |
| 1-Me | 7-Me | H | H | H |
| 2-OMe | 7-OEt | H | H | H |
| 2-$NH_2$ | H | H | H | H |
| 7-OH | H | H | H | H |

*pPAA = acide paraphénylacétique.

et dans laquelle R est H ou un alkyle en $C_1$ à $C_4$.

13. L'utilisation, pour la fabrication d'un médicament pour l'inhibition de la biosynthèse ou de l'action des leucotriènes chez les mammifères, d'un composé de formule I, un composé qui est un sel d'un composé de formule I ou une composition pharmaceutique contenant un tel composé, dans laquelle, dans la formule I, les variables sont comme défini ci-après, X étant en position 3:

| Y | X | $R_1$ | $R_2$ | $R_3$ | $R_4$ | T |
|---|---|---|---|---|---|---|
| S | O | 2-OMe | 7-OMe | H | H | H |
| S | O | 1-OMe | 7-OMe | H | H | H |
| S | O | 2-OMe | 7-OMe | H | H | 1-Br |
| S | O | 1-OMe | 7-OMe | H | H | 2-Br |
| S | O | 1-OMe | 7-OMe | H | H | 4-Br |
| S | O | 1-OMe | 7-OMe | H | H | 2-Cl |
| S | O | 1-OMe | 7-OMe | H | H | 4-Cl |

| Y | X | $R_1$ | $R_2$ | $R_3$ | $R_4$ | T |
|---|---|---|---|---|---|---|
| S | O | 2-OMe | 7-OMe | H | H | 1-Cl |
| S | O | 2-OMe | 7-OMe | H | H | 4-Cl |
| S | O | 2-OEt | 7-OEt | H | H | 1-Br |
| S | O | 2-OEt | 7-OEt | H | H | 4-Br |
| S | O | 2-OEt | 7-OEt | H | H | 1-Cl |
| S | O | 2-OEt | 7-OEt | H | H | 4-Cl |
| S | O | 2-OMe | 7-OMe | 8-OMe | H | 1-Br |
| S | O | 2-OMe | 7-OMe | 8-OMe | H | 4-Br |
| S | O | 2-OMe | 7-OMe | H | H | 4-F |
| S | O | 2-OMe | 7-OMe | H | H | 4-$CF_3$ |
| S | O | 2-OMe | 7-OEt | H | H | 4-Be |
| S | O | 2-OMe | 7-OEt | H | H | 4-Cl |
| S | O | 2-OMe | 7-OEt | H | H | 4-F |
| S | O | 2-OMe | 7-OEt | H | H | 4-$CF_3$ |
| S | O | 2-OEt | 7-OMe | H | H | 4-Br |
| S | O | 2-OEt | 7-OMe | H | H | 4-Cl |
| S | O | 2-OEt | 7-OMe | H | H | 4-F |
| S | O | 2-OEt | 7-OMe | H | H | 4-$CF_3$ |
| S | O | 2-OMe | 7-OMe | H | H | 4-Br |

14. L'utilisation, pour la fabrication d'un médicament pour l'inhibition de la biosynthèse ou de l'action des leucotriènes chez les mammifères, d'un composé de formule I, un composé qui est un sel d'un composé de formule II:

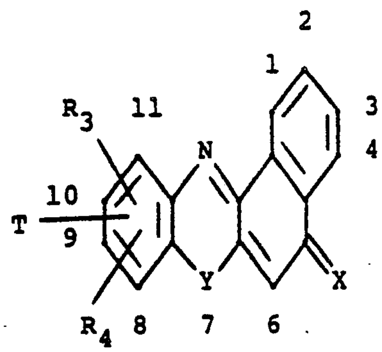

II

dans laquelle les substituants sont:

| Y | X | $R_3$ | $R_4$ | T |
|---|---|---|---|---|
| O | O | H | H | H |
| S | O | H | H | H |
| SO | O | H | H | H |

| Y | X | $R_3$ | $R_4$ | T |
|---|---|---|---|---|
| $SO_2$ | O | H | H | H |
| SO | O | H | H | 6-Cl |
| S | O | 6-$COCH_3$ | H | H |
| S | O | 6-$CH_3$ | H | H |
| $SO_2$ | O | 6-OH | H | H |
| $SO_2$ | O | 6-OMe | H | H |
| S | O | 9-OMe | H | H |
| S | O | 6-OH | H | H |
| S | O | 6-OMe | H | H |
| S | O | 6-NHCOMe | H | H |
| S | O | 6-NHPh | H | H |
| S | O | H | H | 6-Br |
| S | O | 6-NHMe | H | H |
| S | O | 6-NH-t-Bu | H | H |
| S | O | 6-NH-COMe | H | 9-Cl |
| S | O | 6-NH-COMe | 9-Ome | H |
| S | O | 6-NHPh-p-Br | H | 9-Cl |
| O | O | H | H | 6-Cl |
| O | O | H | H | 6-Br |
| O | O | 9-OMe | H | 6-Br |
| O | O | 9-OMe | 6-NHPr | H |
| S | O | 6-$CF_3$ | H | H |
| S | O | 6-S-n-Bu | H | H |
| S | O | 6-OMe | H | 9-Cl |
| S | O | 9-OMe | H | 6-Cl |
| S | O | 6-OMe | 9-OMe | H |
| S | O | 6-Cl | 9-Me | 11-Br |
| S | O | 6-NHPh | 9-Me | 11-Br |
| S | O | 6-Me | H | H |
| O | NH | 9-$NMe_2$ | 10-Me | H |
| O | NH | 9-$N(Et)_2$ | H | H |
| S | O | 6-Cl | H | H |

15. L'utilisation selon l'une quelconque des revendications 1 à 14, appliquée au traitement de (1) les affections pulmonaires, (2) l'inflammation, (3) les allergies, (4) la douleur, (5) les affections cardiovasculaires ou (6) les affections cutanées.

16. Les composés de formule:

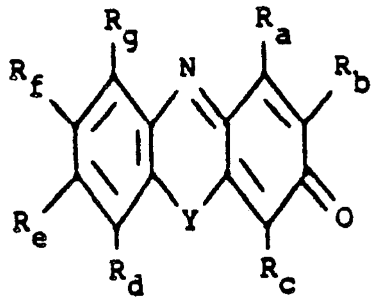

dans laquelle les substituants sont:

| Y | $R_a$ | $R_b$ | $R_c$ | $R_d$ | $R_e$ | $R_f$ | $R_g$ |
|---|---|---|---|---|---|---|---|
| S | H | $SCH_3$ | H | H | H | H | H |
| S | H | H | $SCF_3$ | H | H | H | H |
| S | H | H | CHO | H | H | H | H |
| S | H | H | $COCF_3$ | H | H | H | H |
| S | H | H | H | H | $SCH_3$ | H | H |
| S | H | H | H | H | $CO_2CH_3$ | H | H |
| S | H | H | H | H | $CO_2H$ | H | H |
| S | H | H | H | H | CHO | H | H |
| S | H | H | H | H | $CONH_2$ | H | H |
| S | H | H | H | H | $CH_2OH$ | H | H |
| S | H | H | Cl | H | $CO_2Me$ | H | H |
| S | H | H | Cl | H | $CO_2H$ | H | H |
| S | H | H | Cl | H | CHO | H | H |
| S | H | H | Cl | H | $CONH_2$ | H | H |
| S | H | H | Cl | H | $CH_2OH$ | H | H |
| S | H | O-benzyl | Cl | H | H | H | H |
| S | H | OEt | H | H | $CH_3$ | H | H |
| S | $CH_3$ | H | Cl | H | $CH_3$ | H | H |
| S | H | $CH_3$ | Cl | H | $CH_3$ | H | H |
| S | H | OMe | Br | H | OMe | H | H |
| S | H | OMe | Cl | H | OMe | H | H |
| S | H | OEt | Br | H | OEt | H | H |

| Y | $R_a$ | $R_b$ | $R_c$ | $R_d$ | $R_e$ | $R_f$ | $R_g$ |
|---|---|---|---|---|---|---|---|
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| S | H | OMe | Br | H | Me | H | H |
| S | H | H | CHO | H | H | H | H |
| S | H | H | $COCF_3$ | H | H | H | H |
| S | H | H | H | H | $SCH_3$ | H | H |
| S | H | H | H | H | $OCH_3$ | H | H |
| S | H | H | H | H | $CO_2CH_3$ | H | H |
| S | H | H | H | H | $CO_2H$ | H | H |
| S | H | H | H | H | CN | H | H |
| S | H | H | H | H | CHO | H | H |
| S | H | H | H | H | $CONH_2$ | H | H |
| S | H | H | H | H | $CH_2OH$ | H | H |
| S | H | H | H | H | $CF_3$ | H | H |
| S | $CH_3$ | H | Cl | H | H | H | H |
| S | H | $CH_3$ | Cl | H | H | H | H |
| S | H | H | Cl | H | F | H | H |
| S | H | Cl | Cl | H | F | H | H |
| S | H | $CH_3$ | H | H | F | H | H |
| S | $CH_3$ | H | H | H | F | H | H |
| S | H | H | Cl | H | OMe | H | H |
| S | H | H | Cl | H | $CF_3$ | H | H |
| S | H | H | Cl | H | $CO_2Me$ | H | H |
| S | H | H | Cl | H | $CO_2H$ | H | H |
| S | H | H | Cl | H | CN | H | H |
| S | H | H | Cl | H | CHO | H | H |
| S | H | H | Cl | H | $CONH_2$ | H | H |

| Y | $R_a$ | $R_b$ | $R_c$ | $R_d$ | $R_e$ | $R_f$ | $R_g$ |
|---|---|---|---|---|---|---|---|
| S | H | H | Cl | H | $CH_2OH$ | H | H |
| S | H | H | $OCH_3$ | H | Cl | H | H |
| S | H | H | $CF_3$ | H | Cl | H | H |
| S | H | OEt | Cl | H | H | H | H |
| S | H | OiPr | H | H | H | H | H |
| S | OMe | H | Cl | H | H | H | H |
| S | OEt | H | Cl | H | H | H | H |
| S | H | OiPr | Cl | H | H | H | H |
| S | H | O-benzyl | Cl | H | H | H | H |
| S | H | OEt | H | H | F | H | H |
| S | H | $OCH_3$ | Cl | H | H | H | H |
| S | H | OEt | H | H | $CH_3$ | H | H |
| S | H | OEt | Cl | H | F | H | H |
| S | H | OEt | Cl | H | $CH_3$ | H | H |
| S | H | H | Cl | H | $CH_3$ | H | H |
| S | H | $CH_3$ | Cl | H | H | H | H |
| S | H | $CH_3$ | Br | H | H | H | H |
| S | $CH_3$ | H | H | H | $CH_3$ | H | H |
| S | H | $CH_3$ | H | H | $CH_3$ | H | H |
| S | $CH_3$ | H | Cl | H | $CH_3$ | H | H |
| S | H | $CH_3$ | Cl | H | $CH_3$ | H | H |
| S | Cl | H | Cl | H | F | H | H |
| S | H | OMe | Br | H | OMe | H | H |
| S | H | OMe | Cl | H | OMe | H | H |
| S | H | OEt | Br | H | OEt | H | H |
| S | H | OEt | Cl | H | OEt | H | H |
| S | H | OMe | Cl | H | OEt | H | H |
| S | H | OMe | H | H | SMe | H | H |
| O | H | OMe | Br | H | OMe | H | H |
| O | H | OMe | Cl | H | OMe | H | H |
| O | H | H | Cl | H | H | H | H |
| S | H | OMe | Br | H | Me | H | H |

| Y | $R_a$ | $R_b$ | $R_c$ | $R_d$ | $R_e$ | $R_f$ | $R_g$ |
|---|---|---|---|---|---|---|---|
| $SO_2$ | H | H | OH | H | H | H | H |
| $SO_2$ | H | OMe | OH | H | OMe | H | H |
| $SO_2$ | OMe | OMe | Me | H | H | H | H |
| $SO_2$ | H | H | OMe | H | H | H | H |
| $SO_2$ | H | OMe | OMe | H | OMe | H | H |
| S | H | H | H | H | H | H | $OCH_3$ |
| S | H | $OCH_3$ | H | H | F | H | H |
| S | H | $OCH_3H$ | $OCH_3$ | H | H | H | H |
| S | $OCH_3$ | $OCH_3$ | Me | H | H | H | H |
| S | H | H | $COCH_3$ | H | H | H | H |
| S | $OCH_3$ | H | Br | H | $OCH_3$ | H | H |
| S | $OCH_3$ | Cl | Cl | H | $OCH_3$ | H | H |
| S | $OCH_3$ | H | Cl | H | $OCH_3H$ | H | H |
| S | H | 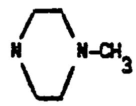 | H | H | $OCH_3$ | H | H |
| S | H | 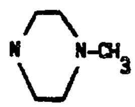 | Br | H | $OCH_3$ | H | H |
| $SO_2$ | H | $OCH_3$ | OH | H | $OCH_3$ | H | H |
| $SO_2$ | NHPr | H | NHPr | H | H | H | H |
| $SO_2$ | 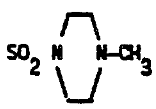 | H | 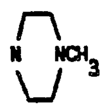 | H | H | H | H |
| $SO_2$ | H | $OCH_3$ | 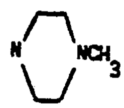 | H | $OCH_3$ | H | H |
| $SO_2$ | H | $OCH_3$ | Br | H | $OCH_3$ | H | H |
| S | NHPR | H | NHPr | H | H | H | H |
| S | NHPr | H | NHPr | H | $OCH_3$ | H | H |
| S | H | NHPr | NHPr | H | H | H | H |
| S | H | NHPr | NHPr | H | $OCH_3$ | H | H |
| S | H | $OCH_3$ | $NH_2$ | H | $OCH_3$ | H | H |
| S | H | $OCH_3$ | NHPr | H | $OCH_3$ | H | H |
| $SO_2$ | H | $OCH_3$ | NHPr | H | $OCH_3$ | H | H |
| O | $OCH_3$ | H | Cl | H | $OCH_3$ | H | H |

| Y | $R_a$ | $R_b$ | $R_c$ | $R_d$ | $R_e$ | $R_f$ | $R_g$ |
|---|---|---|---|---|---|---|---|
| O | $OCH_3$ | H | Br | H | $OCH_3$ | H | H |
| O | NHPr | H | NHPr | H | H | H | H |
| $SO_2$ | H | $OCH_3$ | CN | H | $OCH_3$ | H | H |
| $SO_2$ | H | $OCH_3$ | $NHCH_2CO_2R$* | H | $OCH_3$ | H | H |
| $SO_2$ | H | $OCH_3$ | S-N-Bu | H | $OCH_3$ | H | H |
| $SO_2$ | H | $OCH_3$ | $CH_2CO_2R$* | H | $OCH_3$ | H | H |
| $SO_2$ | H | $OCH_3$ | $SO_2CH_3$ | H | $OCH_3$ | H | H |
| S | H | S-n-Bu | H | H | H | H | H |
| S | H | H | S-n-Bu | H | H | H | H |
| S | H | $CH_3$ | S-n-Bu | H | H | H | H |
| S | H | OMe | Br | H | $CF_3$ | H | H |
| S | H | OMe | Br | H | F | H | H |
| S | H | OMe | Br | H | Cl | H | H |
| S | H | OMe | Br | H | Br | H | H |
| S | H | OMe | Br | H | $NMe_2$ | H | H |
| S | H | OMe | Br | H | SMe | H | H |
| S | H | OMe | Br | H | $SO_2Me$ | H | H |
| S | H | OMe | Br | H | Ph | H | H |
| S | H | H | H | Cl | OMe | H | H |

et R est H ou un alkyle en $C_2$ à $C_4$.

17. Les composés de formule:

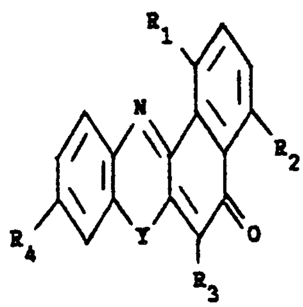

dans laquelle les substituants sont:

| Y | $R_1$ | $R_2$ | $R_3$ | $R_4$ |
|---|---|---|---|---|
| S | H | H | S-n-$CH_4H_9$ | H |
| S | OH | H | $CH_3$ | H |
| S | $OCH_3$ | H | $CH_3$ | H |
| S | H | H | F | H |

| Y | $R_1$ | $R_2$ | $R_3$ | $R_4$ |
|---|---|---|---|---|
| S | H | H | $CF_3$ | H |
| S | H | H | Cl | $CF_3$ |
| S | H | H | Cl | $SCH_3$ |
| S | H | H | Br | Cl |
| S | H | H | $CH_3$ | Br |
| S | H | H | F | Br |
| S | H | H | $COCH_3$ | Cl |
| S | H | H | $CF_3$ | $CH_3$ |
| S | H | H | $SC_4H_9$ | $CH_3$ |
| S | H | H | $CF_3$ | Cl |
| S | H | H | Cl | $CH_2COOR$ |
| S | H | H | Cl | $CH(Me)CO_2R$ |
| S | H | H | Cl | $COCH_3$ |
| S | H | H | H | Cl |
| S | H | H | H | Br |
| S | H | H | H | F |
| S | H | H | H | $CF_3$ |
| S | H | H | H | $CH_3$ |
| S | H | H | H | $CH_2OH$ |
| S | H | H | H | $OCH_3$ |
| S | H | H | H | $SCH_3$ |
| S | H | H | H | COOR |
| S | H | H | H | $CH_2CO_2R$ |
| S | H | H | H | $CH(Me)CO_2R$ |
| $SO_2$ | H | H | NHPr | H |
| $SO_2$ | H | H | 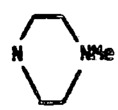 | H |
| $SO_2$ | H | H | $NH_2$ | H |
| $SO_2$ | H | H | NHPr | $OCH_3$ |
| S | -1,4-dihydro- | | H | H |
| S | H | H | NHPr | $OCH_3$ |
| O | H | H | Cl | H |

| Y | $R_1$ | $R_2$ | $R_3$ | $R_4$ |
|---|---|---|---|---|
| O | H | H | Br | H |
| O | H | H | Br | $OCH_3$ |
| O | H | H | NHPr | $OCH_3$ |

et dans laquelle R est H ou un alkyle en $C_1$ à $C_4$.

18. Les composés de formule:

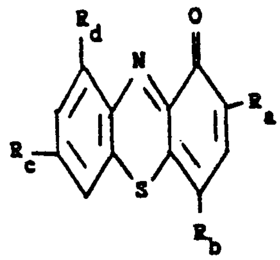

III

dans laquelle les substituants sont:

| $R_a$ | $R_b$ | $R_c$ | $R_d$ |
|---|---|---|---|
| t-Bu | t-Bu | H | H |
| t-Bu | t-Bu | F | H |
| t-Bu | t-Bu | Me | H |
| t-Bu | t-Bu | SMe | H |
| t-Bu | t-Bu | H | OMe |

19. Les composés 2-S-glutathionyl-3H-phénothiazine-3-one et 4-chloro-2-S-glutathionylphénothiazine-3-one.

20. L'utilisation, selon la revendication 1, pour la fabrication d'un médicament qui contient de plus un second ingrédient actif qui est un agent anti-inflammatoire non stéroïdien; un agent analgésique périphérique; un inhibiteur de la cyclooxygénase; un antagoniste des leucotriènes; un agent antihistaminique; un antagoniste des prostaglandines; ou un antagoniste du thromboxane, dans lequel le rapport pondéral du composé de formule I au second ingrédient actif est dans la gamme de 10/1 à 1/10.